# EUROPEAN PATENT APPLICATION

(11) **EP 1 939 198 A1**
(43) Date of publication of application: **02.07.2008**
(21) Application number: 06798198.5
(22) Date of filing: 21.09.2006
(51) Int. Cl.: C07D 473/18, A61K 31/522, A61K 31/5377, A61P 11/02, A61P 11/06, A61P 17/00, A61P 27/02, A61P 31/12, A61P 31/18, A61P 35/00, A61P 37/02, A61P 37/08, A61P 43/00, C07D 473/16, C07D 473/24, C07D 473/34

(54) **NOVEL ADENINE COMPOUND**

(30) Priority: 22.09.2005 JP 2005276171
(71) Applicant: Dainippon Sumitomo Pharma Co., Ltd., Osaka 541-8524 (JP); AstraZeneca AB, 151 85 Södertälje (SE)
(72) Inventor: HASHIMOTO, Kazuki, Osaka-shi, Osaka 5540022 (JP); NAKAMURA, Tomoaki, Osaka-shi, Osaka 5540022 (JP); NAKAMURA, Kei, Osaka-shi, Osaka 5540022 (JP); KURIMOTO, Ayumu, Osaka-shi, Osaka 5540022 (JP); ISOBE, Yoshiaki, Osaka-shi, Osaka 5540022 (JP); OGITA, Haruhisa, Saitama-shi, Saitam 3310802 (JP); MILLICHIP, Ian, LE115RH (GB); MCINALLY, Thomas, LE115RH (GB); BONNERT, Roger, LE115RH (GB)
(74) Representative: Vossius & Partner
(86) International application number: PCT/JP2006/318756
(87) International publication number: WO 2007/034881

(57) **Abstract**

An adenine compound or its pharmaceutically acceptable salt as a medicament as shown following formula (1): 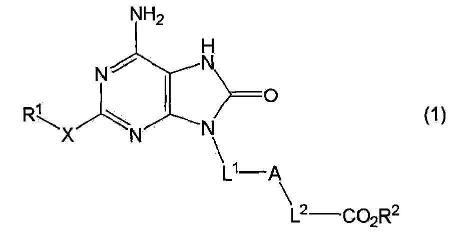 wherein R¹ is optionally substituted alkyl group, etc., X is oxygen atom, etc., A is 4 to 8 membered optionally substituted saturated or unsaturated heterocyclic group containing 1 to 2 hetero atoms selected from 1 to 3 nitrogen atoms, 0 to 1 oxygen atom and 0 to 1 sulfur atom, L¹ and L² are independently straight or branched chain alkylene, or a single bond, R² is optionally substituted alkyl group, etc.

## Description

### TECHNICAL FIELD

The present invention relates to a novel adenine compound useful as a prophylactic or therapeutic agent for allergic diseases, viral diseases or cancers.

### BACKGROUND ART

When a foreign substance such as bacteria, virus or parasite invades into a living body, immune system works to defense the foreign substance. In acquired immune system, once a foreign substance invades, antigen by antigen presenting cells such as dendritic cells (DC) is processed, and naive cells, via mutual action of DC/Th cells, functionally differentiate into Th1 cells or Th2 cells which contribute the main role to immune response in a living body. In this processing, when immune balance deviates to either Th cells or Th2 cells, it is considered that immune diseases develop.

Namely, in a body of a patient suffering from an allergic disease, cytokines such as interleulin-4 (IL-4) and interleukin-5 (IL-5) secreted from Th2-cells are excessively secreted. Therefore, compounds suppressing an immune response of Th2-cell can be expected as an agent for treating allergic diseases. On the other hand, compounds promoting an immune response of Th1-cell can be expected as an agent for treating viral diseases or cancers.

Natural immune system has been considered due to nonspecific phagocytosis. However, the presence of Toll-like receptor (TLR) is confirmed, and activation of the natural immune is found to be mainly done via TLR. Once TLR recognizes ligands, it induces inflammatory cytokines such as IL-12, TNF, etc. As IL-12 induces naive T cells into Th1 cells, ligands of TLR have a function as a Th1/Th2 differentiation controlling agent, the ligands are expected as a prophylaxis or therapeutic agent for immune diseases. In fact it is known that Th2 cells are dominant in the patients suffering from asthma or atopic dermatitis, and DNA (CpG DNA) derived from microorganism, TLR9 agonist is testing as an agent for treatment of asthma in the clinical trial and asthma-targeted clinical trials are carried out for DNA (CpGDNA) derived from microorganism, TLR9 agonist. It is also known that imidazoquinoline derivatives, TLR7/8 agonist (See Patent Document 1) show an activity suppressing the production of interleukin 4 (IL-4) and interleukin 5 (IL-5), Th2 cytokines, and in fact are effective for treatment of allergic diseases in animal model.

On the other hand, compounds having an adenine skeleton and effective for treatment of immune diseases such as viral diseases or allergic diseases are disclosed in following patent documents 2 to 4.
[Patent Document 1] US Patent 4,689,338
[Patent Document 2] WO 98/01448
[Patent Document 3] WO 99/28321
[Patent Document 4] WO 04/029054

### DISCLOSURE OF INVENTION

The problem to be solved by the present invention is to provide TLR activating agents, in more detail, the novel adenine compounds having TLR7 activity, an immune modulator containing them as an active ingredient, such as prophylactic or therapeutic agents for allergic diseases such as asthma, COPD, allergic rhinitis, allergic conjunctivitis or atopic dermatosis, viral diseases such as hepatitis B virus, hepatitis C virus, HIV or human papilloma virus (HPV), bacterial infectious diseases, cancers or dermatosis.

The present inventors earnestly investigated in order to find a therapeutic or prophylactic agent for allergic diseases, viral diseases or cancers, having excellent TLR activity and succeeded in finding a novel adenine compound of the present invention. Namely the compound of the present invention is useful for therapeutic or prophylactic agent of allergic diseases, viral diseases or cancers.

Thus the present invention has been completed based on the above findings.

Namely the present invention relates to the following invention or embodiments.
[1] An adenine compound represented by the following formula (1): 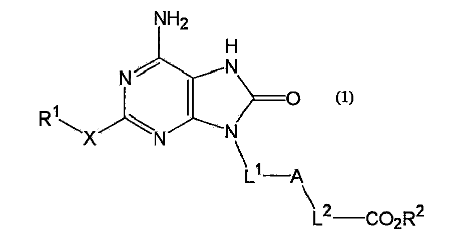 [wherein R¹ is optionally substituted alkyl group, optionally substituted alkenyl group, optionally substituted alkynyl group, optionally substituted cycloalkyl group, optionally substituted aryl group or optionally substituted heteroaryl group;
   R2 is hydrogen atom, optionally substituted alkyl group, optionally substituted alkenyl group, optionally substituted alkynyl group or optionally substituted cycloalkyl group;
   X is oxygen atom, sulfur atom, NR⁴ (wherein R⁴ is hydrogen atom or C₁₋₆ alkyl group), SO, SO₂ or a single bond;
   A is optionally substituted and saturated or unsaturated 4 to 8 membered heterocyclic group containing 1 to 2 hetero atoms selected from 1 to 2 nitrogen atoms, 0 to 1 oxygen atom and 0 to 1 sulfur atom; and
   L¹ and L² are independently, straight or branched chain alkylene or a single bond and one to three methylene groups in said alkylene may be substituted by oxygen atom, sulfur atom, NR⁵ (wherein R⁵ is hydrogen atom or alkyl group.), SO, SO₂ or carbonyl group.] or its pharmaceutically acceptable salt.
[2] The adenine compound described in above [1] or its pharmaceutically acceptable salt,
   wherein substituted alkyl group, substituted alkenyl group, substituted alkynyl group and substituted cycloalkyl group in R² are substituted by one or more substituents independently selected from:
   the group consisting of halogen atom, hydroxy group, carboxy group, mercapt group, C₁₋₆ alkoxy group, C₁₋₆ haloalkoxy group, C₂₋₆ alkoxycarbonyl group, C₁₋₆ alkylsulfonyl group, C₁₋₆ alkylsulfinyl group, C₂₋₆ alkylcarbonyloxy group, C₁₋₆ alkylthio group, optionally substituted amino group, optionally substituted carbamoyl group, optionally substituted sulfamoyl group and 3 to 8 membered cycloalkyl group (said cycloalkyl group may be substituted by halogen atom, hydroxy group, carboxy group, C₁₋₄ alkyl group or C₁₋₄ alkoxy group.),
   substituents of the above substituted amino group, substituted carbamoyl group and substituted sulfamoyl group are one or two substituents independently selected from the group consisting of the following group (a'), or a substituent (b'):
   (a') C₁₋₆ alkyl group, C₂₋₆ alkenyl group, C₂₋₆ alkynyl group, C₂₋₆ alkylcarbonyl group, C₂₋₆ alkoxycarbonyl group, C₁₋₆ alkylsulfonyl group, C₁₋₆ alkylsulfinyl group, 3 to 8 membered cycloalkyl group, 3 to 8 membered cycloalkylcarbonyl group, 3 to 8 membered cycloalkoxycarbonyl group, 3 to 8 membered cycloalkylsulfonyl group and 3 to 8 membered cycloalkylsulfinyl group (wherein the group in this group may be substituted by halogen atom, hydroxy group, C₁₋₆ alkoxy group, carboxy group or C₂₋₅ alkoxycarbonyl group.);
   (b') 4 to 7 membered saturated heterocyclic group having one to two hetero atoms selected from 1 to 2 nitrogen atoms, 0 to 1 oxygen atom and 0 to 1 sulfur atom which is formed by combining two substituents with the nitrogen atom (said saturated heterocyclic group may be substituted on its carbon atom or nitrogen atom, if chemically stable, by halogen atom, hydroxy group, carboxy group, C₁₋₆ alkyl group, C₁₋₆ alkoxy group, C₂₋₆ alkoxycarbonyl group or C₂₋₆ alkylcarbonyl group.),

   A may be substituted by one or more substituents independently select from the group consisting of halogen atom, hydroxy group, carboxy group, C₁₋₆ alkyl group, C₁₋₆ alkoxy group, C₂₋₆ alkoxycarbonyl group, C₂₋₆ alkylcarbonyl group, C₁₋₆ alkylsulfonyl group and C₁₋₆ alkylsulfinyl group,
   substituted alkyl group, substituted alkenyl group and substituted alkynyl group in R¹ are substituted by one or more substituents independently selected from the group consisting of (a) to (c) below:
   (a) halogen atom, hydroxy group, carboxy group, C₁₋₆ haloalkoxy group, and mercapt group;
   (b) C₁₋₆ alkoxy group, C₁₋₆ alkylthio group, C₂₋₆ alkylcarbonyl group, C₂₋₆ alkylcarbonyloxy group, C₁₋₆ alkylsulfonyl group, C₁₋₆ alkylsulfinyl group, and C₂₋₆ alkoxycarbonyl group (wherein the group of this group may be substituted by one or more substituents independently selected from the group consisting of halogen atom, hydroxy group, carboxy group, C₁₋₆ alkoxy group, C₂₋₆ alkoxycarbonyl group, amino group optionally substituted by the same or different and one or two C₁₋₆ alkyl groups, carbamoyl group optionally substituted by the same or different and one or two C₁₋₆ alkyl groups, sulfamoyl group optionally substituted by the same or different and one or two C₁₋₆ alkyl groups and C₁₋₆ alkylsulfonyl group.);
   (c) optionally substituted amino group, optionally substituted carbamoyl group and optionally substituted sulfamoyl group (wherein the group of this group may be substituted by one or more substituents selected from groups (j), (k) and (l) below), optionally substituted 3 to 8 membered cycloalkyl group and optionally substituted 4 to 8 membered saturated heterocyclic group (wherein the group of this group may be substituted by one or more substituents selected from groups (d), (e) and (f) below), and optionally substituted 6 to 10 membered aryl group, optionally substituted 5 to 10 membered heteroaryl group, optionally substituted 6 to 10 membered aryloxy group, and optionally substituted 5 to 10 membered heteroaryloxy group (wherein the group of this group may be substituted by one or more substituents selected from the group consisting of (g), (h) and (i) below);
      cycloalkyl group in R¹ may be substituted by one or more substituents independently selected from the group consisting of (d) to (f) below:
   (d) halogen atom, hydroxy group, carboxy group, mercapt group, C₁₋₆ haloalkyl group and C₁₋₆ haloalkoxy group;
   (e) C₁₋₆ alkyl group, C₂₋₆ alkenyl group, C₂₋₆ alkynyl group, C₁₋₆ alkoxy group, C₂₋₆ alkylcarbonyl group, C₂₋₆ alkylcarbonyloxy group, C₂₋₆ alkoxycarbonyl group, C₁₋₆ alkylthio group, C₁₋₆ alkylsulfonyl group, and C₁₋₆ alkylsulfinyl group (wherein the group of this group may be substituted by one or more substituents independently selected from the group consisting of halogen atom, hydroxy group, carboxy group, C₁₋₆ alkoxy group, C₂₋₆ alkoxycarbonyl group, amino group optionally substituted by the same or different and one or two C₁₋₆ alkyl groups, carbamoyl group optionally substituted by the same or different and one or two C₁₋₆ alkyl groups, sulfamoyl group optionally substituted by the same or different and one or two C₁₋₆ alkyl groups and C₁₋₆ alkylsulfonyl group.);
   (f) optionally substituted amino group, optionally substituted carbamoyl group and optionally substituted sulfamoyl group (wherein the group of this group may be substituted by one or two substituents selected from the group consisting of (j), (k) and (l) below), and optionally substituted 6 to 10 membered aryl group and optionally substituted 5 to 10 membered heteroaryl group (the group of this group may be substituted by one or more substituents selected from the group consisting of (g), (h) and (i) below);
      substituted aryl group and substituted heteroaryl group in R¹ are substituted by one or more substituents independently selected from the group consisting of (g) to (i) below:
   (g) halogen atom, hydroxy group, mercapt group, cyano group, nitro group, C₁₋₆ haloalkyl group, and C₁₋₆ haloalkoxy group;
   (h) C₁₋₆ alkyl group, C₂₋₆ alkenyl group, C₂₋₆ alkynyl group, C₁₋₆ alkoxy group, C₂₋₆ alkylcarbonyl group, C₂₋₆ alkoxycarbonyl group, C₂₋₆ alkylcarbonyloxy group, C₁₋₆ alkylthio group, C₁₋₆ alkylsulfonyl group, C ₁₋₆ alkylsulfinyl group, 3 to 8 membered cycloalkyl group and 4 to 8 membered saturated heterocyclic group (wherein the group of this group may be substituted by one or more substituents independently selected from a group consisting of halogen atom, hydroxy group, carboxy group, C₁₋₆ alkyl group, C₁₋₆ alkoxy group, C₂₋₆ alkoxycarbonyl group, amino group optionally substituted by the same or different and one or two C₁₋₆ alkyl groups, carbamoyl group optionally substituted by the same or different and one or two C₁₋₆ alkyl groups, sulfamoyl group optionally substituted by the same or different and one or two C₁₋₆ alkyl groups and C ₁₋₆ alkylsulfonyl group.);
   (i) optionally substituted amino group, optionally substituted carbamoyl group, and optionally substituted sulfamoyl group (the group of this group may be substituted by one or two substituents selected from the group consisting of (j), (k) and (1) below);
      substituted amino group, substituted carbamoyl group and substituted sulfamoyl group in the above (a) to (i) are substituted by one or two substituents independently selected from the group consisting of (j) to (l) below:
   (j) C₁₋₆ alkyl group, C₂₋₆ alkenyl group, C₂₋₆ alkynyl group, C₂₋₆ alkylcarbonyl group, C₂₋₆ alkoxycarbonyl group, C₁₋₆ alkylsulfonyl group, C ₁₋₆ alkylsulfinyl group, 3 to 8 membered cycloalkyl group, 3 to 8 membered cycloalkylcarbonyl group, 3 to 8 membered cycloalkoxycarbonyl group, 3 to 8 membered cycloalkylsulfonyl group and 3 to 8 membered cycloalkylsulfinyl group (wherein the group of this group may be substituted by one or more substituents independently selected from the group consisting of halogen atom, hydroxy group, carboxy group, C₁₋₆ alkyl group, C₁₋₆ alkoxy group, C₂₋₆ alkoxycarbonyl group, amino group optionally substituted by the same or different and one or two C₁₋₆ alkyl groups, carbamoyl group optionally substituted by the same or different and one or two C₁₋₆ alkyl groups, sulfamoyl group optionally substituted by the same or different and one or two C₁₋₆ alkyl groups and C₁₋₆ alkylsulfonyl group.);
   (k) 6 to 10 membered aryl group, 6 to 10 membered arylcarbonyl group, 6 to 10 membered aryloxycarbonyl group, 6 to 10 membered arylsulfonyl group, 6 to 10 membered arylsulfinyl group, 5 to 10 membered heteroaryl group, 5 to 10 membered heteroarylcarbonyl group, 5 to 10 membered heteroaryloxycarbonyl group, 5 to 10 membered heteroarylsulfonyl group, and 5 to 10 membered heteroarylsulfinyl group (wherein the group of this group may be substituted by halogen atom, hydroxy group, C₁₋₆ alkyl group, C₁₋₆alkoxy group, amino group optionally substituted by the same or different and one or two C₁₋₆ alkyl groups, carbamoyl group optionally substituted by the same or different and one or two C₁₋₆ alkyl groups, sulfamoyl group optionally substituted by the same or different and one or two C₁₋₆ alkyl groups, or C₁₋₆ alkylsulfonyl group.);
   (l) 4 to 7 membered saturated heterocyclic group containing 1 or 2 hetero atoms selected from 1 to 2 nitrogen atoms, 0 to 1 oxygen atom, and 0 to 1 sulfur atom which is formed by combining two substituents with the nitrogen atom (said saturated heterocyclic group may be substituted on appropriate carbon atom or nitrogen atom, if chemically stable, by halogen atom, hydroxy group, carboxy group, C₁₋₆ alkyl group, C₁₋₆ alkoxy group, C₂₋₆ alkoxycarbonyl group, C₂₋₆ alkylcarbonyl group, amino group which may be substituted by the same or different and one or two C₁₋₆ alkyl groups, carbamoyl group which may be substituted by the same or different and one or two C₁₋₆ alkyl groups, sulfamoyl group which may be substituted by the same or different and one or two C₁₋₆ alkyl groups, or C₁₋₆ alkylsulfonyl group.).
[3] The adenine compound or its pharmaceutically acceptable salt described in the above [1] or [2], wherein in the formula (1), A is pyrrolidine, piperidine, piperazine, morpholine, thiomorpholine, thiomorpholine-1-oxide or thiomorphohne-1,1-dioxide.
[4] The adenine compound or its pharmaceutically acceptable salt described in any one of the above [1] to [3], wherein in the formula (1), R² is C ₁₋₄ alkyl group.
[5] The adenine compound or its pharmaceutically acceptable salt described in the above [4], wherein in the formula (1), R² is methyl group.
[6] The adenine compound or its pharmaceutically acceptable salt described in any one of the above [1] to [3] wherein in the formula (1), R² is C ₂₋₆ alkyl group substituted by optionally substituted amino group.
[7] The adenine compound or its pharmaceutically acceptable salt described in any one of the above [1] to [6], wherein in the formula (1), L¹ is a following formula:

   (CH₂)ₙ-(Y¹)ₘ-(CH₂)₁ₐ

   [wherein Y¹ is oxygen atom or NR^{5'} (wherein R^{5'} is hydrogen atom or C₁₋₆ alkyl group.), n and la are independently an integer of 0 to 5, and m is 0 or 1.],
   and L² is a single bond or straight chained C₁₋₄ alkylene.
[8] The adenine compound or its pharmaceutically acceptable salt described in the above [1] selected from the group of the following compounds:
   2-Butoxy-7,8-dihydro-9-[5-(4-methoxycarbonylpiperidin-1-yl)pentyl]-8-oxoadenine;
   2-Butoxy-7,8-dihydro-9-[5-(4-methoxycarbonylmethylpiperidin-1-yl)pentyl]-8-oxoadenine;
   2-Butoxy-7,8-dihydro-9-[5-(3-methoxycarbonylmethylpiperidin-1-yl)pentyl]-8-oxoadenine;
   2-Butoxy-7,8-dihydro-9-[2-(4-methoxycarbonylpiperidin-1-yl)ethyl]-8-oxoadenine;
   2-Butoxy-7,8-dihydro-9-[2-(3-methoxycarbonylpiperidin-1-yl)ethyl]-8-oxoadenine;
   2-Butoxy-7,8-dihydro-9-{2-(2-methoxycarbonylpiperidin-1-yl)ethyl}-8-oxoadenine;
   2-Butoxy-7,8-dihydro-9-[2-{4-(2-methoxycarbonylethyl)piperidin-1-yl}ethyl]-8-oxoadenine;
   2-Butoxy-7,8-dihydro-9-[3-(4-methoxycarbonylpiperidin-1-yl)propyl]-8-oxoadenine;
   2-Butoxy-7,8-dihydro-9-[3-(3-methoxycarbonylpiperidin-1-yl)propyl]-8-oxoadenine;
   2-Butoxy-7,8-dihydro-9-[3-(2-methoxycarbonylpiperidin-1-yl)propyl]-8-oxoadenine;
   2-Butoxy-7,8-dihydro-9-{3-[4-(2-methoxy-2-oxoethyl)piperidin-1-yl]propyl}-8-oxoadenine;
   2-Butoxy-7,8-dihydro-9-{3-[3-(2-methoxy-2-oxoethyl)piperidin-1-yl]propyl}-8-oxoadenine;
   2-Butoxy-7,8-dihydro-9-{3-[4-(3-methoxy-3-oxopropyl)piperazin-1-yl]propyl}-8-oxoadenine;
   2-Butoxy-7,8-dihydro-9-{2-[2-(4-methoxycarbonylpipehdm-1-yl)ethoxy]ethyl}-8-oxoadenine;
   2-Butoxy-7,8-dihydro-9-[6-(4-methoxycarbonylpiperidin-1-yl)hexyl]-8-oxoadenine;
   2-Butoxy-7,8-dihydro-9-[7-(4-methoxycarbonylpiperidin-1-yl)heptyl]-8-oxoadenine;
   2-Butoxy-7,8-dihydro-9-[8-(4-methoxycarbonylpiperidin-1-yl)octyl]-8-oxoadenine;
   2-Butoxy-7,8-dihydro-9-[4-(4-methoxycarbonylpiperidin-1-yl)butyl]-8-oxoadenine;
   2-Butoxy-7,8-dihydro-9-[4-(3-methoxycarbonylpiperidin-1-yl)butyl]-8-oxoadenine;
   2-Butoxy-7,8-dihydro-9-[4-(2-methoxycarbonylpiperidin-1-yl)butyl]-8-oxoadenine;
   2-Butoxy-7,8-dihydro-9-{4-[(4-methoxycarbonylmethyl)piperazin-1-yl]butyl}-8-oxoadenine;
   2-Butoxy-7,8-dihydro-9-{4-[4-(2-methoxycarbonylethyl)piperazin-1-yl]butyl}-8-oxoadenine;
   2-Butoxy-7,8-dihydro-9-[4-(4-methoxycarbonylinethylpiperidin-1-yl)butyl]-8-oxoadenine;
   2-Butoxy-7,8-dihydro-9-[4-(3-methoxycarbonylmethylpiperidin-1-yl)butyl]-8-oxoadenine;
   2-Butoxy-7,8-dihydro-9-(4-methoxycarbonylmethylmorpholin-2-ylmethyl)-8-oxoadenine;
   2-Butoxy-7,8-dihydro-9-(1-methoxycarbonylmethylpiperidin-4-ylmethyl)-8-oxoadenine;
   2-Butoxy-7,8-dihydro-9-[3-(1-methoxycarbonylmethylpiperidin-4-yloxy)propyl]-8-oxoadenine;
   2-Butoxy-7,8-dihydro-9-{3-[1-(2-methoxy-2-oxoethyl)piperidin-4-ylmethyloxy]propyl}-8-oxoadenine;
   2-Butoxy-7,8-dihydro-9-(3-{1-[4-(dimethylamino)propoxycarbonylmethyl]piperidin-4-ylmethoxy}propyl)-8-oxoadenine;
   2-Butoxy-7,8-dihydro-9-(1-methoxycarbonylmethylpiperidin-3-ylmethyl)-8-oxoadenine;
   2-Butoxy-7,8-dihydro-9-[1-(3-methoxy-3-oxopropyl)piperidin-3-ylmethyl]-8-oxoadenine;
   2-Butoxy-7,8-dihydro-9-[2-(1-methoxycarbonylmethylpiperidin-4-yl)ethyl]-8-oxoadenine;
   2-Butoxy-7,8-dihydro-9-{2-[1-(3-methoxy-3-oxopropyl)piperidin-4-yl]ethyl}-8-oxoadenine;
   2-Butoxy-7,8-dihydro-9-[2-(1-methoxycarbonylmethylpiperidin-2-yl)ethyl]-8-oxoadenine;
   2-Butoxy-7,8-dihydro-9-{[1-(3-methoxy-3-oxopropyl)piperidin-4-yl]methyl}-8-oxoadenine;
   2-Butoxy-7,8-dihydro-9-{2-[4-(2-methoxy-2-oxoethyl)morpholin-2-yl]ethyl}-8-oxoadenine;
   2-Butoxy-7,8-dihydro-9-{5-(4-hydroxycarbonylpiperidin-1-yl)pentyl}-8-oxoadenine;
   2-Butoxy-7,8-dihydro-9-[5-(4-hydroxycarbonylinethylpiperidin-1-yl)pentyl]-8-oxoadenine;
   2-Butoxy-7,8-dilxydro-9-[5-(3-hydroxycarbonylmethylpiperidin-1-yl)pentyl]-8-oxoadenine;
   2-Butoxy-7,8-dihydro-9-{2-(4-hydroxycarbonylpiperidin-1-yl)ethyl}-8-oxoadenine;
   2-Butoxy-7,8-dihydro-9-[2-(3-hydroxycarbonylpiperidm-l-yl)ethyl]-8-oxoadenine;
   2-Butoxy-7,8-dihydro-9-{2-(2-hydroxycarbonylpiperidin-1-yl)ethyl}-8-oxoadenine;
   2-Butoxy-7,8-dihydro-9-[2-{4-(2-carboxyethyl)piperidin-1-yl}ethyl]-8-oxoadenine;
   2-Butoxy-7,8-dihydro-9-[3-(4-hydroxycarbonylpiperidin-1-yl)propyl]-8-oxoadenine;
   2-Butoxy-7,8-dihydro-9-[3-(3-hydroxycarbonylpiperidin-1-yl)propyl]-8-oxoadenine;
   2-Butoxy-7,8-dihydro-9-[3-(2-hydroxycarbonylpiperidin-1-yl)propyl]-8-oxoadenine;
   2-Butoxy-7,8-dihydro-9-{3-[4-(2-hydroxy-2-oxoethyl)piperidin-1-yl]propyl}-8-oxoadenine;
   2-Butoxy-7,8-dihydro-9-{3-[3-(2-hydroxy-2-oxoethyl)piperidin-1-yl]propyl}-8-oxoadenine;
   2-Butoxy-7,8-dihydro-9-{3-[4-(3-hydroxy-3-oxopropyl)pyperazin-1-yl]propyl}-8-oxoadenine;
   2-Butoxy-7,8-dihydro-9-{2-[2-(4-hydroxycarbonylpiperidin-1-yl) ethoxy]ethyl}-8-oxoadenine;
   2-Butoxy-7,8-dihydro-9-{6-(4-hydroxycarbonylpiperidin-1-yl)hexyl}-8-oxoadenine;
   2-Butoxy-7,8-dihydro-9-{7-(4-hydroxycaxbonylpiperidin-1-yl)heptyl}-8-oxoadenine;
   2-Butoxy-7,8-dihydro-9-{8-(4-hydroxycarbonylpiperidin-1-yl)octyl}-8-oxoadenine;
   2-Butoxy-7,8-dihydro-9-{4-(4-hydroxycarbonylpiperidin-1-yl)butyl}-8-oxoadenine;
   2-Butoxy-7,8-dihydro-9-{4-(3-hydroxycarbonylpiperidin-1-yl)butyl}-8-oxoadenine;
   2-Butoxy-7,8-dihydro-9-{4-(2-hydroxycarbonylpiperidin-1-yl)butyl}-8-oxoadenine;
   2-Butoxy-7,8-dihydro-9-{4-[(4-hydroxycarbonylmethyl)piperazin-1-yl]butyl}-8-oxoadenine;
   2-Butoxy-7,8-dihydro-9-{4-[4-(2-hydroxycarbonylethyl)piperazin-1-yl]butyl}-8-oxoadenine;
   2-Butoxy-7,8-dihydro-9-[4-(4-hydroxycarbonylmethylpiperidin-1-yl)butyl]-8-oxoadenine;
   2-Butoxy-7,8-dihydro-9-[4-(3-hydroxycarbonylmethylpiperidin-1-yl)butyl]-8-oxoadenine;
   2-Butoxy-7,8-dihydro-9-[3-(1-hydroxycarbonylmethylpiperidin-4-yloxy)propyl]-8-oxoadenine;
   2-Butoxy-7,8-dihydro-9-{3-[1-(2-hydroxy-2-oxoethyl)piperidin-4-ylmethyloxy]propyl}-8-oxoadenine;
   2-Butoxy-7,8-dihydro-9-(1-hydroxycarbonylmethylpiperidin-3-ylmethyl)-8-oxoadenine;
   2-Butoxy-7,8-dihydro-9-{[1-(3-hydroxy-3-oxopropyl)piperidin-4-yl]methyl}-8-oxoadenine;
   2-Butoxy-7,8-dihydro-9-{2-[(R)-2-methoxycarbonylpyrrolidin-1-yl]ethyl}-8-oxoadenine;
   2-Butoxy-7,8-dihydro-9-{2-[(S)-2-methoxycarbonylpyrrolidin-1-yl]ethyl}-8-oxoadenine;
   2-Butoxy-7,8-dihydro-9-{3-[(S)-2-t-butoxycarbonylpyrrolidin-1-yl]propyl}-8-oxoadenine;
   2-Butoxy-7,8-dihydro-9-{3-[(S)-2-methoxycarbonylpyrrolidin-1-yl]propyl}-8-oxoadenine;
   2-Butoxy-7,8-dihydro-9-{3-[(S)-2-carboxypyrrolidin-1-yl]propyl}-8-oxoadenine;
   2-Butoxy-7,8-dihydro-9-{4-[(S)-2-methoxycarbonylpyrrolidin-1-yl]butyl}-8-oxoadenine;
   2-Butoxy-7,8-dihydro-9-{4-[(S)-2-methoxycarbonylpyrrolidin-1-yl]butyl}-8-oxoadenine fumarate and
   2-Butoxy-7,8-dihydro-9-[2-(4-methoxycarbonylmethylpiperazin-1-yl)ethyl]-8-oxoadenine.
[9] A pharmaceutical composition containing the adenine compound or a pharmaceutically acceptable salt thereof as described in any one of the above [1] to [8] as an active ingredient.
[10] A TLR7 activator containing the adenine compound or a pharmaceutically acceptable salt thereof as described in any one of the above [1] to [8] as an active ingredient.
[11] An immuno-modifier containing the adenine compound or a pharmaceutically acceptable salt thereof as described in any one of the above [1] to [8] as an active ingredient.
[12] A therapeutic or prophylactic agent for allergic diseases, viral diseases or cancers containing the adenine compound or a pharmaceutically acceptable salt thereof as described in any one of the above [1] to [8] as an active ingredient.
[13] A therapeutic or prophylactic agent for asthma, COPD, allergic rhinitis, allergic conjunctivitis, atopic dermatosis, cancer, hepatitis B virus, hepatitis C virus, HIV, HPV, a bacterial infectious disease, or dermatosis containing the adenine compound or a pharmaceutically acceptable salt thereof as described in any one of the above [1] to [8] as an active ingredient.
[14] A medicament for topical administration containing the adenine compound or a pharmaceutically acceptable salt thereof as described in any one of the above [1] to [8] as an active ingredient.

### EFFECT OF THE INVENTION

According to the present invention it is possible to provide a novel adenine compound useful as a prophylactic or therapeutic agent for allergic diseases, viral diseases or cancers.

### THE BEST MODE FOR CARRYING OUT THE INVENTION

The present invention is described in detail below.

"Halogen atom" in the present specification includes fluorine atom, chlorine atom, bromine atom, or iodine atom, preferably fluorine atom or chlorine atom.

"Alkyl group" includes C₁₋₁₀ straight or branched chain alkyl group, such as methyl group, ethyl group, propyl group, 1-methylethyl group, butyl group, 2-methylpropyl group, 1-methylpropyl group, 1,1-dimethylethyl group, pentyl group, 3-methylbutyl group, 2-methylbutyl group, 2,2-dimethylpropyl group, 1-ethylpropyl group, 1,1-dimethylpropyl group, hexyl group, 4-methylpentyl group, 3-methylpentyl group, 2-methylpentyl group, 1-methylpentyl group, 3,3-dimethylbutyl group, 2,2-dimethylbutyl group, 1,1-dimethylbutyl group, 1,2-dimethylbutyl group, heptyl group, 1-methylhexyl group, 1-ethylpentyl group, octyl group, 1-methylheptyl group, 2-ethylhexyl group, nonyl group, decyl group, etc., preferably C₁₋₆ alkyl group, more preferably C₁₋₄ alkyl group.

"Alkenyl group" includes C₂₋₁₀ straight or branched chain alkenyl group, such as ethenyl group, propenyl group, 1-methylethenyl group, butenyl group, 2-methylpropenyl group, 1-methylpropenyl group, pentenyl group, 3-methylbutenyl group, 2-methylbutenyl group, 1-ethylpropenyl group, hexenyl group, 4-methylpentenyl group, 3-methylpentenyl group, 2-methylpentenyl group, 1-methylpentenyl group, 3,3-dimethylbutenyl group, 1,2-dimethylbutenyl group, heptenyl group, 1-methylhexenyl group, 1-ethylpentenyl group, octenyl group, 1-methylheptenyl group, 2-ethylhexenyl group, nonenyl group, decenyl group, etc., preferably C₂₋₆ alkenyl group, more preferably C₂₋₄ alkenyl group.

"Alkynyl group" includes C₂₋₁₀ straight or branched chain alkynyl group, such as ethynyl group, propynyl group, butynyl group, pentynyl group, 3-methylbutynyl group, hexynyl group, 4-methylpentynyl group, 3-methylpentynyl group, 3,3-dimethylbutynyl group, heptynyl group, octynyl group, 3-methylheptynyl group, 3-ethylhexynyl group, nonynyl group, decynyl group, etc., preferably C ₂₋₆ alkynyl group, more preferably, C ₂₋₄ alkynyl group.

"Cycloalkyl group" includes 3 to 8 membered monocyclic cycloalkyl group, such as cyclopropyl group, cyclobutyl group, cyclopentyl group, cyclohexyl group, cycloheptyl group, or cyclooctyl group.

"Cycloalkoxy group" includes 3 to 8 membered monocyclic cycloalkoxy group, such as cyclopropyloxy group, cyclobutyloxy group, cyclopentyloxy group, cyclohexyloxy group, cycloheptyloxy group, or cyclooctyloxy group.

"Aryl group" includes 6 to 10 membered aryl group, such as phenyl group, 1-naphthyl group, or 2-naphthyl group.

"Heteroaryl group" includes 5 to 10 membered monocyclic or bicyclic heteroaryl group containing 1 to 4 hetero atoms selected from 0 to 3 nitrogen atoms, 0 to 1 oxygen atom and 0 to 1 sulfur atom, such as furyl group, thienyl group, pyrrolyl group, pyridyl group, indolyl group, isoindolyl group, quinolyl group, isoquinolyl group, pyrazolyl group, imidazolyl group, pyrimidinyl group, pyrazinyl group, pyridazinyl group, thiazolyl group, oxazolyl group, etc. The binding site in the heteroaryl group is not specifically limited and it may be on any of nitrogen or carbon atoms, if chemically stable.

"Saturated heterocyclic group" includes 4 to 10 membered mono or bicyclic saturated heterocyclic group containing 1 to 3 hetero atoms selected from 0 to 3 nitrogen atoms, 0 to 1 oxygen atom and 0 to 1 sulfur atom and said sulfur atom may be substituted by one or two oxygen atoms, such as pyrrolidinyl group, piperidinyl group, piperazinyl group, morpholinyl group, thiomorpholinyl group, 1-oxothiomorpholinyl group, 1,1-dioxothiomorpholinyl group, tetrahydrofuranyl group, oxazolydinyl group, etc. The binding site on the heterocyclic group is not specifically limited and it may be on any of nitrogen or carbon atoms, if chemically stable. 4 to 8 Membered monocyclic saturated heterocyclic group is preferably illustrated.

"Alkylene" includes straight or branched chain C₁₋₁₀ alkylene, such as methylene, ethylene, trimethylene, tetramethylene, pentamethylene, hexamethylene, heptamethylene, octamethylene, nonamethylene, decamethylene, 1-methylmethylene, 1-ethylmethylene, 1-propylmethylene, 1-methylethylene, 2-methylethylene, 1-methyltrimethylene, 2-methyltrimethylene, 2-methyltetramethylene, 3-methylpentamethylene, etc., preferably C₁₋₆ alkylene.

"Haloalkyl group" includes C₁₋₆ alkyl group substituted by the same or different and 1 to 5 halogen atoms, such as trifluoromethyl group, 2,2,2-trifluoroethyl group, 2,2-difluoroethyl group, pentafluoroethyl group, etc.

"Alkoxy group" includes C₁₋₁₀ straight or branched chain alkoxy group, for example methoxy group, ethoxy group, propoxy group, 1-methylethoxy group, butoxy group, 2-methylpropoxy group, 1-methylpropoxy group, 1,1-dimethylethoxy group, pentoxy group, 3-methylbutoxy group, 2-methylbutoxy group, 2,2-dimethylpropoxy group, 1-ethylpropoxy group, 1,1-dimethylpropoxy group, hexyloxy group, 4-methylpentyloxy group, 3-methylpentyloxy group, 2-methylpentyloxy group, 1-methylpentyloxy group, 3,3-dimethylbutoxy group, 2,2-dimethylbutoxy group, 1,1-dimethylbutoxy group, 1,2-dimethylbutoxy group, heptyloxy group, 1-methylhexyloxy group, 1-ethylpentyloxy group, octyloxy group, 1-methylheptyloxy group, 2-ethylhexyloxy group, nonyloxy group, decyloxy group, etc, preferably C₁₋₆ alkoxy group, more preferably C₁₋₄ alkoxy group.

"Haloalkoxy group" included C₁₋₆ alkoxy group substituted by the same or different and 1 to 5 halogen atoms, such as trifluoromethoxy group, 2,2,2-trifluoroethoxy group, 2,2-difluoroethoxy group, 2-fluoroethoxy, pentafluoroethoxy group, etc.

"Alkylthio group" includes straight or branched chain C₁₋₁₀ alkylthio group, such as methylthio group, ethylthio group, propylthio group, 1-methylethylthio group, butylthio group, 2-methylpropylthio group, 1-methylpropylthio group, 1,1-dimethylethylthio group, pentylthio group, 3-methylbutylthio group, 2-methylbutylthio group, 2,2-dimethylpropylthio group, 1-ethylpropylthio group, 1,1-dimethylpropylthio group, hexylthio group, 4-methylpentylthio group, 3-methylpentylthio group, 2-methylpentylthio group, 1-methylpentylthio group, 3,3-dimethylbutylthio group, 2,2-dimethylbutylthio group, 1,1-dimethylbutylthio group, 1,2-dimethylbutylthio group, heptylthio group, 1-methylhexylthio group, 1-ethylpentylthio group, octylthio group, 1-methylheptylthio group, 2-ethylhexylthio group, nonylthio group, decylthio group, etc., preferably C₁₋₆ alkylthio group, more preferably C₁₋₄ alkylthio group.

"Alkyl moiety" in "alkylcarbonyl group", "alkylcarbonyloxy group", "alkylsulfonyl group" or "alkylsulfinyl group" includes the same as the alkyl group as mentioned above.

"Alkylcarbonyl group" includes such as acetyl group, propanoyl group, butanoyl group, 2-methylpropanoyl group, pentanoyl group, 3-methylbutanoyl group, 2-methylbutanoyl group, 2,2-dimethylpropanoyl (pivaloyl) group, hexanoyl group, 4-methylpentanoyl group, 3-methylpentanoyl group, 2-methylpentanoyl group, 3,3-dimethylbutanoyl group, 2,2-dimethylbutanoyl group, heptanoyl group, octanoyl group, 2-ethylhexanoyl group, nonanoyl group, decanoyl group, etc., preferably C ₂₋₆ alkylcarbonyl group, more preferably, straight or branched chain C₂₋₅ alkylcarbonyl group.

"Alkylcarbonyloxy group" includes such as acetoxy group, propanoyloxy group, butanoyloxy group, 2-methylpropanoyloxy group, pentanoyloxy group, 3-methylbutanoyloxy group, 2-methylbutanoyloxy group, 2,2-dimethylpropanoyloxy (pivaloyloxy) group, hexanoyloxy group, 4-methylpentanoyloxy group, 3-methylpentanoyloxy group, 2-methylpentanoyloxy group, 3,3-dimethylbutanoyloxy group, 2,2-dimethylbutanoyloxy group, heptanoyloxy group, octanoyloxy group, 2-ethylhexanoyloxy group, nonanoyloxy group, decanoyloxy group, etc., preferably C₂₋₆ alkylcarbonyloxy group, more preferably straight or branched chain C₂₋₅ alkylcarbonyloxy group.

"Alkylsulfonyl group" includes such as methanesulfonyl group, ethanesulfonyl group, propylsulfonyl group, 1-methylethylsulfonyl group, butylsulfonyl group, 2-methylpropylsulfonyl group, 1-methylpropylsulfonyl group, 1,1-dimethylethylsulfonyl group, pentylsulfonyl group, 3-methylbutylsulfonyl group, 2-methylbutylsulfonyl group, 2,2-dimethylpropylsulfonyl group, 1-ethylpropylsulfonyl group, 1,1-dimethylpropylsulfonyl group, hexylsulfonyl group, 4-methylpentylsulfonyl group, 3-methylpentylsulfonyl group, 2-methylpentylsulfonyl group, 1-methylpentylsulfonyl group, 3,3-dimethylbutylsulfonyl group, 2,2-dimethylbutylsulfonyl group, 1,1-dimethylbutylsulfonyl group, 1,2-dimethylbutylsulfonyl group, heptylsulfonyl group, 1-methylhexylsulfonyl group, 1-ethylpentylsulfonyl group, octylsulfonyl group, 1-methylheptylsulfonyl group, 2-ethylhexylsulfonyl group, nonylsulfonyl group, decylsulfonyl group, etc., preferably C₁₋₆ alkylsulfonyl group, more preferably straight or branched chain C₁₋₄ alkylsulfonyl group.

"Alkylsulfinyl group" includes such as methylsulfinyl group, ethylsulfinyl group, propylsulfinyl group, 1-methylethylsulfinyl group, butylsulfinyl group, 2-methylpropylsulfinyl group, 1-methylpropylsulfinyl group, 1,1-dimethylethylsulfinyl group, pentylsulfinyl group, 3-methylbutylsulfinyl group, 2-methylbutylsulfinyl group, 2,2-dimethylpropylsulfinyl group, 1-ethylpropylsulfinyl group, 1,1-dimethylpropylsulfinyl group, hexylsulfinyl group, 4-methylpentylsulfinyl group, 3-methylpentylsulfinyl group, 2-methylpentylsulfinyl group, 1-methylpentylsulfinyl group, 3,3-dimethylbutylsulfinyl group, 2,2-dimethylbutylsulfinyl group, 1,1-dimethylbutylsulfinyl group, 1,2-dimethylbutylsulfinyl group, heptylsulfinyl group, 1-methylhexylsulfinyl group, 1-ethylpentylsulfinyl group, octylsulfinyl group, 1-methylheptylsulfinyl group, 2-ethylhexylsulfinyl group, nonylsulfinyl group, decylsulfinyl group, etc., preferably C₁₋₆ alkylsulfinyl group, more preferably straight or branched chain C₁₋₄ alkylsulfinyl group.

"Alkoxy moiety" in "alkoxycarbonyl group" is the same as the alkoxy group mentioned above. Examples of "alkoxycarbonyl group" are methoxycarbonyl group, ethoxycarbonyl group, propoxycarbonyl group, 1-methylethoxycarbonyl group, butoxycarbonyl group, 2-methylpropoxycarbonyl group, 1-methylpropoxycarbonyl group, 1,1-dimethylethoxycarbonyl group, pentoxycarbonyl group, 3-methylbutoxycarbonyl group, 2-methylbutoxycarbonyl group, 2,2-dimethylpropoxycarbonyl group, 1-ethylpropoxycarbonyl group, 1,1-dimethylpropoxycarbonyl group, hexyloxycarbonyl group, 4-methylpentyloxycarbonyl group, 3-methylpentyloxycarbonyl group, 2-methylpentyloxycarbonyl group, 1-methylpentyloxycarbonyl group, 3,3-dimethylbutoxycarbonyl group, 2,2-dimethylbutoxycarbonyl group, 1,1-dimethylbutoxycarbonyl group, 1,2-dimethylbutoxycarbonyl group, heptyloxycarbonyl group, 1-methylhexyloxycarbonyl group, 1-ethylpentyloxycarbonyl group, octyloxycarbonyl group, 1-methylheptyloxycarbonyl group, 2-ethylhexyloxycarbonyl group, nonyloxycarbonyl group, decyloxycarbonyl group, etc., preferably C₂₋₆ alkoxycarbonyl group, more preferably straight or branched chain C₂₋₄ alkoxycarbonyl group.

"Alkenyl moiety" in "Alkenyloxy group", "alkenylcarbonyl group", "alkenylcarbonyloxy group", "alkenylsulfonyl group", "alkenylsulfinyl group" and "alkenyloxycarbonyl group" is the same as the alkenyl group mentioned above.

"Alkenyloxy group" includes for example, ethenyloxy group, propenyloxy group, 1-methylethenyloxy group, butenyloxy group, 2-methylpropenyloxy group, 1-methylpropenyloxy group, pentenyloxy group, 3-methylbutenyloxy group, 2-methylbutenyloxy group, 1-ethylpropenyloxy group, hexenyloxy group, 4-methylpentenyloxy group, 3-methylpentenyloxy group, 2-methylpentenyloxy group, 1-methylpentenyloxy group, 3,3-dimethylbutenyloxy group, 1,2-dimethylbutenyloxy group, heptenyloxy group, 1-methylhexenyloxy group, 1-ethylpentenyloxy group, octenyloxy group, 1-methylheptenyloxy group, 2-ethylhexenyloxy group, nonenyloxy group, decenyloxy group, etc., preferably C₂₋₆, more preferably C₂₋₅ alkenyloxy group.

"Alkenylcarbonyl group" includes such as, ethenylcarbonyl group, propenylcarbonyl group, 1-methylethenylcarbonyl group, butenylcarbonyl group, 2-methylpropenylcarbonyl group, 1-methylpropenylcarbonyl group, pentenylcarbonyl group, 3-methylbutenylcarbonyl group, 2-methylbutenylcarbonyl group, 1-ethylpropenylcarbonyl group, hexenylcarbonyl group, 4-methylpentenylcarbonyl group, 3-methylpentenylcarbonyl group, 2-methylpentenylcarbonyl group, 1-methylpentenylcarbonyl group, 3,3-dimethylbutenylcarbonyl group, 1,2-dimethylbutenylcarbonyl group, heptenylcarbonyl group, 1-methylhexenylcarbonyl group, 1-ethylpentenylcarbonyl group, octenylcarbonyl group, 1-methylheptenylcarbonyl group, 2-ethylhexenylcarbonyl group, nonenylcarbonyl group, decenylcarbonyl group, etc., preferably C₃₋₅ alkenylcarbonyl group.

"Alkenylcarbonyloxy group" includes one constituted by binding an oxygen atom to carbonyl moiety of alkenylcarbonyl group mentioned above, preferably, C₃₋₆, and more preferably C₃₋₅ alkenylcarbonyloxy group.

"Alkenylsulfonyl group" includes such as ethenylsulfonyl group, propenylsulfonyl group, 1-methylethenylsulfonyl group, butenylsulfonyl group, 2-methylpropenylsulfonyl group, 1-methylpropenylsulfonyl group, pentenylsulfonyl group, 3-methylbutenylsulfonyl group, 2-methylbutenylsulfonyl group, 1-ethylpropenylsulfonyl group, hexenylsulfonyl group, 4-methylpentenylsulfonyl group, 3-methylpentenylsulfonyl group, 2-methylpentenylsulfonyl group, 1-methylpentenylsulfonyl group, 3,3-dimethylbutenylsulfonyl group, 1,2-dimethylbutenylsulfonyl group, heptenylsulfonyl group, 1-methylhexenylsulfonyl group, 1-ethylpentenylsulfonyl group, octenylsulfonyl group, 1-methylheptenylsulfonyl group, 2-ethylhexenylsulfonyl group, nonenylsulfonyl group, decenylsulfonyl group, etc., preferably C₂₋₆, more preferably C₂₋₅ alkenylsulfonyl group.

"Alkenylsulfinyl group" includes such as ethenylsulfinyl group, propenylsulfinyl group, 1-methylethenylsulfinyl group, butenylsulfinyl group, 2-methylpropenylsulfinyl group, 1-methylpropenylsulfinyl group, pentenylsulfinyl group, 3-methylbutenylsulfinyl group, 2-methylbutenylsulfinyl group, 1-ethylpropenylsulfinyl group, hexenylsulfinyl group, 4-methylpentenylsulfinyl group, 3-methylpentenylsulfinyl group, 2-methylpentenylsulfinyl group, 1-methylpentenylsulfinyl group, 3,3-dimethylbutenylsulfinyl group, 1,2-dimethylbutenylsulfinyl group, heptenylsulfinyl group, 1-methylhexenylsulfinyl group, 1-ethylpentenylsulfinyl group, octenylsulfinyl group, 1-methylheptenylsulfinyl group, 2-ethylhexenylsulfinyl group, nonenylsulfinyl group, decenylsulfinyl group, etc., preferably C₂₋₆, more preferably C₂₋₅ *alkenylsulfinyl group.

"Alkenyloxycarbonyl group" includes such as ethenyloxycarbonyl group, propenyloxycarbonyl group, 1-methylethenyloxycarbonyl group, butenyloxycarbonyl group, 2-methylpropenyloxycarbonyl group, 1-methylpropenyloxycarbonyl group, pentenyloxycarbonyl group, 3-methylbutenyloxycarbonyl group, 2-methylbutenyloxycarbonyl group, 1-ethylpropenyloxycarbonyl group, hexenyloxycarbonyl group, 4-methylpentenyloxycarbonyl group, 3-methylpentenyloxycarbonyl group, 2-methylpentenyloxycarbonyl group, 1-methylpentenyloxycarbonyl group, 3,3-dimethylbutenyloxycarbonyl group, 1,2-dimethylbutenyloxycarbonyl group, heptenyloxycarbonyl group, 1-methylhexenyloxycarbonyl group, 1-ethylpentenyloxycarbonyl group, octenyloxycarbonyl group, 1-methylheptenyloxycarbonyl group, 2-ethylhexenyloxycarbonyl group, nonenyloxycarbonyl group, decenyloxycarbonyl group, etc., preferably C₃₋₆, and more preferably C₃₋₅ alkenyloxycarbonyl group.

"Alkynyl moiety" in "alkynyloxy group", "alkynylcarbonyl group", "alkylcarbonyloxy group", "alkynylsulfonyl group", "alkynylsulfinyl group" and "alkynyloxycarbonyl group" is the same as the alkynyl group as mentioned above.

"Alkynyloxy group" includes such as ethynyloxy group, propynyloxy group, butynyloxy group, pentynyloxy group, 3-methylbutynyloxy group, hexynyloxy group, 4-methylpentynyloxy group, 3-methylpentynyloxy group, 3,3-dimethylbutynyloxy group, heptynyloxy group, octynyloxy group, 3-methylheptynyloxy group, 3-ethylhexynyloxy group, nonynyloxy group, decynyloxy group, etc., preferably C ₂₋₅ alkynyloxy group.

"Alkynylcarbonyl group" includes such as ethynylcarbonyl group, propynylcarbonyl group, butynylcarbonyl group, pentynylcarbonyl group, 3-methylbutynylcarbonyl group, hexynylcarbonyl group, 4-methylpentynylcarbonyl group, 3-methylpentynylcarbonyl group, 3,3-dimethylbutynylcarbonyl group, heptynylcarbonyl group, octynylcarbonyl group, 3-methylheptynylcarbonyl group, 3-ethylhexynylcarbonyl group, nonynylcarbonyl group, decynylcarbonyl group, etc., preferably C₃₋₆, more preferably C₃₋₅ alkynylcarbonyl group.

"Alkynylcarbonyloxy group" includes for example, one constituted by combining an oxygen atom to carbonyl moiety of the above "alkynylcarbonyl group". Preferably C₃₋₆, and more preferably C₃₋₅ alkynylcarbonyloxy groups are illustrated.

"Alkynylsulfonyl group", includes, for example ethynylsulfonyl group, propynylsulfonyl group, butynylsulfonyl group, pentynylsulfonyl group, 3-methylbutynylsulfonyl group, hexynylsulfonyl group, 4-methylpentynylsulfonyl group, 3-methylpentynylsulfonyl group, 3,3-dimethylbutynylsulfonyl group, heptynylsulfonyl group, octynylsulfonyl group, 3-methylheptynylsulfonyl group, 3-ethylhexynylsulfonyl group, nonynylsulfonyl group, or decynylsulfonyl group, preferably C₂₋₆, more preferably C₂₋₅ alkynylsulfonyl group.

"Alkynylsulfinyl group", the following groups includes, for example ethynylsulfinyl group, propynylsulfinyl group, butynylsulfinyl group, pentynylsulfinyl group, 3-methylbutynylsulfinyl group, hexynylsulfinyl group, 4-methylpentynylsulfinyl group, 3-methylpentynylsulfinyl group, 3,3-dimethylbutynylsulfinyl group, heptynylsulfinyl group, octynylsulfinyl group, 3-methylheptynylsulfinyl group, 3-ethylhexynylsulfinyl group, nonylsulfinyl group, or decynylsulfinyl group, preferably C₂₋₆, more preferably C₂₋₅ alkynylsulfinyl group.

As "alkynyloxycarbonyl group ", the following groups are illustrated; ethynyloxycarbonyl group, propynyloxycarbonyl group, butynyloxycarbonyl group, pentynyloxycarbonyl group, 3-methylbutynyloxycarbonyl group, hexynyloxycarbonyl group, 4-methylpentynyloxycarbonyl group, 3-methylpentynyloxycarbonyl group, 3,3-dimethylbutynyloxycarbonyl group, heptynyloxycarbonyl group, octynyloxycarbonyl group, 3-methylheptynyloxycarbonyl group, 3-ethylhexynyloxycarbonyl group, nonynyloxycarbonyl group, or decynyloxycarbonyl group, preferably C₃₋₆, more preferably C₃₋₅ alkynyloxycarbonyl group.

As "cycloalkyl" in "cycloalkylcarbonyl group", "cycloalkylcarbonyloxy group", "cycloalkylsulfonyl group" and "cycloalkylsulfinyl group", the same groups as the above cycloalkyl groups are illustrated.

As "cycloalkylcarbonyl group", the following groups are illustrated; cyclopropylcarbonyl group, cyclobutylcarbonyl group, cyclopentylcarbonyl group, cyclohexylcarbonyl group, cycloheptylcarbonyl group, or cyclooctylcarbonyl group.

As "cycloalkylcarbonyloxy group" one constituted by binding an oxygen atom to carbonyl moiety of "cloalkylcarbonyl group" are illustrated. For example cyclopropylcarbonyloxy group, cyclobutylcarbonyloxy group, cyclopentylcarbonyloxy group, cyclohexylcarbonyloxy group, cycloheptylcarbonyloxy group, or cyclooctylcarbonyloxy group are illustrated.

As "cycloalkylsulfonyl group", the following groups are illustrated; cyclopropylsulfonyl group, cyclobutylsulfonyl group, cyclopentylsulfonyl group, cyclohexylsulfonyl group, cycloheptylsulfonyl group, or cyclooctylsulfonyl group.

As "cycloalkylsulfinyl group", the following groups are illustrated; cyclopropylsulfinyl group, cyclobutylsulfinyl group, cyclopentylsulfinyl group, cyclohexylsulfinyl group, cycloheptylsulfinyl group or cyclooctylsulfinyl group.

As "cycloalkoxy" in "cycloalkoxycarbonyl group", the same as the above cycloalkoxy group is illustrated. For example, cyclopropyloxycarbonyl group, cyclobutyloxycarbonyl group, cyclopentyloxycarbonyl group, cyclohexyloxycarbonyl group, cycloheptyloxycarbonyl group, or cyclooctyloxycarbonyl group is illustrated.

As aryl in "aryloxy group", "arylcarbonyl group", "aryloxycarbonyl group", "arylcarbonyloxy group", "arylsulfonyl group" and "arylsulfinyl group", the same as the above aryl group are illustrated. As "aryloxy group", phenoxy group, 1-naphthoxy group or 2-naphthoxy group is illustrated. As "arylcarbonyl group", benzoyl group, 1-naphthaloyl group or 2-naphthaloyl group is illustrated. As "aryloxycarbonyl group", phenoxycarbonyl group, 1-naphthoxycarbonyl group or 2-naphthoxycarbonyl group is illustrated. As "arylcarbonyloxy group", benzoyloxy group, 1-naphthoyloxy group or 2-naphthoyloxy group is illustrated. As "arylsulfonyl group", phenylsulfonyl group, 1-naphthylsulfonyl group, or 2-naphthylsulfonyl group is illustrated. As "arylsulfinyl group ", phenylsulfinyl group, 1-naphthylsulfinyl group, or 2-naphthylsulfinyl group is illustrated.

As heteroaryl group in "heteroaryloxy group", "heteroarylcarbonyl group", "heteroaryloxycarbonyl group", "heteroarylcarbonyloxy group", "heteroarylsulfonyl group" and "heteroarylsulfinyl group", the same as the above heteroaryl groups are illustrated. As "heteroaryloxy group", pyrrolyloxy group, pyridyloxy group, pyrazinyloxy group, pyrimidinyloxy group, pyridazynyloxy group, furyloxy group, or thienyloxy group is illustrated. As "heteroarylcarbonyl group", pyrrolylcarbonyl group, pyridylcarbonyl group, pyrazinylcarbonyl group, pyrimidinylcarbonyl group, pyridazinylcarbonyl group, furylcarbonyl group, thienylcarbonyl group, etc. is illustrated. As "heteroaryloxycarbonyl group", pyrrolyloxycarbonyl group, pyridyloxycarbonyl group, pyrazinyloxycarbonyl group, pyrimidinyloxycarbonyl group, pyridazinyloxycarbonyl group, furyloxycarbonyl group, or thienyloxycarbonyl group is illustrated. As "heteroarylcarbonyloxy group", pyrrolylcarbonyloxy group, pyridylcarbonyloxy group, pyrazinylcarbonyloxy group, pyrimidinylcarbonyloxy group, pyridazinylcarbonyloxy group, furylcarbonyloxy group, or thienylcarbonyloxy group is illustrated. As "heteroarylsulfonyl group", pyrrolylsulfonyl group, pyridylsulfonyl group, pyrazinylsulfonyl group, pyrimidinylsulfonyl group, pyridazinylsulfonyl group, furylsulfonyl group, or thienylsulfonyl group is illustrated. As "heteroarylsulfinyl group", pyrrolylsulfinyl group, pyridylsulfinyl group, pyrazinylsulfinyl group, pyrimidinylsulfinyl group, pyridazinylsulfinyl group, furylsulfinyl group, or thienylsulfinyl group is illustrated.

As "saturated or unsaturated heterocyclic group containing nitrogen atom" in A, 4 to 8 membered, preferably 4 to 7 membered and saturated or unsaturated heterocyclic group containing 1 to 2 hetero atoms selected from 1 to 2 nitrogen atoms, 0 to 1 oxygen atom and 0 to 1 sulfur atom, and at least one is a nitrogen atom and the sulfur atom may be substituted by 1 or 2 oxygen atoms. As "saturated heterocyclic group containing nitrogen atom" in A, azetidine, pyrrolidine, piperidine, piperazine, morpholine, thiomorpholine, thiomorpholine-1-oxide, thiomorpholine-1,1-dioxide, perhydroazepine, etc. is illustrated.

As "unsaturated heterocyclic group containing hetero atom" in A, 4 to 8 membered unsaturated non aromatic heterocyclic group having 1 to 2 double bonds in its ring and containing 1 to 2 hetero atoms selected from 1 to 2 nitrogen atoms, 0 to 1 oxygen atom and 0 to 1 sulfur atom, and at least one is a nitrogen atom and the sulfur atom may be substituted by 1 or 2 oxygen atoms. Five membered unsaturated non aromatic heterocyclic group having one double bond in its ring or 6 to 8 membered unsaturated non aromatic heterocyclic group having 1 to 2 double bonds in its ring is illustrated.

The substituent group when "unsaturated or unsaturated heterocyclic group" in A is substituted, is selected from halogen atom, hydroxy group, carboxy group, alkyl group, alkoxy group, alkoxycarbonyl group, alkylcarbonyl group, alkylsulfonyl group and alkylsulfinyl group.

As said 4 to 8 membered saturated or unsaturated heterocyclic group containing nitrogen atom, saturated heterocyclic group containing nitrogen atom and having divalent is selected from the following formulas (2) to (8): 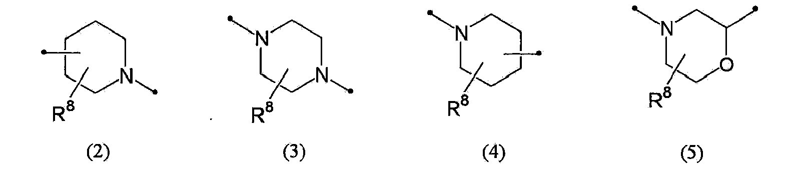 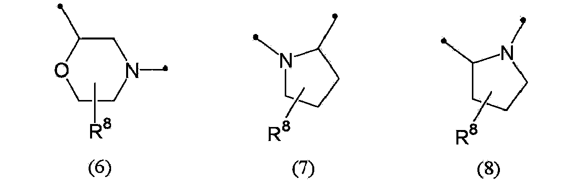 (wherein, R⁸ is hydrogen atom, halogen atom, hydroxy group, carboxy group, alkyl group, alkoxy group, alkoxycarbonyl group, alkylcarbonyl group, alkylsulfonyl group or alkylsulfinyl group, and the binding site is not specifically limited and it may be on any of the carbon or nitrogen atom, if chemically stable),
or in the above groups having divalent, an unsaturated heterocyclic group containing nitrogen atom and having divalent (wherein double bond is formed between one or two C-C bonds or C-N bonds selected optionally) is preferably illustrated. The divalent group of the above formulas (2) to (8) binds at left side with L¹, and at right side with L², respectively.

A is preferably selected from saturated heterocyclic group containing nitrogen atom and having divalent shown in the above formulas (2) to (8).

When A is the formula (7), L² is preferably a single bond.

In the formula (1), L¹ is preferably shown by the following formula:

(CH₂)ₙ-(Y¹)ₘ-(CH₂)₁ₐ

wherein, Y¹ is oxygen atom or NR⁵ (wherein R⁵ is the same as defined above.), n and la are independently integers, 0 to 5, and m is 0 or 1.

When A is a formula (2), (6) or (8), and m is 1, n is preferably 2 or more. When A is the formula (1), (3), (4), (5) or (7), and m is 1, n and 1a are preferably 2 or more.

Preferable L¹ in the formula (1) is illustrated below. Namely when L¹ is bound to a nitrogen atom in ring A, preferable L¹ is the formula (I) or (II) (wherein L¹ is bound to adenine skeletons at the left side.):
(I) -(CH₂)₂₋₈-,
(II) -(CH₂)₂₋₅-O-(CH₂)₂₋₅-·

When L¹ is bound to a carbon atom in ring A, preferable L¹ is the formula (III), (IV) or (V) (wherein L¹ is bound to adenine skeletons at the left side.):

(III) -(CH₂)₀₋₈-,

(IV) -(CH₂)₂₋₈-O-(CH₂)₀₋₃-,

(V) -(CH₂)₂₋₈-NR⁵-(CH₂)₀₋₃- (wherein R⁵ is the same as defined above.).

L2 in the formula (1) is preferable a single bond or C₁₋₄ straight chained alkylene.

In the present specification, the substituents of the substituted alkyl group, alkenyl group, alkynyl group, alkylcarbonyl group, alkoxycarbonyl group, alkylsulfonyl group or alkylsulfinyl group include the following (a) to (c):
(a) halogen atom, hydroxy group, carboxy group, haloalkoxy group, and mercapt group;
(b) alkoxy group, alkylthio group, alkylcarbonyl group, alkylcarbonyloxy group, alkylsulfonyl group, alkylsulfinyl group, alkoxycarbonyl group, alkenyloxy group, alkenylcarbonyl group, alkenylcarbonyloxy group, alkenylsulfonyl group, alkenylsulfinyl group, alkenyloxycarbonyl group, alkynyloxy group, alkynylcarbonyl group, alkynylcarbonyloxy group, alkynylsulfonyl group, alkynylsulfinyl group, and alkynyloxycarbonyl group (the group of this group may be substituted by one or more groups independently selected from the group consisting of halogen atom, hydroxy group, alkoxy group, carboxyl group, alkoxycarbonyl group, amino group optionally substituted by the same or different and one or two alkyl groups, carbamoyl group optionally substituted by the same or different and one or two alkyl groups, sulfamoyl group optionally substituted by the same or different and one or two alkyl groups, and alkylsulfonyl group.);
(c) optionally substituted amino group, optionally substituted carbamoyl group and optionally substituted sulfamoyl group (the group of this group may be substituted by one or two groups independently selected from the group consisting of the following (j), (k) and (1).), optionally substituted aryl group, optionally substituted aryloxy group, optionally substituted arylcarbonyl group, optionally substituted arylcarbonyloxy group, optionally substituted arylsulfonyl group, optionally substituted arylsulfinyl group, optionally substituted aryloxycarbonyl group, optionally substituted heteroaryl group, optionally substituted heteroaryloxy group, optionally substituted heteroarylcarbonyl group, optionally substituted heteroarylcarbonyloxy group, optionally substituted heteroarylsulfonyl group, optionally substituted heteroarylsulfinyl group and optionally substituted heteroaryloxycarbonyl group (the group of this group may be substituted by one or two groups independently selected from the group consisting of the following (g), (h) and (i).), and optionally substituted cycloalkyl group, optionally substituted cycloalkoxy group, optionally substituted cycloalkylcarbonyl group, optionally substituted cycloalkylcarbonyloxy group, optionally substituted cycloalkylsulfonyl group, optionally substituted cycloalkylsulfinyl group, optionally substituted cycloalkoxycarbonyl group and optionally substituted saturated heterocyclic group (this group may be substituted by one or two groups independently selected from the group consisting of the following (d), (e) and (f).);
   and this group can be substituted by one or more, and the same or different groups, preferably 1 to 5, more preferably 1 to 3 groups.
   In the present specification, when cycloalkyl group, cycloalkoxy group, cycloalkylcarbonyl group, cycloalkylsulfonyl group, cycloalkylsulfinyl group, cycloalkylcarbonyloxy group, cycloalkoxycarbonyl group or saturated heterocyclic group is substituted, "said substituent" is selected from the group consisting of the following (d) to (f):
(d) halogen atom, hydroxy, group, carboxy group, mercapt group, haloalkyl group, and haloalkoxy group;
(e) alkyl group, alkenyl group, alkynyl group, alkoxy group, alkylthio group, alkylcarbonyl group, alkylcarbonyloxy group, alkylsulfonyl group, alkylsulfinyl group, and alkoxycarbonyl group (the group of this group may be substituted by one or more groups independently selected from the group consisting of halogen atom, hydroxy group, alkoxy group, carboxyl group, alkoxycarbonyl group, amino group optionally substituted by the same or different and one or two alkyl groups, carbamoyl group optionally substituted by the same or different and one or two alkyl groups, a sulfamoyl group optionally substituted by the same or different and one or two alkyl groups, and alkylsulfonyl group.);
(f) optionally substituted aryl group and optionally substituted heteroaryl group (the group of this group may be substituted by the same or different and one or two groups described in the following (g), (h) and (i).), or optionally substituted amino group, optionally substituted carbamoyl group or optionally substituted sulfamoyl group (the group of this group may be substituted by the same or different and one or two groups described in the following (j), (k) and (l).);
   and this group can be substituted by one or more, and the same or different groups, preferably 1 to 5, more preferably 1 to 3 groups.
   In the present specification, when aryl group, heteroaryl group, aromatic carbocycle or aromatic hetero cycle is substituted, said substituent is selected from the group consisting of the following (g) to (i):
(g) halogen atom, hydroxy group, carboxy group, mercapt group, cyano group, nitro group, haloalkyl group, and haloalkoxy group;
(h) alkyl group, alkoxy group, alkylthio group, alkylcarbonyl group, alkoxycarbonyl group, alkylcarbonyloxy group, alkylsulfonyl group, alkylsulfinyl group, alkenyl group, alkynyl group, cycloalkyl group, and saturated heterocyclic group (the group of this group may be substituted by groups independently selected from the group consisting of halogen atom, hydroxy group, alkyl group, alkoxy group, carboxyl group, alkoxycarbonyl group, amino group optionally substituted by the same or different and one or two alkyl groups, carbamoyl group optionally substituted by the same or different and one or two alkyl groups, sulfamoyl group optionally substituted by the same or different and one or two alkyl groups, and alkylsulfonyl group.);
(i) optionally substituted amino group, optionally substituted carbamoyl group and optionally substituted sulfamoyl group;
   and this group can be substituted by one or more, and the same or different groups, preferably 1 to 5, more preferably 1 to 3 groups.
   "Substituent" in "optionally substituted amino group", "optionally substituted carbamoyl group " and "optionally substituted sulfamoyl group" is selected from the group consisting of the following (j), (k) and (l):
(j) alkyl group, alkenyl group, alkynyl group, alkylcarbonyl group, alkoxycarbonyl group, alkylsulfonyl group, alkylsulfinyl group, alkenylcarbonyl group, alkenyloxycarbonyl group, alkenylsulfonyl group, alkenylsulfinyl group, alkynylcarbonyl group, alkynyloxycarbonyl group, alkynylsulfonyl group, alkynylsulfinyl group, cycloalkyl group, cycloalkylcarbonyl group, cycloalkoxycarbonyl group, cycloalkylsulfonyl group, cycloalkylsulfinyl group, and saturated heterocyclic group (the group of this group may be substituted by groups independently selected from the group consisting of halogen atom, hydroxy group, alkyl group, alkoxy group, carboxyl group, alkoxycarbonyl group, amino group optionally substituted by the same or different and one or two alkyl groups, carbamoyl group optionally substituted by the same or different and one or two alkyl groups, sulfamoyl group optionally substituted by the same or different and one or two alkyl groups, and alkylsulfonyl group.);
(k) aryl group, arylcarbonyl group, aryloxycarbonyl group, arylsulfonyl group, arylsulfinyl group, heteroaryl group, heteroarylcarbonyl group, heteroaryloxycarbonyl group, heteroarylsulfonyl group, and heteroarylsulfinyl group (the group of this group may be substituted by groups independently selected from the group consisting of halogen atom, hydroxy group, alkyl group, alkoxy group, carboxyl group, alkoxycarbonyl group, amino group optionally substituted by the same or different and one or two alkyl groups, carbamoyl group optionally substituted by the same or different and one or two alkyl groups, sulfamoyl group optionally substituted by the same or different and one or two alkyl groups, and alkylsulfonyl group.);
(l) 4 to 7 membered saturated heterocyclic group containing 1 to 4 hetero atoms selected from 1 to 2 nitrogen atoms, 0 to 1 oxygen atom and 0 to 1 sulfur atom, and the sulfur atom may be substituted by 1 or 2 oxygen atoms, which is formed by combining two substituents on amino group, carbamoyl group or sulfamoyl group with the nitrogen atom (this saturated heterocyclic group may be substituted, if chemically stable, on optional its carbon atom or nitrogen atom, by one or two groups independently selected from the group consisting of halogen atom, hydroxy group, alkyl group, alkoxy group, carboxyl group, alkoxycarbonyl group, amino group optionally substituted by the same or different and one or two alkyl groups, carbamoyl group optionally substituted by the same or different and one or two alkyl groups, sulfamoyl group optionally substituted by the same or different and one or two alkyl groups, and alkylsulfonyl group.);

And said group may be substituted by one or two substituents, if chemically stable.

"Substituent" in substituted alkyl group, substituted alkenyl group and substituted alkynyl group in R² of the formula (1) is selected from the following group:
halogen atom, hydroxy group, carboxy group, mercapt group, C₁₋₆ alkoxy group, C₁₋₆ haloalkoxy group, C₂₋₆ alkoxycarbonyl group, C₁₋₆ alkylsulfonyl group, C₁₋₆ alkylsulfinyl group, C₂₋₆ alkylcarbonyloxy group, C₁₋₆ alkylthio group, optionally substituted amino group, optionally substituted carbamoyl group, optionally substituted sulfamoyl group and 3 to 8 membered cycloalkyl group (wherein the above cycloalkyl group may be substituted by halogen atom, hydroxy group, carboxy group, C₁₋₄ alkyl group or C₁₋₄ alkoxy group.).
"Substituent" in the above substituted amino group, substituted carbamoyl group and substituted sulfamoyl group is selected from one or two substituents selected from the following (a') or a group selected from (b'):
   (a') C₁₋₆ alkyl group, C₂₋₆ alkenyl group, C₂₋₆ alkynyl group, C₂₋₆ alkylcarbonyl group, C₂₋₆ alkoxycarbonyl group, C₁₋₆ alkylsulfonyl group, C ₁₋₆ alkylsulfinyl group, 3 to 8 membered cycloalkyl group, 3 to 8 membered cycloalkylcarbonyl group, 3 to 8 membered cycloalkoxycarbonyl group, 3 to 8 membered cycloalkylsulfonyl group, and 3 to 8 membered cycloalkylsulfinyl group (wherein the group of this group may be substituted by halogen atom, hydroxy group, C₁₋₆ alkoxy group, carboxy group or C₂₋₅ alkoxycarbonyl group.);
   (b') 4 to 7 membered saturated heterocyclic group containing 1 to 4 hetero atoms selected from 1 to 2 nitrogen atoms, 0 to 1 oxygen atom and 0 to 1 sulfur atom, which is formed by binding two substituent groups with the nitrogen atom (said saturated heterocyclic group containing nitrogen atom, if chemically stable, may be substituted on optional carbon atom or nitrogen atom by halogen atom, hydroxy group, carboxy group, C₁₋₆ alkyl group, C₁₋₆ alkoxy group, C₂₋₆ alkoxycarbonyl group or C₂₋₆ alkylcarbonyl group.).

R² is preferably C₁₋₄ alkyl group, C₃₋₈ alkylcarbonyloxyalkyl group, or alkyl group substituted by amino group optionally substituted by the above (a') or (b').

The above "amino group optionally substituted by (a') or (b')" includes alkyl group substituted by dialkylaminoalkyl group, morpholino group, 1-piperidinyl group, piperazino group or 1-pyrrolidinyl group, such as 4-dimethylaminobutyl group, 4-morpholinobutyl group, or 4-dimethylaminobutyl group. The above alkylcarbonyloxyalkyl group includes acetoxymethyl group, 1-acetoxyethyl group, etc. R² is more preferably methyl group.

When X in the formula (1) is NR⁴, R⁴ is preferably, hydrogen atom or C₁₋₃ alkyl group, more preferably hydrogen atom or methyl group. X is preferably oxygen atom or a single bond.

R¹ in the formula (1) is preferably optionally substituted straight or branched chain C₁₋₆ alkyl group. For example, methyl group, ethyl group, propyl group, butyl group, pentyl group, 1-methylethyl group, 1-methylpropyl group, and 2-methylbutyl group optionally substituted, respectively, and more preferably straight chain C₁₋₄ alkyl group are illustrated.

When R¹ is substituted alkyl group, said substituent is a substituent on alkyl group mentioned above, and its preferable substituent is fluorine atom, hydroxy group, straight or branched chain C₁₋₄ alkoxy group, or straight or branched chain C₁₋₄ alkylthio group, more preferably hydroxy group or straight or branched chain C₁₋₃ alkoxy group, and said alkoxy group may be substituted by 1 to 3 substituents.

The adenine compound of the present invention includes all tautomers, geometrical isomers and stereoisomers which are formed in accordance with the kind of the substituent, and a mixture thereof.

Namely, in a case where there are one or more asymmetrical carbon atoms in the compound of the formula (1), there exist diastereomers and optical isomers, and mixtures of those diastereomers and optical isomers and separated ones are also included in the present invention.

Additionally, the adenine compound shown by the formula (1) and its tautomer is chemically equivalent, and the adenine compound of the present invention includes such a tautomer. The tautomer is specifically a hydroxy compound shown by the formula (1'): 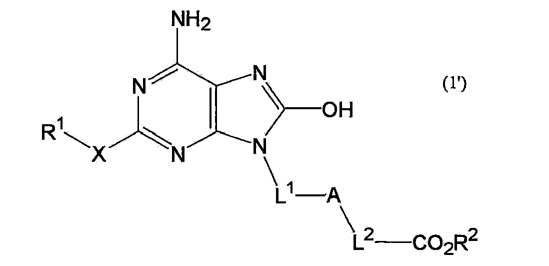 wherein R¹, R², A, X, L¹, and L² are the same as define above.

The pharmaceutically acceptable salt is exemplified by an acid salt and a base addition salt. The acid salt is, for example, an inorganic acid salt such as hydrochloride, hydrobromide, sulfate, hydroiodide, nitrate and phosphate, and an organic acid salt such as citrate, oxalate, acetate, formate, propionate, benzoate, trifluoroacetate, fumarate, maleate, succinate, tartrate, lactate, pyruvate, methanesulfonate, benzenesulfonate and p-toluenesulfonate, and the base salt is exemplified by an inorganic base salt such as sodium salt, potassium salt, calcium salt, magnesium salt and ammonium salt, and an organic base salt such as triethylammonium salt, triethanolammonium salt, pyridinium salt and diisopropylammonium salt, and further a basic or acidic amino acid salt such as arginine salt, aspartic acid salt and glutamic acid salt. The compound shown by the formula (1) may be hydrate and a solvate such as ethanolate.

The compounds shown by the formula (1) can be prepared by the following methods. The starting compounds not disclosed below can be prepared by a similar method to the following method or by a known method or its similar method.

### Preparation method 1

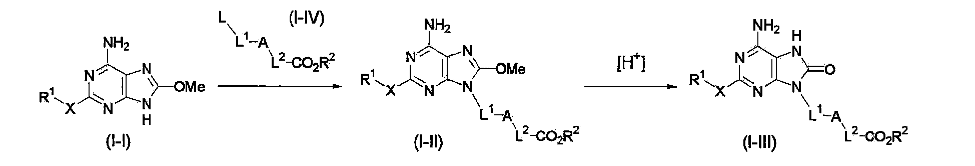

wherein L is a leaving group, A, R¹, R², X, L¹, and L² are the same as defined above.
The leaving group herein includes halogen atom, or sulfonyl group such as p-toluenesulfonyl group or methanesulfonyl group in case of alkylation or acylation.

Compound (I-II) can be prepared by reacting compound (I-I) and compound (I-IV) in the presence of a base. The base includes an alkali metal carbonate such as sodium carbonate or potassium carbonate, alkaline earth metal carbonate such as calcium carbonate, metal hydroxide such as sodium hydroxide or potassium hydroxide, or a metal hydride such as sodium hydride, or a metal alkoxide such as potassium t-butoxide. The solvent includes an aprotic solvent such as dimethylformamide, dimethyl sulfoxide or acetonitrile, a halogenated hydrocarbon such as carbon tetrachloride, chloroform or methylene chloride, or an ether such as diethyl ether, tetrahydrofuran or 1,4-dioxane. The reaction is carried out at about 0°C to the boiling point of the solvent.

Compound (I-III) can be prepared by treating compound (I-II) under acidic condition. As an acid for acidic treatment, an inorganic acid such as hydrochloric acid, hydrobromic acid or sulfuric acid, or an organic acid such as trifluoroacetic acid can be used. As a solvent, water or a mixture of water and an organic solvent can be used. The organic solvent includes an ether solvent such as diethylether or tetrahydrofuran, an aprotic solvent such as dimethylformamide or acetonitrile, or an alcohol such as methanol or ethanol. The reaction is carried out at about 0°C to the boiling point of the solvent.

Further, compound (I-IV) can be prepared by the following method. 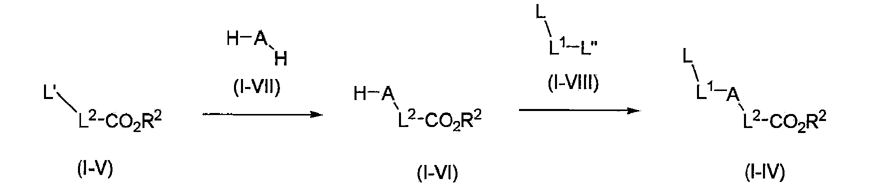 wherein L, L' and L" are the same or different and are a leaving group, A, R², L¹ and L² are the same as defined above.

The leaving group herein includes halogen atom in case of alkylation or acylation, hydroxy group in case of dehydrative condensation and oxo group in case of reductive alkylation of amine.

Compound (I-VI) can be prepared starting from compound (I-V) and compound (I-VII) by selecting a suitable method from the well known methods for the skilled person in the art according to the structures of L¹ and A (such as, alkylation, dehydrative condensation on a carboxylic acid and amine compound, or reductive alkylation of an amine compound).

For example, compound (I-VI) can be prepared by reacting compound (I-V) and compound (I-VII) in the presence of a base. Then, compound (I-IV) can be prepared by reacting compound (I-VI) and compound (I-VIII) in the presence of a base. The base includes an alkali metal carbonate such as sodium carbonate or potassium carbonate, alkaline earth metal carbonate such as calcium carbonate, metal hydroxide such as sodium hydroxide or potassium hydroxide, an organic base such as triethylamine, diisopropylethylamine, pyridine or 4-dimethylaminopyridine, or a metal alkoxide such as potassium t-butoxide. The solvent includes a halogenated hydrocarbon such as methylene chloride, an ether such as diethyl ether, tetrahydrofuran or 1,4-dioxane, an alcohol such as methanol or ethanol, or an aprotic solvent such as dimethylformamide, dimethyl sulfoxide or acetonitrile. The reaction is carried out at about 0°C to the boiling point of the solvent.

Furthermore, when the compound of the present invention or its intermediate has a functional group such as amino group, carboxy group, hydroxy group or oxo group, the protection or deprotection technique is applied to it, if necessary. Preferable protective groups, protecting methods and deprotechting methods are described in detail in "Protective groups in Organic Synthesis 2nd Edition (John Wiley & Sons, Inc.; 1990)" and so on.

Furthermore, compound (I-I) can be prepared by the following method. 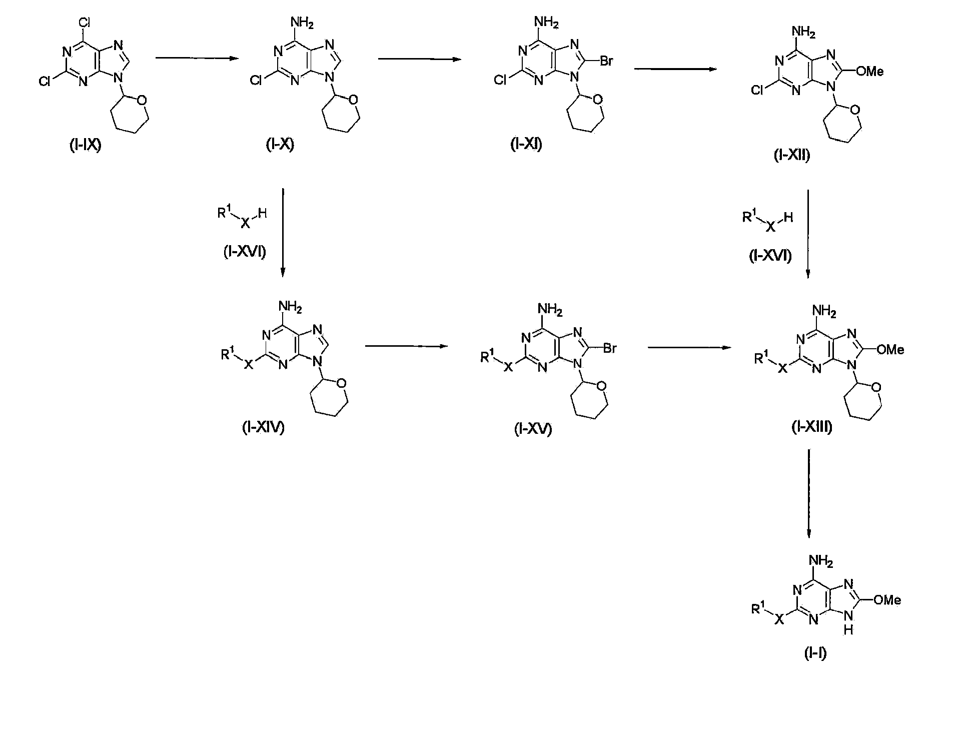 wherein R¹ and X are the same as defined above.

Compound (I-X) can be prepared by reacting compound (I-IX) and ammonia in water, an organic solvent or a mixture of water and an organic solvent.

The organic solvent includes an alcohol solvent such as methanol, ethanol, propanol or butanol, an ether solvent such as tetrahydrofuran, 1,4-dioxane or diglyme, or an aprotic solvent such as acetonitrile, etc. The reaction temperature is selected for example from a range of about room temperature to 200°C. The reaction may be carried out in an autoclave, if necessary.

Compound (I-XI) is prepared by brominating compound (I-X). As the brominating agent, bromine, hydroperbromic acid, or N-bromosuccinimide can be used.

In this reaction a reaction auxiliary such as sodium acetate may be added. The solvents such as a halogenated hydrocarbon, like carbon tetrachloride, methylene chloride or dichloroethane, an ether like diethyl ether, acetic acid, or carbon disulfide can be used. The reaction temperature is selected from a range of about 0°C to around the boiling point of the solvent.

Compound (I-XII) can be prepared by reacting compound (I-XI) and sodium methoxide.

The solvents include an ether such as diethyl ether, tetrahydrofuran or 1,4-dioxane, an aprotic solvent such as dimethylformamide, or an alcohol such as methanol. The reaction temperature is selected from a range of room temperature to around the boiling point of the solvent.

Compound (I-XII) can be also prepared by treating compound (I-XI) in an alkaline aqueous solution containing methanol.

As the alkaline aqueous solution, an aqueous solution containing alkali metal hydroxide such as sodium hydroxide or potassium hydroxide can be used. The reaction temperature is selected from a range of room temperature to around the boiling point of the solvent.

Compound (I-XIII) can be prepared by reacting compound (I-XII) and compound (I-XVI).

When X is NR⁴ (wherein R⁴ is the same as defined above.), the reaction is carried out in the presence or absence of a base. Can be used the base such as an alkali metal carbonate, like sodium carbonate or potassium carbonate, an alkaline earth metal carbonate, like calcium carbonate, a metal hydroxide, like sodium hydroxide or potassium hydroxide, or an organic base, like triethylamine, diisopropylethylamine or 4-dimethylaminopyridine. Can be used the solvent such as an ether, like tetrahydrofuran, 1,4-dioxane or diglyme, an alcohol, like propanol or butanol, or an aprotic solvent, like dimethylformamide. The reaction may be carried out in the absence of a solvent. The reaction temperature is selected from a range of about 50°C to 200°C.

When X is oxygen atom or sulfur atom, the reaction is carried out in the presence of a base. Can be used the base such as an alkali metal, like sodium or potassium, an alkali metal hydride, like sodium hydride. Can be used the solvent such as an ether, like tetrahydrofuran, 1,4-dioxane or diglyme, or an aprotic solvent, like dimethylformamide or dimethyl sulfoxide. The reaction may be carried out in the absence of a solvent. The reaction temperature is selected from a range of about 50°C to 200°C.

The compound wherein X is SO₂, can be obtained by oxidizing the intermediate compound wherein the corresponding X is sulfur atom, with Oxone or m-chloroperbenzoic acid (m-CPBA).

In the process of preparing the compound (I-XIII) from the compound (I-X), the compound (V-XIV) is synthesized by the same method as mentioned above to convert to compound (I-XV) and then, compound (I-XIII) can also be obtained.

Compound (I-I) can be obtained by treating compound (I-XIII) with trifluoroacetic acid in an organic solvent such as methanol. As the acid, an inorganic acid such as hydrochloric acid, hydrobromic acid or sulfuric acid, or an organic acid such as trifluoroacetic acid can be used. As the solvent, water or a mixture of water and an organic solvent can be used. The organic solvent includes an ether such as diethylether or tetrahydrofuran, an aprotic solvent such as dimethylformamide or acetonitrile, or an alcohol such as methanol or ethanol. The reaction temperature is selected from a range of room temperature to around the boiling point of the solvent.

### Preparation method 2

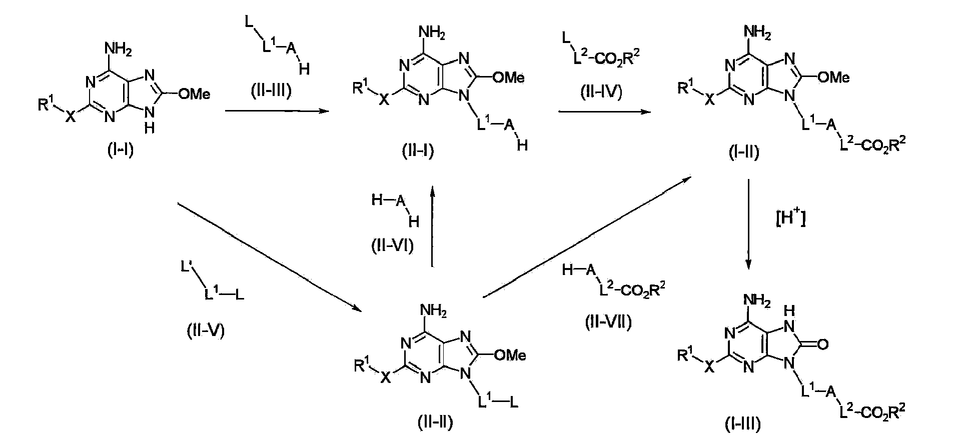

wherein L and L' are the same or different and are a leaving group, A, R¹, R², X, L¹ and L² are the same as defined above.

Compound (II-I) can be prepared by reacting compound (I-I) and compound (II-III) in the presence of a base.

Compound (I-II) can be prepared by reacting compound (II-I) and compound (II-IV) in the presence of a base, or can be also obtained by a conventional method known to the person in the art such as dehydrative condensation or reductive alkylation.

Compound (II-I) can be prepared by further reacting under the basic condition, compound (II-VI) with compound (II-II) which is prepared by reacting compound (I-I) and compound (II-V) under the base condition, or can be also obtained by a conventional method known to the person in the art such as dehydrative condensation or reductive alkylation. Compound (I-II) can be also prepared by reacting compound (II-II) and compound (II-VII) under the basic condition.

Can be used the base such as an alkali metal carbonate, like sodium carbonate or potassium carbonate, an alkaline earth metal carbonate such as calcium carbonate, a metal hydroxide, sodium hydroxide or potassium hydroxide, an organic base, like triethylamine, diisopropylethylamine, pyridine or 4-dimethylaminopyridine, or a metal alkoxide such as sodium methoxide. Can be used the solvent such as a halogenated hydrocarbon, like methylene chloride, an ether, like diethyl ether, tetrahydrofuran, 1,4-dioxane or diglyme, an alcohol, like methanol or ethanol, or an aprotic solvent, like dimethylformamide, dimethyl sulfoxide or acetonitrile. The reaction temperature is selected from a range of about 0°C to around the boiling point of the solvent.

Compound (I-II) is also prepared by reacting compound (II-II) and compound (II-VII) with a conventional method known to the person in the art such as dehydrative condensation or reductive alkylation.

Compound (I-III) can be prepared by treating compound (I-II) under the acidic condition.

The compound of the generic formula (1) can be also prepared by the following method. The starting compounds which are not described below can be prepared in accordance with the following method or by a known method or in accordance with a known method.

### Preparation method 3

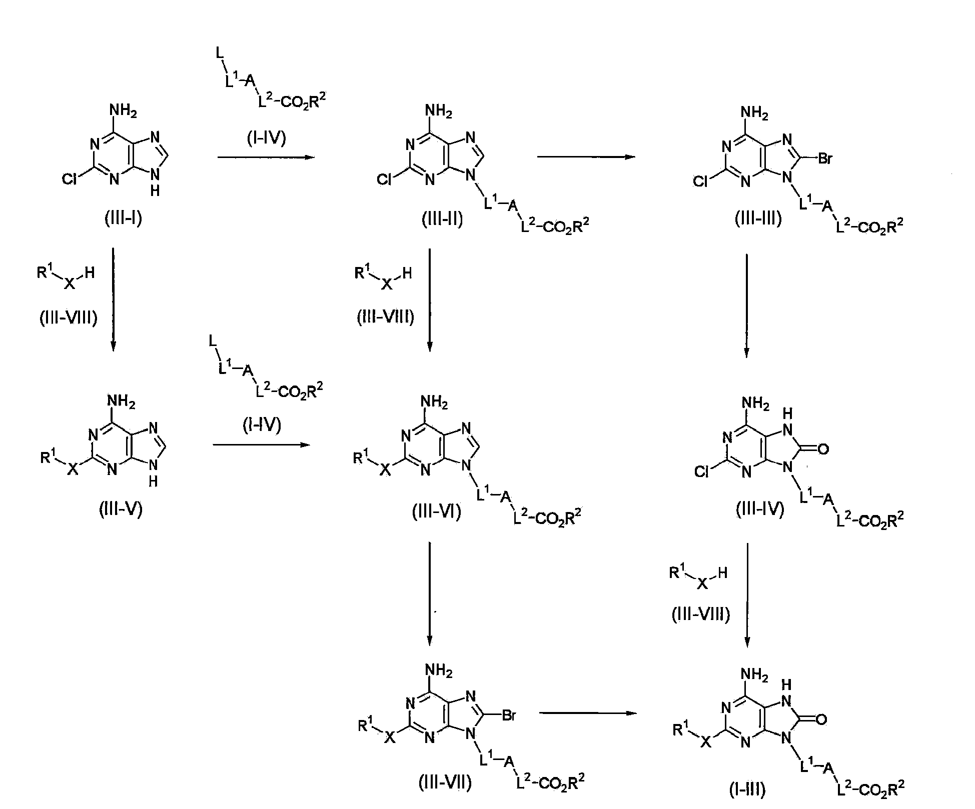

wherein L is a leaving group, A, R¹, R², X, L¹ and L² are the same as defined above.

Compound (III-II) can be prepared by reacting compound (III-I) and compound (I-IV) in the presence of a base.

Can be used the base such as an alkali metal carbonate, like sodium carbonate or potassium carbonate, an alkaline earth metal carbonate, like calcium carbonate, a metal hydroxide, like sodium hydroxide or potassium hydroxide, a metal hydride, like sodium hydride, or a metal alkoxide, like potassium t-butoxide. Can be used the solvent such as a halogenated hydrocarbon, like carbon tetrachloride, chloroform or methylene chloride, an ether, like diethyl ether, tetrahydrofuran or 1,4-dioxane, or an aprotic solvent, like dimethylformamide, dimethyl sulfoxide or acetonitrile. The reaction temperature is selected from a range of about 0°C to around the boiling point of the solvent.

Compound (III-III) can be prepared by brominating compound (III-II). As the brominating agent, bromine, hydroperbromic acid, or N-bromosuccinimide is used. In this reaction a reaction auxiary such as sodium acetate may be added. The solvents such as a halogenated hydrocarbon, like carbon tetrachloride, methylene chloride or dichloroethane, an ether like diethyl ether, acetic acid, or carbon disulfide can be used. The reaction temperature is selected from a range of about 0°C to around the boiling point of the solvent.

Compound (III-IV) can be prepared by treating compound (III-III) with a metal alkoxide such as sodium methoxide, etc., under the acidic condition.

The solvents used in reacting with a metal alkoxide include an ether such as diethyl ether, tetrahydrofuran or 1,4-dioxane, an aprotic solvent such as dimethylformamide, or an alcohol such as methanol corresponding to a metal alkoxide used. The reaction temperature is selected from a range of room temperature to around the boiling point of the solvent.

The acid used in acid-treatment includes an inorganic acid such as hydrochloric acid, hydrobromic acid or sulfuric acid, or an organic acid such as trifluoroacetic acid. Can be used the solvent such as water or a mixture of water and an organic solvent. The organic solvent includes an ether such as diethyl ether or tetrahydrofuran, an aprotic solvent such as dimethylformamide or acetonitrile, or an alcohol such as methanol or ethanol. The reaction temperature is selected from a range of room temperature to around the boiling point of the solvent.

Compound (I-III) can be prepared by reacting compound (III-IV) and compound (III-VIII).

When X is NR⁴ (wherein R⁴ is the same as defined above), the reaction is carried out in the presence or absence of a base. Can be used the base such as an alkali metal carbonate, like sodium carbonate or potassium carbonate, an alkaline earth metal carbonate, like calcium carbonate, a metal hydroxide, like sodium hydroxide or potassium hydroxide, or an organic base, like triethylamine, diisopropylethylamine or 4-dimethylaminopyridine. Can be used the solvent such as an ether, like tetrahydrofuran, 1,4-dioxane or diglyme, an alcohol, like propanol or butanol, or an aprotic solvent, like dimethylformamide. The reaction may be carried out in the absence of a solvent. The reaction temperature is selected from a range of about 50°C to 200°C.

When X¹ is oxygen atom or sulfur atom, the reaction is carried out in the presence of a base. Can be used the base such as an alkali metal, like sodium or potassium, an alkali metal hydride, like sodium hydride. Can be used the solvent such as an ether, like tetrahydrofuran, 1,4-dioxane or diglyme, or an aprotic solvent, like dimethylformamide or dimethyl sulfoxide. The reaction may be carried out in the absence of a solvent. The reaction temperature is selected from a range of about 50°C to 200°C.

The compound wherein X is SO₂, can be obtained by oxidizing the intermediate compound wherein the corresponding X is sulfur atom, with Oxone® or m-chloroperbenzoic acid (m-CPBA).

In the process of preparing compound (I-III) from compound (III-I), compound (III-VI) is synthesized from compound (III-II) by the same method as mentioned above, or compound (III-VI) is prepared via compound (III-V) from compound (III-I) and then after converting compound (III-VI) into compound (III-VII), compound (I-III) can also be obtained.

### Preparation method 4

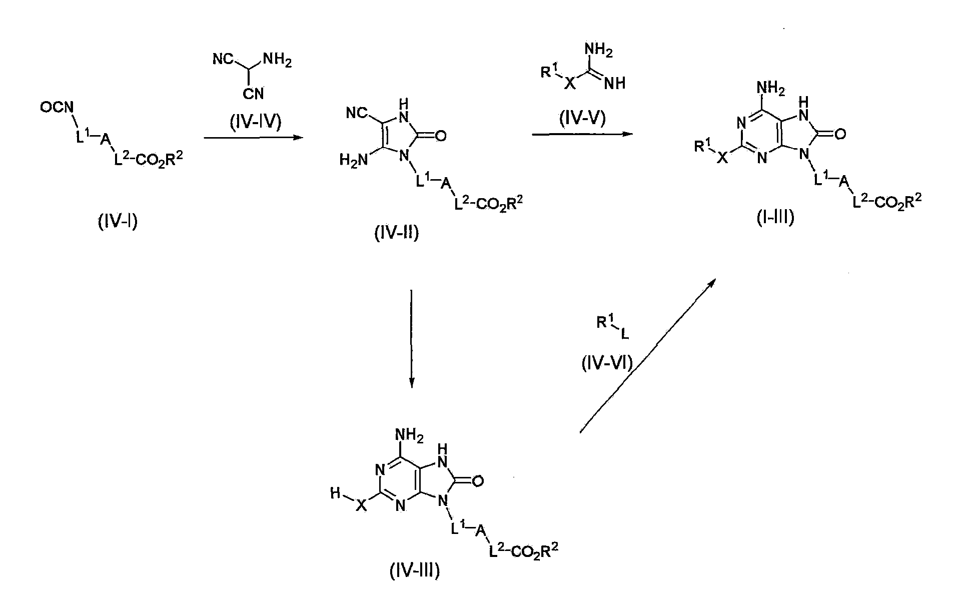

wherein L is a leaving group, A, R¹, R², X, L¹ and L² are the same as defined above.

Compound (IV-II) can be prepared by reacting compound (IV-I) and compound (IV -IV) in the presence of a base.

Can be used the base such as an alkali metal carbonate, like sodium carbonate or potassium carbonate, an alkaline earth metal carbonate, like calcium carbonate, a metal hydroxide, like sodium hydroxide or potassium hydroxide, an organic base, like triethylamine, diisopropylethylamine, pyridine or 4-dimethylaminopyridine, or metal alkoxide, like sodium methoxide. Can be used the solvent such as a halogenated hydrocarbon like methylene chloride, an ether, like diethyl ether, tetrahydrofuran or 1,4-dioxane, an alcohol, like methanol orethanol, or an aprotic solvent, like dimethylformamide, dimethyl sulfoxide or acetonitrile. The reaction may be carried out in the absence of a solvent. The reaction temperature is selected from a range of about 0°C to around the boiling point of the solvent.

Compound (I-III) can be prepared by reacting compound (IV-II) and compound (IV-V) in the presence or absence of a base.

Can be used the base such as an alkali metal carbonate, like sodium carbonate or potassium carbonate, an alkaline earth metal carbonate like calcium carbonate, a metal hydroxide, like sodium hydroxide or potassium hydroxide, an organic base, like triethylamine, diisopropylethylamine, pyridine or 4-dimethylaminopyridine, or metal alkoxide, like sodium methoxide. Can be used the solvent such as an ether, like tetrahydrofuran, 1,4-dioxane or diglyme, an alcohol, like methanol or ehanol, or an aprotic solvent, like toluene, dimethylformamide or dimethyl sulfoxide. The reaction may be carried out in the absence of a solvent. The reaction temperature is selected from a range of room temperature to around the boiling point of the solvent.

In the process of preparing compound (I-III) from compound (IV-II), compound (IV-III) is prepared and then the compound can be converted to compound (I-III).

When X is NR⁴ (wherein R⁴ is hydrogen atom or alkyl group), compound (IV-III) can be prepared by reacting compound (IV-II) and guanidine in the presence or absence of a base. Can be used the base such as an alkali metal carbonate, like sodium carbonate or potassium carbonate, an alkaline earth metal carbonate like calcium carbonate, a metal, like hydroxide, sodium hydroxide or potassium hydroxide, an organic base, like triethylamine, diisopropylethylamine, pyridine or 4-dimethylaminopyridine, or a metal alkoxide, like sodium methoxide. Can be used the solvent such as an ether, like tetrahydrofuran, 1,4-dioxane or diglyme, an alcohol, like methanol or ethanol, or an aprotic solvent, like toluene, dimethylformamide or dimethyl sulfoxide. The reaction may be carried out in the absence of a solvent. The reaction temperature is selected from a range of room temperature to around the boiling point of the solvent.

When X is oxygen atom, compounds (IV-III) can be prepared by reacting compound (IV-II) and urea in the presence or absence of a base. Can be used the base such as an alkali metal carbonate, like sodium carbonate or potassium carbonate, an alkaline earth metal carbonate like calcium carbonate, a metal hydroxide, like sodium hydroxide or potassium hydroxide, an organic base, like triethylamine, diisopropylethylamine, pyridine or 4-dimethylaminopyridine, or metal alkoxide, like sodium methoxide. Can be used the solvent such as an ether, like tetrahydrofuran, 1,4-dioxane or diglyme, an alcohol, like methanol or ethanol, or an aprotic solvent, like toluene, dimethylformamide or dimethyl sulfoxide. The reaction may be carried out in the absence of a solvent. The reaction temperature is selected from a range of room temperature to around the boiling point of the solvent.

When X is sulfur atom, compounds (IV-III) can be prepared by reacting compound (IV-II) and benzoylisocyanate in the presence or absence of a base, followed by cyclization.

As the base used in the reaction with benzoylisocyanate, can be illustrated the base such as an alkali metal carbonate, like sodium carbonate or potassium carbonate, an alkaline earth metal carbonate like calcium carbonate, or an organic base, like triethylamine, diisopropylethylamine, pyridine or 4-dimethylaminopyridine. Can be used the solvent such as a halogenated hydrocarbon, like methylene chloride, an ether, like tetrahydrofuran or 1,4-dioxane, or an aprotic solvent, like dimethylformamide or dimethyl sulfoxide. The reaction temperature is selected from a range of about 0°C to around the boiling point of the solvent.

As the base used in the cyclization reaction, can be illustrated the base such as an alkali metal hydroxide, like sodium hydroxide or potassium hydroxide, or metal alkoxide, like sodium methoxide or potassium t-butoxide. Can be used the solvent such as an ether, like tetrahydrofuran, an alcohol, like ethanol or 2-propanol, or an aprotic solvent, like dimethylformamide or dimethyl sulfoxide. The reaction temperature is selected from a range of around room temperature to around the boiling point of the solvent.

Compound (I-III) can be prepared by reacting compounds (IV-III) and compound (IV-VI) in the presence of a base. Can be used the base such as an alkali metal hydrogencarbonate like sodium hydrogencarbonate, an alkaline earth metal carbonate, like calcium carbonate or potassium carbonate, a metal hydroxide, like sodium hydroxide or potassium hydroxide, an organic base, like triethylamine, diisopropylethylamine, pyridine or 4-dimethylaminopyridine, or a metal alkoxide like potassium t-butoxide. Can be used the solvent such as a halogenated hydrocarbon, like carbon tetrachloride, chloroform or methylene chloride, an ether, like diethyl ether, tetrahydrofuran or 1,4-dioxane, or an aprotic solvent, like dimethylformamide, dimethyl sulfoxide or acetonitrile. The reaction temperature is selected from a range of about 0°C to around the boiling point of the solvent.

In a case where the adenine compound of the present invention or its intermediate or the starting compound has a functional group, a reaction for increasing a carbon atom, a reaction for introducing a substituent or a reaction for conversion of the functional group can be conducted optionally according to a manner conventional to the skilled artisan in an appropriate step, namely in an intermittent step in each of the preparation methods described in the preparation method 1 or 2. For this purpose, the methods described in "JIKKEN KAGAKU-KOZA (edited by NIHON KAGAKU-KAI, MARUZEN)", or "Comprehensive Organic Transformation, R. C. Lalock (VCH Publishers, Inc. 1989)" can be used. The reaction for increasing a carbon atom includes a method comprising converting an ester group to hydroxymethyl group using a reducing agent such as aluminum lithium hydride, introducing a leaving group and then introducing a cyano group. The reaction for conversion of a functional group includes a reaction for conducting acylation or sulfonylation using an acid halide, a sulfonyl halide, etc., a reaction for reacting an alkylation agent such as a halogenated alkyl, a hydrolysis reaction, a reaction for C-C bond formation such as Friedel-Crafts reaction, Wittig reaction, a reaction of oxidation or reduction, etc.

In a case where the compound of the present invention or its intermediate contains a functional group such as amino group, carboxy group, hydroxy group or oxo group, a technology of protection and deprotection can optionally be used. A preferable protecting group, a protection method and a deprotection method are described in details in "Protective Groups in Organic Synthesis 2nd Edition (John Wiley & Sons, Inc.; 1990)", etc.

The compound (1) of the present invention and the intermediate compound for production thereof can be purified by a method known to the skilled artisan. For instance, purification can be conducted by column chromatography (e.g. silica gel column chromatography or ion exchange chromatography) or recrystallization. As a recrystallization solvent, for instance, can be used an alcohol such as methanol, ethanol or 2-propanol, an ether such as diethyl ether, an ester such as ethyl acetate, an aromatic hydrocarbon such as benzene or toluene, a ketone such as acetone, a hydrocarbon such as hexane, an aprotic solvent such as dimethylformamide or acetonitrile, water, or a mixture of two or more thereof. As other purification method, can be used those described in "JIKKEN KAGAKU-KOZA (edited by NIHON KAGAKU-KAI, MARUZEN) Vol. 1", etc.

In a case where the compound of the formula (1) of the present invention contains one or more asymmetric carbon, its production can be conducted by using the starting material containing those asymmetric carbons or by introducing the asymmetric carbon during the production steps. For instance, in a case of an optical isomer, the object can be obtained by using an optically active starting material or by conducting an optical resolution at a suitable stage of the production steps. The optical resolution can be conducted by a diastereomer method comprising a step of allowing the compound of the formula (1) or its intermediate to form a salt with an optically active acid (e.g. a monocarboxylic acid such as mandelic acid, N-benzyloxyalanine or lactic acid, a dicarboxylic acid such as tartaric acid, o-diisopropylidene tartaric acid or malic acid, or a sulfonic acid such as camphor sulfonic acid or bromocamphor sulfonic acid) in an inert solvent (e.g. an alcohol such as methanol, ethanol, or 2-propanol, an ether such as diethyl ether, an ester such as ethyl acetate, a hydrocarbon such as toluene, an aprotic solvent such as acetonitrile, or a mixture of two or more thereof).

In a case where the compound of the formula (1) or its intermediate contains an acid functional group such as carboxylic group, the object can be attained also by forming a salt with an optically active amine (e.g. an organic amine such as α-phenethylamine, quinine, quinidine, cinchonidine, cinchonine or strychnine).

The temperature for formation of the salt is selected from room temperature to the boiling point of the solvent. In order to increase optical purity, the temperature is preferably once increased up to the boiling point of the solvent. Upon recovering the salt formed by filtration, the yield can be increased optionally by cooling. An amount of the optical active acid or amine is about 0.5 to about 2.0 equivalent, preferably around 1 equivalent, relative to the substrate. An optically active salt with highly optical purity can be obtained optionally by recrystallization from an inert solvent (e.g. an alcohol such as methanol, ethanol or 2-propanol, an ether such as diethyl ether, an ester such as ethyl acetate, a hydrocarbon such as toluene, an aprotic solvent such as acetonitrile, or a mixture of two or more thereof). If necessary, the optically resoluted salt can be converted into a free form by treating with an acid or a base by the conventional method.

The adenine compound or its pharmaceutically acceptable salt of the present invention activates Toll-like receptor (TLR), concretely TLR7 and is useful as an immuno-modulator and thus useful as a therapeutic and prophylactic agent for diseases associated with an abnormal immune response (e.g. autoimmune diseases and allergic diseases) and various infectious diseases and cancers which are required for activation of an immune response. For instance, the adenine compound or its pharmaceutically acceptable salt of the present invention is useful as a therapeutic and prophylactic agent for the diseases mentioned in the following (1)-(8).
(1) (Respiratory diseases) asthma, including bronchial, allergic, intrinsic, extrinsic, exercise-induced, drug-induced (including NSAID such as aspirin and indomethacin) and dust-induced asthma both intermittent and persistent and of all severities, and other causes of airway hyper-responsiveness due to other causes; chronic obstructive pulmonary disease (COPD); bronchitis, including infectious and eosinophilic bronchitis; emphysema; bronchiectasis; cystic fibrosis; sarcoidosis; farmer's lung and related diseases; hypersensitivity pneumonitis; lung fibrosis, including cryptogenic fibrosing alveolitis, idiopathic interstitial pneumonias, fibrosis complicating anti-neoplastic therapy and chronic infection, including tuberculosis and aspergillosis and other fungal infections; complications of lung transplantation; vasculitic and thrombotic disorders of the lung vasculature, and pulmonary hypertension; antitussive activity including treatment of chronic cough associated with inflammatory and secretory conditions of the airways, and iatrogenic cough; acute and chronic rhinitis including rhinitis medicamentosa, and vasomotor rhinitis; perennial and seasonal allergic rhinitis including rhinitis nervosa (hay fever); nasal polyposis; acute viral infection including the common cold, and infection due to respiratory syncytial virus, influenza, coronavirus (including SARS) and adenovirus;
(2) (Skin) psoriasis, atopic dermatitis, contact dermatitis or other eczematous dermatoses, and delayed-type hypersensitivity reactions; phyto-and photodermatitis; seborrheic dermatitis, dermatitis herpetiformis, lichen planus, lichen sclerosus, lichen sclerosus et atrophicus, pyoderma gangrenosum, skin sarcoidosis, discoid lupus erythematosus, pemphigus, pemphigoid, epidermolysis bullosa, urticaria, angioedema, vasculitides, toxic erythemas, cutaneous eosinophilias, alopecia areata, male-pattern baldness, Sweet's syndrome, Weber-Christian syndrome, erythema multiforme; cellulitis, both infective and non-infective; panniculitis; cutaneous lymphomas, non-melanoma skin cancer and other dysplastic lesions; drug-induced disorders including fixed drug eruptions;
(3) (Eyes) blepharitis; conjunctivitis, including perennial and vernal allergic conjunctivitis; iritis; anterior and posterior uveitis; choroiditis; autoimmune; degenerative or inflammatory disorders affecting the retina; ophthalmitis including sympathetic ophthalmitis; sarcoidosis; infections including viral, fungal, and bacterial;
(4) (Genitourinary) nephritis including interstitial and glomerulonephritis; nephrotic syndrome; cystitis including acute and chronic (interstitial) cystitis and Hunner's ulcer; acute and chronic urethritis, prostatitis, epididymitis, oophoritis and salpingitis; vulvo-vaginitis; Peyronie's disease; erectile dysfunction (both male and female);
(5) (Allograft rejection) acute and chronic following, for example, transplantation of kidney, heart, liver, lung, bone marrow, skin or cornea or following blood transfusion; or chronic graft versus host disease;
(6) Other auto-immune and allergic disorders including rheumatoid arthritis, irritable bowel syndrome, systemic lupus erythematosus, multiple sclerosis, Hashimoto's thyroiditis, Graves' disease, Addison's disease, diabetes, idiopathic thrombocytopaenic purpura, eosinophilic fasciitis, hyper-IgE syndrome, antiphospholipid syndrome;
(7) (Oncology) treatment of common cancers including prostate, breast, lung, ovarian, pancreatic malignant, liver bowel and colon, stomach, skin and brain tumors and malignant bone marrow neoplasm (including the leukaemias) and lymphoproliferative systems neoplasm, such as Hodgkin's and non-Hodgkin's lymphoma; including the prevention and treatment of metasis and tumor recurrences, and paraneoplastic syndromes; and
(8) (Infectious diseases) viral diseases such as genital warts, common warts, plantar warts, hepatitis B, hepatitis C, herpes simplex virus, molluscum contagiosum, variola, acquired immunodeficiency syndrome (HIV), or infectious diseases due to human papilloma virus (HPV), cytomegalo virus (CMV), varicella zoster virus (VZV), rhinovirus, adenovirus, coronavirus, influenza, or para-influenza; bacterial diseases such as tuberculosis, mycobacterium avium, or leprosy; other infectious diseases, such as fungal diseases, candida chlamydia, or aspergillus, cryptococcal meningitis, pneumocystis carnii, cryptosporidiosis, histoplasmosis, toxoplasmosis, trypanosome infection, or leishmaniasis.

The adenine compounds or pharmaceutically acceptable salt thereof can also be used as vaccine adjuvant.

The adenine compound of the present invention, or its pharmaceutically acceptable salt has an activating effect of TLR, concretely TLR7. The adenine compound of the present invention, or its pharmaceutically acceptable salt shows an interferon-α or interferon-γ inducing activity and a suppressing activity of the production of IL-4 or IL-5, and thus shows an effect as a medicament having an immunomodulating activity specific against type 1 helper T-cell (Th1 cell) / type 2 helper T-cell (Th2 cell), namely, preferably useful as a prophylactic or therapeutic agent for allergic diseases such as asthma, COPD, allergic rhinitis, allergic conjunctivitis and atopic dermatosis due to the Th2 cell selective immuno-suppressive action. On the other hand, due to its immune activating effect, it is useful as a prophylactic or therapeutic agent for cancer, hepatitis B, hepatitis C, acquired immunodeficiency syndrome (HIV) and viral disease caused by infection with human papilloma virus (HPV), a bacterial infectious disease and dermatosis such psoriasis.

The adenine compound of the present invention, or its pharmaceutically acceptable salt is useful as a prophylactic or therapeutic agent for airway obstruction such as asthma or COPD, or for reduction of the risk thereof.

The adenine compound of the present invention or its pharmaceutically acceptable salt has no limitation as to its administration formulation and is administered orally or parenterally. The preparation for oral administration can be exemplified by capsules, powders, tablets, granules, fine-grain, syrups, solutions, suspensions, etc., and the preparation for parenteral administration can be exemplified by injections, drips, eye-drops, intrarectal preparations, inhalations, sprays (e.g. liquids/suspensions for sprays, aerosols, orcartridge spray for inhalators or insufflators), lotions, gels, ointments, creams, transdermal preparations, transmucosa preparations, nasal drops, ear drops, tapes, transdermal patches, cataplasms, powders for external application, and the like. Those preparations can be prepared by known manners, and acceptable conventional carriers, fillers, binders, lubricants, stabilizers, disintegrants, buffering agents, solubilizing agents, isotonic agents, surfactants, antiseptics, perfumes, and so on can be used. Two or more pharmaceutical carriers can be appropriately used.

The compound of the present invention, or its pharmaceutically acceptable salt is admixed with a pharmaceutically acceptable carrier by the conventional method for the person in the art to prepare the pharmaceutical composition suitable for administration. For example, the pharmaceutical composition containing the compound of the present invention or its pharmaceutically acceptable salt 0.05-99 weight %, preferably 0.05 - 80 weight %, more preferably 0.1 - 70 weight %, and further more preferably 0.1 - 50 weight % as an active ingredient can be prepared.

The liquid preparation such as emulsions and syrups, among the preparations for oral administration, can be prepared by using additives for a pharmaceutical preparation including water; a sugar such as sucrose, sorbitol and fructose; ehanol; a glycol such as polyethylene glycol and propylene glycol; an oil such as sesame oil, olive oil and soybean oil; an preservative such as p-hydroxybenzoate; a sweetening such as saccharin, a thickening agent such as carboxymethyl cellulose, a flavor such as strawberry flavor and peppermint flavor, a coloring agent and so on.

The solid preparation such as capsules, tablets, powders and granules can be prepared by appropriately using following fillers; a carrier such as lactose, glucose, sucrose sorbitol, mannitol, mannite and a cellulose derivative; a disintegrant such as starch (potato starch, corn starch, amylopectin, etc.), and sodium alginate; a lubricant such as magnesium stearate, calcium stearate, polyethylene glycol, wax, paraffin and talc; a binder such as polyvinyl alcohol, polyvinyl pyrrolidone, hydroxypropyl cellulose and gelatin; a surfactant such as a fatty acid ester; or a plasticizer such as glycerin. In case of preparation of sugar coated tablets, a condensed sugar solution, which may contain gum arabic, gelatin, talc, or titanium oxide is coated on the core of tables prepared by using fillers as described above. There can be also prepared a film coated tablet, which is coated by a suitable polymer film dissolved in an easily removable organic solvent.

In case of preparation of soft gelatin capsules, the capsules can be prepared by mixing the compound of the present invention with for example, vegetable oil or polyethylene glycol. In case of preparation of hard gelatin capsules, the capsules can be prepared by using granules of the compound of the present invention which are prepared by mixing it with a suitable carrier as described above.

The liquid preparation such as injections, drips, eye-drops and ear drops, among the preparations for parenteral administration, can be prepared preferably as a sterilized isotonic liquid preparation. For instance, injections can be prepared by using an aqueous medium such as a salt solution, a glucose solution or a mixture of a salt solution and a glucose solution. The preparation for intrarectal administration can be prepared by using a carrier such as cacao butter usually in the form of suppository.

The ointments, creams and gels contain the compound of the present invention usually in an amount of 0.01-10 w/w%, and there may be incorporated a thickener suitable to an aqueous or oily base and/or a gelling agent and/or a solvent. The base is exemplified by water and/or an oil such as liquid paraffin, a vegetable oil such as arachis oil and castor oil, a solvent such as polyethylene glycol, and so on. The thickener and gelling agent are exemplified by soft paraffin, aluminum stearate, cetostearic alcohol, polyethylene glycol, sheep fat, beeswax, carboxypolymethylene and cellulose derivatives and/or glyceryl monostearate and/or nonionic emulsifiers.

The lotions contain the compound of the present invention usually in an amount of 0.01-10 w/w%, and it may be prepared with the use of an aqueous or oily base, it may contain generally emulsifiers, stabilizers, dispersing agents, precipitation inhibitors and also thickeners.

Powders for external use contain the compound of the present invention usually an amount of 0.01-10 w/w%, and it may be formulated using a suitable powdery base such as talc, lactose and starch.

The drips may be formulated by using an aqueous or non-aqueous base, and may contain dispersing agents, solubilizing agents, precipitation inhibitors or preservatives.

The sprays (sprays, aerosols, dry-powders, etc.) may be formulated into an aqueous solution or suspension using a suitable liquid propellant, or into an aerosol distributed from a pressured package such as a metered-dose inhaler. Dry-powders preparations can be used.

The aerosols suitable to inhalation may be a suspension or aqueous solution, and they contain generally the compound of the present invention and a suitable propellant such as fluorocarbon, hydrogencontaining chlorofluorocarbon and a mixture thereof, particularly hydrofluoroalkane, specifically 1,1,1,2-tetrafluoroethane, heptafluoroalkane (HFA) such as 1,1,1,2,3,3,3-heptafluoro-n-propane or a mixture thereof. The aerosols may contain optionally additional excipients well known in the art such as a surfactant, (e.g., oleic acid or lecithin) and a co-solvent such as ethanol. For example, an inhaler known as Turubuhaler® is illustrated.

The gelatin capsules or cartridges used for inhalator or insufflator may be formulated by using a powdery mixture of the compounds used in the present invention and a powdery base such as lactose and starch. They contain the compound of the present invention usually in an amount of 20µg-10mg. The compound of the present invention may be administered without using excipients such as lactose as an alternative method.

In case of being orally or nasally inhalated in the form of pressured HFA aerosols or dry-powders preparations, the adenine compound of the present invention, or its pharmaceutically acceptable salt is pulverized in a size of less than 10µm and it is dispersed in a dispersing agent such as C₈₋₂₀ fatty acid or its salt (e.g., oleic acid), bile salt, phospholipid, an alkyl saccharide, a completely fluorinated or polyethoxylated surfactant, or a pharmaceutically acceptable dispersing agent.

The adenine compound of the present invention is preferably parenterally administered as a preparation for topical administration. For example, a compound wherein R² is an optionally substituted alkyl group, an optionally substituted alkenyl group or an optionally substituted cycloalkyl group is illustrated. The suitable preparation is exemplified by ointments, lotions(solutions or suspensions), creams, gels, tapes, transdermal patches, cataplasms, sprays, aerosols, dry-powders, aqueous solutions/suspensions for cartridge spray for inhalators or insufflators, eye-drops, ear drops, nasal drops, transdermal agents, pulmonary absorbent, air-way absorbent, powders for external administrations and so on.

A ratio of the active compound of the present invention in the preparation for topical administration of the present invention is, though depending upon the formulation, generally 0.001-10 wt%, preferably 0.005-1%. The ratio used in powders for inhalation or insufflation is 0.1-5%.

In a case of aerosols, the compound of the present invention is preferably contained in an amount of 20-2000µg, more preferably about 20µg-500µg per each a measured amount or one sprayed amount. The dosage is once or several times per day, for instance, 2, 3, 4 or 8 times, and one to three units are administered per each time.

The pharmacological activity can be measured by any of conventional evaluation methods, preferably by an in vitro evaluation method. An example of the methods is a method described in examples of the present specification.

The invention further relates to combination therapies wherein a compound of the formula (1) or its pharmaceutically acceptable salt or a pharmaceutical composition comprising a compound of the formula (1) or its pharmaceutically acceptable salt is administered concurrently or sequentially or as a combined preparation with other therapeutic agent(s), for the treatment of one or more of the conditions listed in the specification.

In particular, for the treatment of the inflammatory diseases, COPD, asthma and allergic rhinitis, the compounds of the invention may be combined with agents such as tumour necrosis factor alpha (TNF-α) inhibitors such as anti-TNF monoclonal antibodies (for example Remicade, CDP-870 and adalimumab) and TNF receptor immunoglobulin molecules (such as Enbrel); non-selective cyclo-oxygenase COX-1/COX-2 inhibitors whether applied topically or systemically (such as piroxicam, diclofenac, propionic acids such as naproxen, flubiprofen, fenoprofen, ketoprofen and ibuprofen, fenamates such as mefenamic acid, indomethacin, sulindac, azapropazone, pyrazolones such as phenylbutazone, salicylates such as aspirin), COX-2 inhibitors (such as meloxicam, celecoxib, rofecoxib, valdecoxib, lumarocoxib, parecoxib and etoricoxib); glucocorticosteroids (whether administered by topical, oral, intramuscular, intravenous, or intra-articular routes); methotrexate, leflunomide; hydroxychloroquine, d-penicillamine, auranofin or other parenteral or oral gold preparations.

The present invention still further relates to combination therapies of a compound of the invention together with a leukotriene biosynthesis inhibitor, 5-lipoxygenase (5-LO) inhibitor or 5-lipoxygenase activating protein (FLAP) antagonist such as; zileuton; ABT-761; fenleuton; tepoxalin; Abbott-79175; Abbott-85761; N-(5-substituted)-thiophene-2-alkylsulfonamides; 2,6-di-tert-butylphenol hydrazones; methoxytetrahydropyrans such as Zeneca ZD-2138; SB-210661; pyridinyl-substituted 2-cyanonaphthalene compounds such as L-739,010; 2-cyanoquinoline compounds such as L-746,530; MK-591, MK-886, and BAY-x-1005.

The present invention still further relates to combination therapies of a compound of the invention together with a receptor antagonist for leukotrienes (LT)B4, LTC4, LTD4 and LTE4 selected from the group consisting of phenothiazin compound such as L-651,392; amidino compounds such as CGS-25019C; benzoxalamines such as ontazolast; benzenecarboximidamides such as BIIL 284/260; and compounds such as zafirlukast, ablukast, montelukast, pranlukast, verlukast (MK-679), RG-12525, Ro-245913, iralukast (CGP 45715A), and BAY-x-7195.

The present invention still further relates to combination therapies of a compound of the invention together with a phosphodiesterase (PDE) inhibitor such as the methylxanthanines including theophylline and aminophylline; and selective PDE isoenzyme inhibitors including PDE4 inhibitors and inhibitors of isoform PDE4D, and inhibitors ofPDES.

The present invention still further relates to combination therapies of a compound of the invention together with histamine type 1 receptor antagonists such as cetirizine, loratadine, desloratadine, fexofenadine, acrivastine, terfenadine, astemizole, azelastine, levocabastine, chlorpheniramine, promethazine, cyclizine, or mizolastine, which is applied orally, topically or parenterally.

The present invention still further relates to combination therapies of a compound of the invention together with a gastroprotective histamine type 2 receptor antagonist.

The present invention still further relates to combination therapies of a compound of the invention with an antagonist of the histamine type 4 receptor.

The present invention still further relates to combination therapies of a compound of the invention together with an alpha-1/alpha-2 adrenoceptor agonist, vasoconstrictor sympathomimetic agent, such as propylhexedrine, phenylephrine, phenylpropanolamine, ephedrine, pseudoephedrine, naphazoline hydrochloride, oxymetazoline hydrochloride, tetrahydrozoline hydrochloride, xylometazoline hydrochloride, tramazoline hydrochloride, or ethylnorepinephrine hydrochloride.

The present invention still further relates to combination therapies of a compound of the invention together with an anticholinergic agent including muscarinic receptor (M1, M2 and M3) antagonists such as atropine, hyoscine, glycopyrrolate, ipratropium bromide; tiotropium bromide; oxitropium bromide; pirenzepine; or telenzepine.

The present invention still further relates to combination therapies of a compound of the invention together with a beta-adrenoceptor agonist (including beta receptor subtypes 1-4) such as isoprenaline, salbutamol, formoterol, salmeterol, terbutaline, orciprenaline, bitolterol mesylate, or pirbuterol.

The present invention still further relates to combination therapies of a compound of the invention together with a chromone, such as sodium cromoglycate or nedocromil sodium.

The present invention still further relates to combination therapies of a compound of the invention together with an insulin-like growth factor type I (IGF-1) mimetic.

The present invention still further relates to combination therapies of a compound of the invention together with an inhaled glucocorticoid, such as flunisolide, triamcinolone acetonide, beclomethasone dipropionate, budesonide, fluticasone propionate, ciclesonide, or mometasone furoate.

The present invention still further relates to combination therapies of a compound of the invention together with an inhibitor of matrix metalloproteases, i.e., an inhibitor of stromelysin, collagenase, gelatinase, aggrecanase; especially collagenase-1 (MMP-1), collagenase-2 (MMP-8), collagenase-3 (MMP-13), stromelysin-1 (MMP-3), stromelysin-2 (MMP-10), stromelysin-3 (MMP-11), MMP-9 or MMP-12.

The present invention still further relates to combination therapies of a compound of the invention together with modulators of chemokine receptor function such as antagonists of CCR1, CCR2, CCR2A, CCR2B, CCR3, CCR4, CCR5, CCR6, CCR7, CCR8, CCR9, CCR10 and CCR11 (for the C-C family); CXCR1, CXCR2, CXCR3, CXCR4 and CXCR5 (for the C-X-C family) and CX3CR1 (for the C-X3-C family).

The present invention still further relates to combination therapies of a compound of the invention together with a cytokine or a modulator of cytokine function including agents which act on cytokine signalling pathways, such as alpha-, beta-, and gamma-interferon; interleukins (IL) including IL-1 to IL-15, and interleukin antagonists or inhibitors.

The present invention still further relates to combination therapies of a compound of the invention together with an immunoglobulin (Ig), an Ig preparation, or an antagonist or antibody modulating Ig function such as anti-IgE (omalizumab).

The present invention still further relates to combination therapies of a compound of the invention together with systemic or topically-applied anti-inflammatory agents such as thalidomide or its derivatives, retinoids, dithranol, or calcipotriol.

The present invention still further relates to combination therapies of a compound of the invention together with an antibacterial agent including penicillin derivatives, tetracyclines, macrolides, beta-lactams, flouroquinolones, metronidazole and inhaled aminoglycosides; an antiviral agent including acyclovir, famciclovir, valaciclovir, ganciclovir, cidofovir, amantadine, rimantadine, ribavirin; zanamavir or oseltamavir; enzyme inhibitors such as indinavir, nelfinavir, ritonavir, or saquinavir; nucleoside reverse transcriptase inhibitors such as didanosine, lamivudine, stavudine, zalcitabine and zidovudine; or non-nucleoside reverse transcriptase inhibitors such as nevirapine or efavirenz.

The present invention still further relates to combination therapies of a compound of the invention together with agents used for treatment of cancers. Suitable agents to be used in the combination therapies include:
(i) antiproliferative/antineoplastic drugs and combinations thereof, which are used as an anticancer agent, such as alkylating agents (for example cisplatin, carboplatin, cyclophosphamide, nitrogen mustard, melphalan, chlorambucil, busulphan or nitrosoureas); antimetabolites (for example fluoropyrimidines, like 5-fluorouracil and tegafur, antifolates such as raltitrexed, methotrexate, cytosine arabinoside, hydroxyurea, gemcitabine or paclitaxel); antitumour antibiotics (for example anthracyclines, such as adriamycin, bleomycin, doxorubicin, daunomycin, epirubicin, idarubicin, mitomycin-C, dactinomycin or mithramycin); antimitotic agents (for example vinca alkaloids, such as vincristine, vinblastine, vindesine or vinorelbine and taxoids, such as taxol and taxotere); or topoisomerase inhibitors (for example epipodophyllotoxins, such as like etoposide, teniposide, amsacrine, topotecan or camptothecins);
(ii) cytostatic agents such as antiestrogens (for example tamoxifen, toremifene, raloxifene, droloxifene or iodoxyfene), estrogen receptor down regulators (for example fulvestrant), antiandrogens (for example bicalutamide, flutamide, nilutamide and cyproterone acetate), LHRH antagonists or LHRH agonists (for example goserelin, leuprorelin or buserelin), progestogens (for example megestrol acetate), aromatase inhibitors (for example as anastrozole, letrozole, vorazole or exemestane) and inhibitors of 5α-reductase such as finasteride;
(iii) agents which inhibit cancer cell invasion (for example metalloproteinase inhibitors, such as marimastat or inhibitors of urokinase plasminogen activator receptor function);
(iv) inhibitors of growth factor function, for example such inhibitors include growth factor antibodies, growth factor receptor antibodies (for example the anti erbb2 antibody trastuzumab or the anti erbb 1 antibody cetuximab [C225]), farnesyl transferase inhibitors, tyrosine kinase inhibitors or serine/threonine kinase inhibitors; for example inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib, AZD1839), N-(3-ethynylphenyl)-6,7-bis(2-methoxyethoxy)quinazolin-4-amine (erlotinib, OSI 774) or 6-acrylamido-N-(3-chloro-4-fluorophenyl)-7-(3-morpholinopropoxy)quinazolin-4-amine (CI 1033)); for example inhibitors of the platelet-derived growth factor family; or for example inhibitors of the hepatocyte growth factor family;
(v) antiangiogenic agents such as those which inhibit the effects of vascular endothelial growth factor, (for example the anti vascular endothelial cell growth factor antibody bevacizumab, compounds disclosed in WO 97/22596, WO 97/30035, WO 97/32856 or WO 98/13354) or compounds that work by other mechanisms (for example linomide, inhibitors of integrin αvβ3 function or angiostatin);
(vi) vascular damaging agents such as combretastatin A4 or compounds disclosed in WO 99/02166, WO00/40529, WO 00/41669, WO 01/92224, WO 02/04434 and WO 02/08213;
(vii) antisense therapies, for example those which are directed to the targets listed above, such as ISIS 2503, an anti-ras antisense;
(viii) gene therapy approaches, including for example approaches to replace aberrant genes such as aberrant p53 or aberrant BRCA1 or BRCA2, GDEPT (gene directed enzyme pro-drug therapy) approaches such as those using cytosine deaminase, thymidine kinase or a bacterial nitroreductase enzyme and approaches to increase patient tolerance to chemotherapy or radiotherapy such as multi drug resistance gene therapy; or
(ix) immunotherapy approaches, including for example ex vivo and in vivo approaches to increase the immunogenicity of patient tumour cells, such as exposure with cytokines such as interleukin 2, interleukin 4 or granulocyte macrophage colony stimulating factor (GM-CSF), approaches to decrease T cell anergy, approaches using transplanted immune cells such as cytokine exposed dendritic cells, approaches using cytokine exposed tumour cell lines and approaches using anti idiotypic antibodies.

### Example

The present invention is illustratively described in the following examples, but should be not limited by these examples.

### Example 1

### 2-Butoxy-7,8-dihydro-9-[5-(4-methoxylcarbonylpiperidin-1-yl)pentyl]-8-oxoadenine

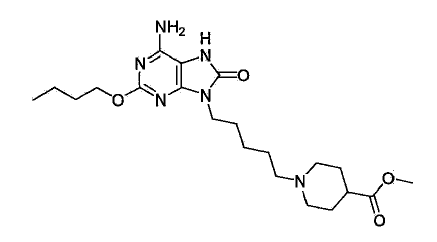

### Step (i)

### 9-(5-Bromopentyl)-2-butoxy-8-methoxyadenine

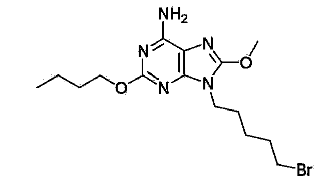

To 2-butoxy-8-methoxyadenine 2.00g (8.43mmol) in a DMF (30ml) were added potassium carbonate 1.40g (10.1mmol) and 1,5-dibromopentane 3.87g (16.9mmol), and the mixture was stirred at room temperature for 6 hours. After removal of the solvent, water 80ml was added thereto and the mixture was extracted with 5% methanol-chloroform (100ml). The organic layer was washed with water and saturated brine, successively, dried over sodium sulfate, concentrated in vacuo and purified with silica gel chromatography to give the object compound 1.69g as a pale pink solid. Yield 52%
¹H NMR (DMSO-d₆) δ 6.78 (2H, bs), 4.16 (2H, t, J= 6.6 Hz), 4.03 (3H, s), 3.84 (2H, t, J= 6.8 Hz), 1.86-1.78 (2H, m), 1.74-1.60 (4H, m), 1.45-1.35 (2H, m), 1.35-1.28 (2H, m), 0.92 (3H, t, J= 7.4 Hz).

### Step (ii)

### 2-Butoxy-8-methoxy-9-[5-(4-methoxylcarbonylpiperidin-1-yl)pentyl]adenine

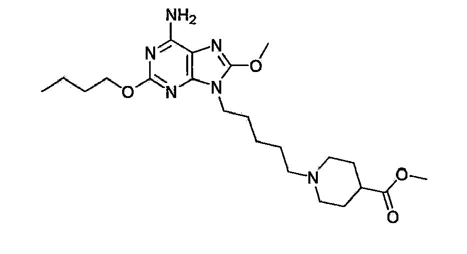

To the compound 300mg (0.78mmol) obtained in step (i) in DMF (10ml) were added potassium carbonate 322 mg (2.33mmol) and methyl isonipecotate 222mg (1.55mmol), and the mixture was stirred for 15 hours at room temperature. After removal of the solvent, thereto was added water 80ml and the mixture was extracted with 5% methanol-chloroform (100ml). The organic layer was washed with water, and saturated brine, successively and dried over sodium sulfate. After being concentrated in vacuo, the residue was purified by silica gel chromatography to give the object compound 240mg as a colorless oil. Yield 69%
¹H NMR (DMSO-d₆) δ 6.77 (2H, bs), 4.16 (2H, t, J= 6.6 Hz), 4.03 (3H, s), 3.82 (2H, t, J= 6.8 Hz), 3.58 (3H, s), 2.73-2.68 (2H, m), 2.29-2.21 (1H, m), 2.18 (2H, t, J= 7.2 Hz), 1.89-1.81 (2H, m), 1.78-1.71 (2H, m), 1.71-1.60 (4H, m), 1.55-1.46 (2H, m), 1.46-1.35 (4H, m), 1.23-1.14 (2H, m), 0.92 (3H, t, J= 7.4 Hz).

### Step (iii)

### 2-Butoxy-7,8-dihydro-9-[5-(4-methoxylcarbonylpiperidin-1-yl)pentyl]-8-oxoadenine)

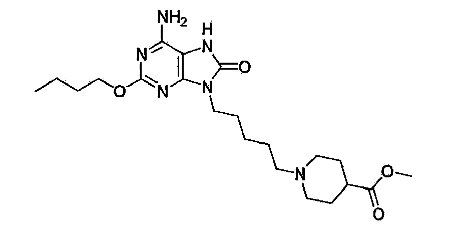

To the compound 240mg (0.54mmol) obtained in step (ii) in methanol (5ml) was added concentrated sulfuric acid (200µl) and the mixture was stirred under refluxing for 4 hours. After the mixture was neutralized with 28% aqueous ammonia, the solvent was removed. To the residue was added water and the resulting solid was filtered and purified by silica gel chromatography to give the object compound 160mg as a white solid. Yield 69%
¹H NMR (DMSO-d₆) δ 9.83 (1H, bs), 6.39 (1H, bs), 4.13 (2H, t, J= 6.6 Hz), 3.65 (2H, t, J= 6.8 Hz), 3.58 (3H, s), 3.75-3.69 (2H, m), 2.30-2.20 (1H, m), 2.17 (2H, t, J= 7.2 Hz), 1.89-1.82 (2H, m), 1.78-1.72 (2H, m), 1.68-1.59 (4H, m), 1.55-1.45 (2H, m), 1.45-1.32 (4H, m), 1.24-1.16 (2H, m), 0.91 (3H, t, J= 7.4 Hz).

### Example 2

### 2-Butoxy-7,8-dihydro-9-[5-(4-methoxylcarbonylmethylpiperidin-1-yl)pentyl]-8-oxoadenine

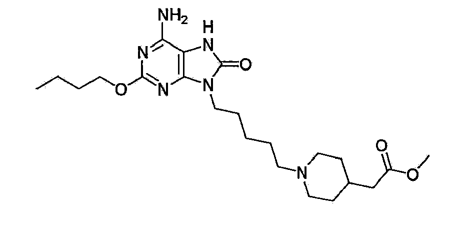

### Step (i)

### 2-Butoxy-8-methoxy-9-[5-(4-methoxylcarbonylmethylpiperidin-1-yl)pentyl]adenine

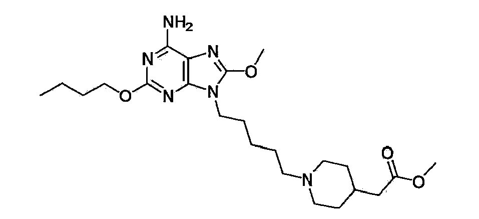

Using the compound 300mg (0.78mmol) obtained in step (i) of example 1, in the same manner as step (ii) of example 1, there was obtained the object compound 281mg as a pale pink oil. Yield 78% ¹H NMR (DMSO-d₆) δ 6.77 (2H, bs), 4.15 (2H, t, J= 6.6 Hz), 4.04 (3H, s), 3.82 (2H, t, J= 6.8 Hz), 3.57 (3H, s), 2.76-2.71 (2H, m), 2.20 (2H, d, J= 6.8 Hz), 2.16 (2H, t, J= 7.3 Hz), 1.80-1.72 (2H, m), 1.72-1.60 (4H, m), 1.59-1.52 (2H, m), 1.45-1.35 (5H, m), 1.23-1.14 (2H, m), 1.14-1.05 (2H, m), 0.92 (3H, t, J= 7.4 Hz).

### Step (ii)

### 2-Butoxy-7,8-dihydro-9-[5-(4-methoxylcarbonylmethylpiperidin-1-yl)pentyl]-8-oxoadenine

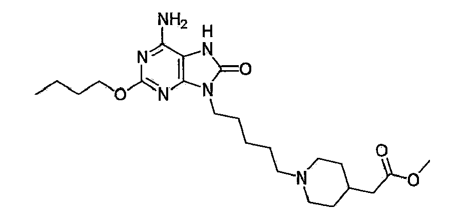

Using the compound 280mg (0.61mmol) obtained in step (i) of example 2, in the same manner as step (iii) of example 1, there was obtained the object compound 230mg as a pale pink oil. Yield 85% ¹H NMR (DMSO-d₆) δ 9.85 (1H, bs), 6.41 (2H, bs), 4.13 (2H, t, J= 6.6 Hz), 3.65 (2H, t, J= 6.8 Hz), 3.58 (3H, s), 2.92-2.76 (2H, m), 2.33-2.18 (4H, m), 1.72-1.59 (7H, m), 1.55-1.44 (2H, m), 1.44-1.32 (4H, m), 1.27-1.18 (4H, m), 0.91 (3H, t, J= 7.4 Hz).

### Example 3

### 2-Butoxy-7,8-dihydro-9-[5-(3-methoxylcarbonylmethylpiperidin-1-yl)pentyl]-8-oxoadenine

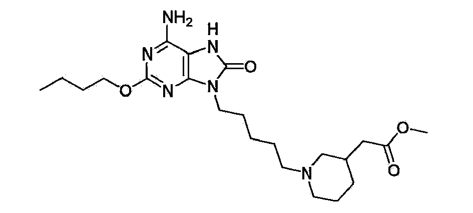

### Step (i)

### 2-Butoxy-9-[5-(3-ethoxylcarbonylmethylpiperidin-1-yl)pentyl]-8-methoxyadenine

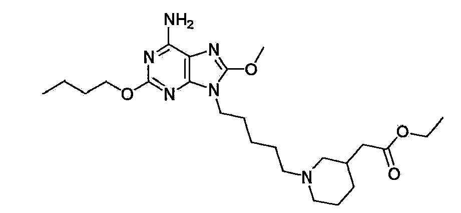

Using the compound 300mg (0.78mmol) obtained in step (i) of example 1, in the same manner as step (ii) of example 1, there was obtained the object compound 264mg as a pale pink oil. Yield 71% ¹H NMR (DMSO-d₆) δ 6.77 (2H, bs), 4.16 (2H, t, J= 6.6 Hz), 4.04 (3H, s), 4.03 (2H, q, J= 7.1 Hz), 3.82 (2H, t, J= 6.9 Hz), 2.67-2.57 (2H, m), 2.22-2.10 (4H, m), 1.89-1.75 (2H, m), 1.72-1.50 (7H, m), 1.45-1.31 (6H, m), 1.23-1.13 (2H, m), 1.16 (3H, t, J= 7.1 Hz), 0.92 (3H, t, J= 7.4 Hz).

### Step (ii)

### 2-Butoxy-7,8-dihydro-9-[5-(3-methoxylcarbonylmethylpiperidin-1-yl)pentyl]-8-oxoadenine

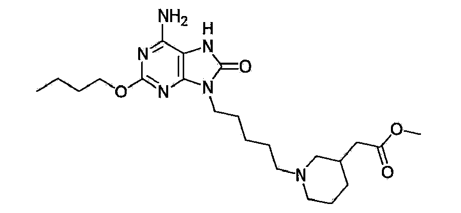

Using the compound 260mg (0.55mmol) obtained in step (ii) of example 3, in the same manner as step (iii) of example 1, there was obtained the object compound 160mg as a pale pink oil. Yield 65% ¹H NMR (DMSO-d₆) δ 9.83 (1H, bs), 6.39 (2H, bs), 4.13 (2H, t, J= 6.6 Hz), 3.64 (2H, t, J= 6.8 Hz), 3.58 (3H, s), 2.74-2.60 (2H, m), 2.27-2.12 (4H, m), 1.94-1.78 (2H, m), 1.70-1.50 (7H, m), 1.49-1.31 (5H, m), 1.26-1.16 (2H, m), 0.95-0.89 (1H, m), 0.91 (3H, t, J= 7.4 Hz).

### Example 4

### 2-Butoxy-7,8-dihydro-9-[2-(4-methoxycarbonylpiperidin-1-yl)ethyl]-8-oxoadenine

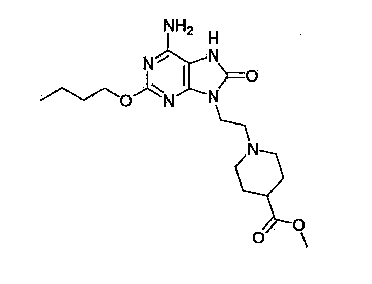

### Step (i)

### 9-(2-Bromoethyl)-2-butoxy-8-methoxyadenine

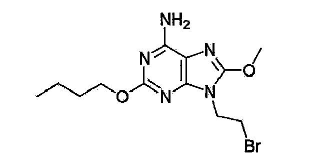

Using 2-butoxy-8-methoxyadenine 500mg (2.1 1mmol), in the same manner as step (i) of example 1, there was obtained the object compound 573mg as a white solid. Yield 79% ¹H NMR (CDCl₃) δ 5.31 (2H, brs), 4.32 (2H, t, J= 7.0 Hz), 4.27 (2H, t, J= 6.7 Hz), 4.12 (3H, s), 3.66 (2H, t, J= 7.0 Hz), 1.79-1.72 (2H, m), 1.52-1.46 (2H, m), 0.95 (3H, t, J= 7.4 Hz).

### Step (ii)

### 2-Butoxy-8-methoxy-9-[2-(4-methoxycarbonylpiperidin-1-yl)ethyl]adenine

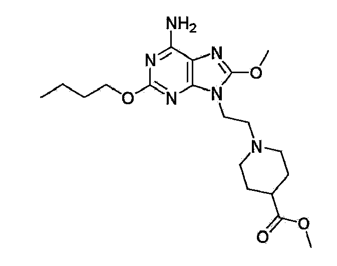

Using the compound 200mg (0.58mmol) obtained in step (i), in the same manner as step (ii) of example 1, there was obtained the object compound 99mg as a white solid. Yield 42% ¹H NMR (CDCl₃) δ 5.15 (2H, brs), 4.26 (2H, t, J= 6.7 Hz), 4.11 (3H, s), 4.04 (2H, t, J= 6.8 Hz), 3.67 (3H, s), 2.93-2.88 (2H, m), 2.69 (2H, t, J= 6.8 Hz), 2.28-2.23 (1H, m), 2.15-2.08 (2H, m), 1.87-1.64 (6H, m), 1.52-1.46 (2H, m), 0.96 (3H, t, J= 7.4 Hz).

### Step (iii)

### 2-Butoxy-7,8-dihydro-9-[2-(4-methoxycarbonylpiperidin-1-yl)ethyl]-8-oxoadenine

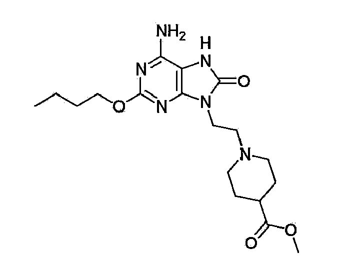

To the compound 99mg (0.24mmol) obtained in step (ii) was added hydrochloric acid-methanol (20ml) and the mixture was stirred at room temperature for 12 hours. The mixture was concentrated in vacuo, and to the residue was added water. After the mixture was extracted with chloroform and the organic layer was dried over sodium sulfate. After removal of the solvent, to the residue was added hexane and the resulting crystals were filtered to give the object compound 38mg as a white solid.
Yield 40%
¹H NMR (DMSO-d₆) δ 9.90 (1H, brs), 6.42 (2H, brs), 4.14 (2H, t, J= 6.6 Hz), 3.76 (2H, t, J= 6.3 Hz), 3.58 (3H, s), 2.88-2.83 (2H, m), 2.55 (2H, t, J= 6.8 Hz), 2.32-2.25 (1H, m), 2.08-1.95 (2H, m), 1.75-1.37 (8H, m), 0.91 (3H, t, J= 7.4 Hz).

### Example 5

### 2-Butoxy-7,8-dihydro-9-[2-(3-methoxycarbonylpiperidin-1-yl)ethyl]-8-oxoadenine

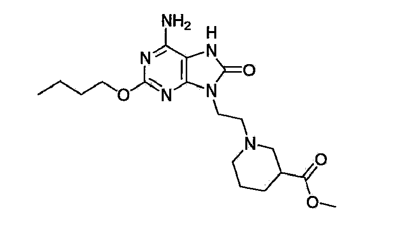

### Step (i)

### 2-Butoxy-9-[2-(3-ethoxycarbonylpiperidin-1-yl)ethyl]-8-methoxyadenine

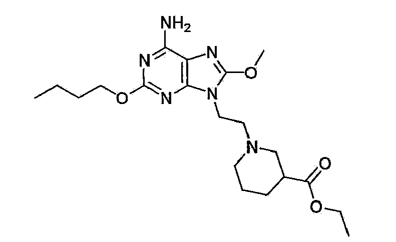

Using the compound 200mg (0.58mmol) obtained in step (i) of example 4, in the same manner as step (ii) of example 1, there was obtained the object compound 162mg as a white solid. Yield 66% ¹H NMR (CDCl₃) δ 5.19 (2H, brs), 4.26 (2H, t, J= 6.6 Hz), 4.11 (3H, s), 4.39 (2H, q, J= 7.1 Hz), 4.04 (2H, t, J= 6.7 Hz), 3.67 (3H, s), 3.09 (1H, m), 2.81 (1H, m), 2.69 (2H, t, J= 6.7 Hz), 2.50-2.42 (1H, m), 2.21 (1H, m), 2.08 (1H, m), 1.80-1.38 (8H, m), 1.23 (3H, t, J= 7.1 Hz), 0.96 (3H, t, J= 7.4 Hz).

### Step (ii)

### 2-Butoxy-7,8-dihydro-9-[2-(3-methoxycarbonylpiperidin-1-yl)ethyl]-8-oxoadenine

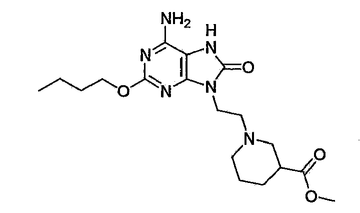

Using the compound 162mg (0.39mmol) obtained in step (i), in the same manner as step (iii) of example 1, there was obtained the object compound 123mg as a white solid. Yield 81% ¹H NMR (DMSO-d₆) δ 9.87(1H, brs), 6.40 (2H, brs), 4.15-4.11 (2H, m), 3.79-3.73 (2H, m), 3.56 (3H, s), 2.94 (1H, m), 2.70 (1H, m), 2.61-2.56 (2H, m), 2.41 (1H, m), 2.1b (1H, m), 2.08 (1H, m), 1.80-1.34 (8H, m), 0.92 (3H, t, J= 7.4 Hz).

### Example 6

### 2-Butoxy-7,8-dihydro-9-{2-(2-methoxylcarbonylpiperidin-1-yl)ethyl}-8-oxoadenine

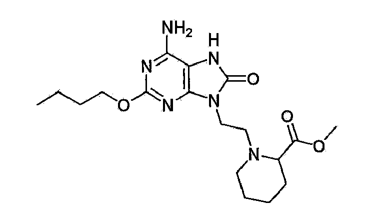

### Step (i)

### 2-Butoxy-9-{2-(2-ethoxylcarbonylpiperidin-1-yl)ethyl}-8-methoxyadenine

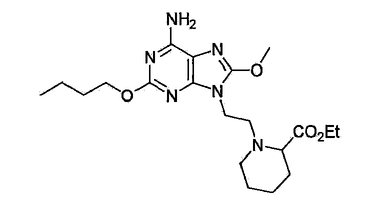

Using the compound 400mg (1.16mmol) obtained in step (i) of example 4, in the same manner as step (ii) of example 1, there was obtained the object compound 425mg as a white solid. Yield 87%

### Step (ii)

### 2-Butoxy-7,8-dihydro-9-{2-(2-methoxylcarbonylpiperidin-1-yl)ethyl}-8-oxoadenine

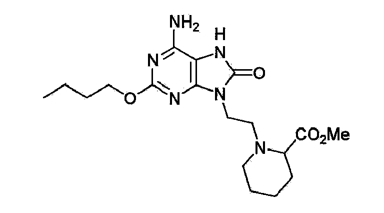

To the compound 425 mg (0.98 mmol) obtained in step (i) was added aqueous 1N NaOH solution (3 ml) and the mixture was stirred at 100°C for 5 hours. After neutralization with concentrated hydrochloric acid, the solvent was removed. To the residue were added methanol 5ml, and concentrated sulfuric acid 600µl and the mixture was stirred at 90°C for 17 hours. After neutralization with 28% aqueous ammonia, the solvent was removed. After addition of water, the resulting white solid was filtered. The white solid was purified by column chromatography to give the object compound 110mg (0.29mmol) as a white solid. Yield 29% ¹H NMR (DMSO-d₆) δ 9.83 (1H, bs), 6.38 (1H, bs), 4.13 (2H, t, J= 6.6 Hz), 3.78-3.59 (2H, m), 3.51 (2H, s), 3.28-3.23 (1H, m), 3.06-2.94 (1H, m), 2.82-2.73 (1H, m), 2.65-2.57 (1H, m), 2.38-2.29 (1H, m), 1.69-1.49 (5H, m), 1.49-1.30 (5H, m), 0.90 (3H, t, J= 7.4 Hz).

### Example 7

### 2-Butoxy-7,8-dihydro-9-[2-{4-(2-methoxycarbonylethyl)piperazin-1-yl}ethyl]-8-oxoadenine

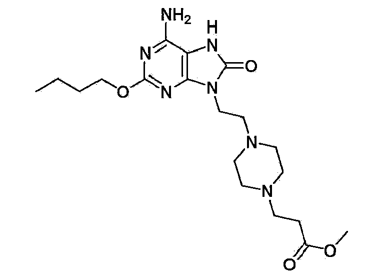

### Step (i)

### 2-Butoxy-8-methoxy-9-[2-{4-(2-methoxylcarbonylethyl)piperazin-1-yl}ethyl]adenine

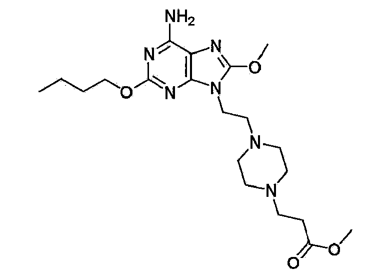

Using the compound 300mg (0.87mmol) obtained in step (i) of example 4, in the same manner as step (ii) of example 1, there was obtained the object compound 169mg as a colorless oil. Yield 45% ¹H NMR (DMSO-d₆) δ 6.76 (1H, bs), 4.14 (2H, t, J= 6.6 Hz), 4.03 (3H, s), 3.92 (2H, t, J= 6.4 Hz), 3.57 (3H, s), 2.57 (2H, t, J= 6.4 Hz), 2.50-2.47 (2H, m), 2.47-2.34 (6H, m), 2.34-2.23 (4H, m), 1.68-1.60 (2H, m), 1.44-1.34 (2H, m), 0.92 (3H, t, J= 7.4 Hz).

### Step (ii)

### 2-Butoxy-7,8-dihydro-9-[2-{4-(2-methoxycarbonylethyl)piperazin-1-yl}ethyl]-8-oxoadenine

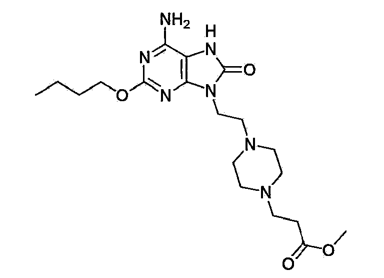

Using the compound 160mg (0.37mmol) obtained in step (i), in the same manner as step (iii) of example 1, there was obtained the object compound 90mg as a white solid. Yield 58% ¹H NMR (DMSO-d₆) 6 9.84 (1H, bs), 6.40 (1H, bs), 4.13 (2H, t, J= 6.6 Hz), 3.77 (2H, t, J= 6.0 Hz), 3.57 (3H, s), 2.59-2.53 (2H, m), 2.53-2.48 (2H, m), 2.47-2.22 (10H, m), 1.67-1.59 (2H, m), 1.43-1.33 (2H, m), 0.91 (3H, t, J= 7.4 Hz).

### Example 8

### 2-Butoxy-7,8-dihydro-9-[3-(4-methoxycarbonylpiperidin-1-yl)propyl]-8-oxoadenine

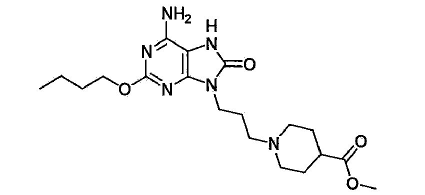

### Step (i)

### 9-(3-Bromopropyl)-2-butoxy-8-methoxyadenine

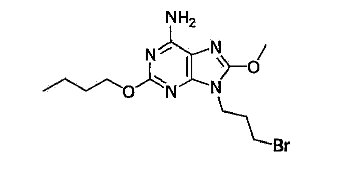

Using 2-butoxy-8-methoxyadenine 2.00g (8.43mmol), in the same manner as step (i) of example 1, there was obtained the object compound 0.75g as a white solid. Yield 25% ¹H NMR (CDCl₃) δ 5.21 (2H, brs), 4.28 (2H, t, J= 6.6 Hz), 4.12 (3H, s), 4.09 (2H, t, J= 6.6 Hz), 3.38 (2H, t, J= 6.6 Hz), 2.36-2.32 (2H, m), 1.79-1.73 (2H, m), 1.52-1.46 (2H, m), 0.96 (3H, t, J= 7.4 Hz).

### Step (ii)

### 2-Butoxy-8-methoxy-9-[3-(4-methoxycarbonylpiperidin-1-yl)propyl]adenine

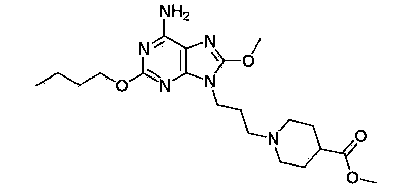

Using the compound 150mg (0.42mmol) obtained in step (i), in the same manner as step (ii) of example 1, there was obtained the object compound 120mg as a white solid. Yield 68% ¹H NMR (CDCl₃) δ 5.24 (2H, brs), 4.26 (2H, t, J= 6.6 Hz), 4.10 (3H, s), 3.97 (2H, t, J= 7.0 Hz), 3.67 (3H, s), 2.83-2.79 (2H, m), 2.34 (2H, t, J= 7.0 Hz), 2.31-2.24 (1H, m), 2.00-1.51 (10H, m), 1.52-1.46 (2H, m), 0.96 (3H, t, J= 7.4 Hz).

### Step (iii)

### 2-Butoxy-7,8-dihydro-9-[3-(4-methoxyearbonylpiperidin-1-yl)propyl]-8-oxoadenine

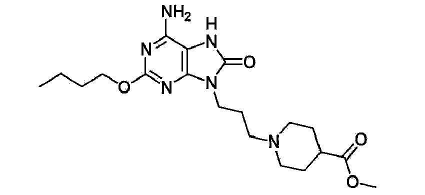

Using the compound 120mg (0.29mmol) obtained in step (ii), in the same manner as step (iii) of example 1, there was obtained the object compound 96mg as a white solid. Yield 83%
¹H NMR (DMSO-d₆) δ 9.90 (1H, brs), 6.41 (2H, brs), 4.14 (2H, t, J= 6.6 Hz), 3.69 (2H, t, J= 6.9 Hz), 3.58 (3H, s), 2.75-2.71 (2H, m), 2.28-2.23 (3H, m), 1.87-1.35 (12H, m), 0.92 (3H, t, J= 7.4 Hz).

### Example 9

### 2-Butoxy-7,8-dihydro-9-[3-(3-methoxycarbonylpiperidin-1-yl)propyl]-8-oxoadenine

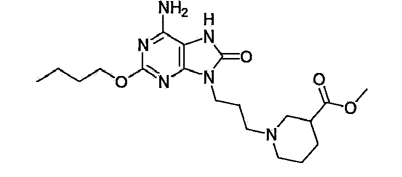

### Step (i)

### 2-Butoxy-9-[3-(3-ethoxycarbonylpiperidin-1-yl)propyl]-8-methoxyadenine

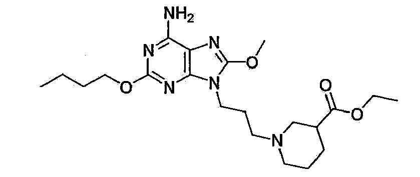

Using the compound 150mg (0.42mmol) obtained in step (i) of example 9, in the same manner as step (ii) of example 1, there was obtained the object compound 174mg as a white solid. Yield 96% ¹H NMR (CDCl₃) δ 5.19 (2H, brs), 4.27 (2H, t, J= 6.7 Hz), 4.11 (2H, q, J= 7.1 HZ), 4.10 (3H, s), 3.97 (2H, t, J= 7.0 Hz), 2.92 (1H, m), 2.70 (1H, m), 2.51-2.42 (1H, m), 2.38 (2H, t, J= 7.4 Hz), 1.96 (1H, m), 1.94-1.45 (11H, m), 1.25 (3H, t, J= 7.1 Hz), 0.96 (3H, t, J= 7.4 Hz).

### Step (ii)

### 2-Butoxy-7,8-dihydro-9-[3-(3-methoxycarbonylpiperidin-1-yl)propyl]-8-oxoadenine

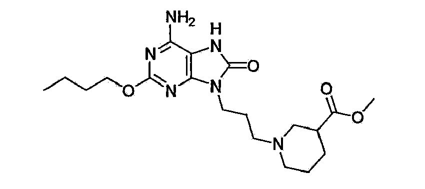

Using the compound 174mg (0.40mmol) obtained in step (i), in the same manner as step (iii) of example 1, there was obtained the object compound 98mg as a white solid. Yield 60% ¹H NMR (DMSO-d₆) δ 9.86 (1H, brs), 6.40 (2H, brs), 4.14 (2H, t, J= 6.6 Hz), 3.67 (2H, t, J= 6.9 Hz), 3.58 (3H, s), 2.75 (1H, m), 2.62 (1H, m), 2.39 (1H, m), 2.29-2.25 (2H, m), 2.07 (1H, m), 1.88 (1H, m), 1.82-1.32 (10H, m), 0.92 (3H, t, J= 7.4 Hz).

### Example 10

### 2-Butoxy-7,8-dihydro-9-[3-(2-methoxycarbonylpiperidin-1-yl)propyl]-8-oxoadenine

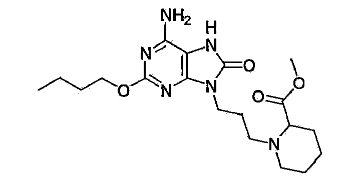

To the compound 31mg (0.079mmol) obtained by example 46 in methanol (20ml) was added concentrated sulfuric acid (2ml) and the mixture was refluxed for 36 hours. After being cooled, the mixture was neutralized with ammonia and evaporated to dryness in vacuo. To the residue was added water and the resulting crystals were filtered to give the object compound 17mg as a white solid. Yield 53% ¹H NMR (DMSO-d₆) δ 6.39 (2H, brs), 4.14 (2H, t, J= 6.6 Hz), 3.82-3.56 (2H, m), 3.55 (3H, s), 3.20 (1H, m), 2.83 (1H, m), 2.55-1.34 (15H, m), 0.92 (3H, t, J= 7.4 Hz).

### Example 11

### 2-Butoxy-7,8-dihydro-9-{3-[4-(2-methoxy-2-oxoethyl)piperidin-1-yl]propyl}-8-oxoadenine

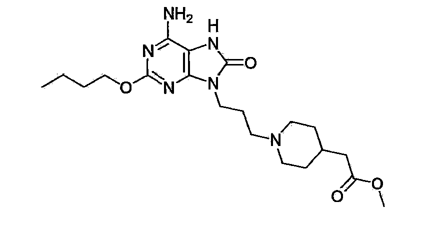

### Step (i)

### Methyl piperidin-4-ylacetate hydrochloride

To methyl 1-Boc-piperidin-4-ylacetate hydrochloride 1.00g (3.89mmol) was added 4 N hydrochloric acid-dioxane (20ml) and the mixture was stirred for 4 hours at room temperature, followed by concentration in vacuo. To the residue was added hexane and the resulting crystals were filtered to give the object compound 0.57g as a white solid. Yield 76% ¹H NMR (DMSO-d₆) δ 8.76 (1H, brs), 3.60 (3H, s), 3.21 (2H, m), 2.85 (2H, m), 2.30 (2H, d, J= 7.0 Hz), 1.95 (1H, m), 1.78 (2H, m), 1.37 (2H, m).

### Step (ii)

### 2-Butoxy-8-methoxy-9-{3-[4-(2-methoxy-2-oxoethyl)piperidin-1-yl]propyl}adenine

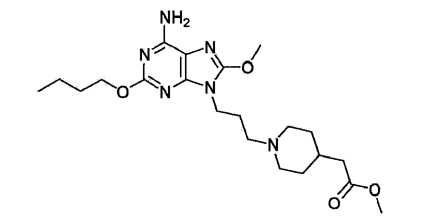

Using the compound 150mg (0.42mmol) obtained in step (i) of example 8 and the compound 162mg (0.84mmol) obtained in step (i) of example 11, in the same manner as step (ii) of example 1, there was obtained the object compound 102mg as a white solid. Yield 56% ¹H NMR (CDCl₃) δ 5.11 (2H, brs), 4.27 (2H, t, J= 6.7 Hz), 4.10 (3H, s), 3.97 (2H, t, J= 7.1 Hz), 3.66 (3H, s), 2.85-2.80 (2H, m), 2.34 (2H, t, J= 7.0 Hz), 2.22 (2H, d, J= 7.0 Hz), 1.95-1.85 (4H, m), 1.79-1.65 (5H, m), 1.52-1.46 (2H, m), 1.30-1.17 (2H, m), 0.96 (3H, t, J= 7.4 Hz).

### Step (iii)

### 2-Butoxy-7,8-dihydro-9-{3-[4-(2-methoxy-2-oxoethyl)piperidin-1-yl]propyl}-8-oxoadenine

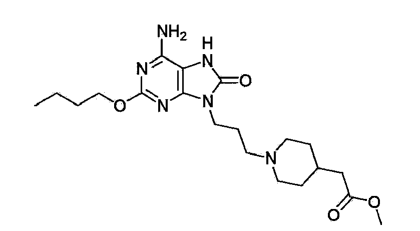

Using the compound 102mg (0.24mmol) obtained in step (ii), in the same manner as step (iii) of example 1, there was obtained the object compound 60mg as a white solid. Yield 61% ¹H NMR (DMSO-d₆) δ 6.37 (2H, brs), 4.13 (2H, t, J= 6.4 Hz), 3.67 (2H, t, J= 6.8 Hz), 3.58 (3H, s), 2.76-2.72 (2H, m), 2.28-2.17 (4H, m), 1.87-1.03 (13H, m), 0.89 (3H, t, J= 7.4 Hz).

### Example 12

### 2-Butoxy-7,8-dihydro-9-{3-[3-(2-methoxy-2-oxoethyl)piperidin-1-yl]propyl}-8-oxoadenine

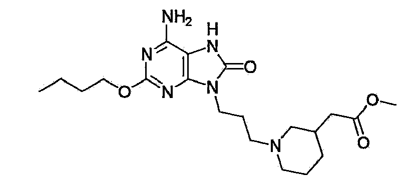

### Step (i)

### 2-Butoxy-8-methoxy-9-{3-[3-(2-ethoxy-2-oxoethyl)piperidin-1-yl]propyl}adenine

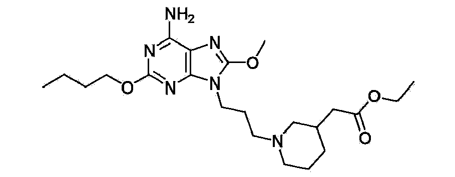

Using the compound 150mg (0.42mmol) obtained in step (i) of example 8, in the same manner as step (ii) of example 1, there was obtained the object compound 150mg as a white solid. Yield 80% ¹H NMR (CDCl₃) δ 5.11 (2H, brs), 4.27 (2H, t, J= 6.7 Hz), 4.13 (2H, q, J= 7.1 Hz), 4.10 (3H, s), 3.96 (2H, t, J= 6.8 Hz), 2.76-2.71 (2H, m), 2.32 (2H, t, J= 7.8 Hz), 2.19 (2H, d, J= 7.0 Hz), 2.12-1.71 (4H, m), 1.69-1.46 (9H, m), 1.24 (3H, t, J= 7.1 Hz), 0.96 (3H, t, J= 7.4 Hz).

### Step (ii)

### 2-Butoxy-7,8-dihydro-9-{3-[3-(2-methoxy-2-oxoethyl)piperidin-1-yl]propyl}-8-oxoadenine

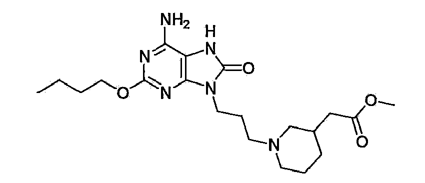

Using the compound 150mg (0.33mmol) obtained in step (i), in the same manner as step (iii) of example 1, there was obtained the object compound 84mg as a white solid. Yield 60% ¹H NMR (DMSO-d₆) δ 9.85 (1H, brs), 6.37 (2H, brs), 4.15 (2H, t, J= 6.6 Hz), 3.68 (2H, t, J= 6.8 Hz), 3.58 (3H, s), 2.66-2.62 (2H, m), 2.24-2.18 (4H, m), 1.90-1.35 (13H, m), 0.91 (3H, t, J= 7.4 Hz).

### Example 13

### 2-Butoxy-7,8-dihydro-9-{3-[4-(3-methoxy-3-oxopropyl)pyperazin-1-yl]propyl}-8-oxoadenine

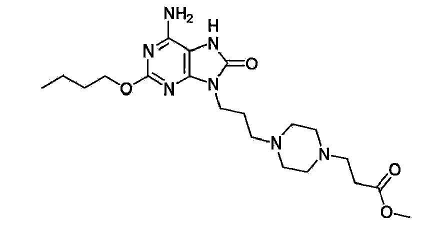

### Step (i)

### 2-Butoxy-8-methoxy-9-{3-[(4-(3-methoxy-3-oxopropyl)piperazin-1-yl)propyl]adenine}

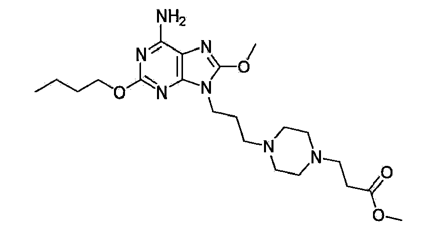

Using the compound 200mg (0.56 mmol) obtained in step (i) of example 8, in the same manner as step (ii) of example 1, there was obtained the object compound 110mg as a white solid. Yield 44% ¹H NMR (CDCl₃) δ 5.14 (2H, brs), 4.26 (2H, t, J= 6.7 Hz), 4.10 (3H, s), 3.97 (2H, t, J= 7.1 Hz), 3.68 (3H, s), 2.68 (2H, t, J= 7.2 Hz), 2.49 (2H, t, J= 7.2 Hz), 2.43 (8H, m), 2.37 (2H, d, J= 7.2 Hz), 1.96-1.91 (2H, m), 1.78-1.72 (2H, m), 1.52-1.46 (2H, m), 0.96 (3H, t, J= 7.4 Hz).

### Step (ii)

### 2-Butoxy-7,8-dihydro-9-{3-[4-(3-methoxy-3-oxopropyl)pyperazin-1-yl]propyl}-8-oxoadenine

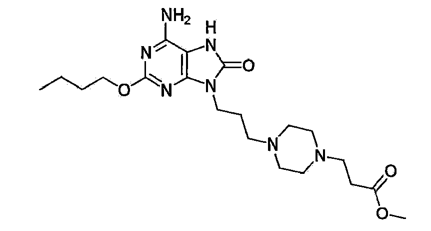

Using the compound 110mg (0.25mmol) obtained in step (i), in the same manner as step (iii) of example 1, there was obtained the object compound 83mg as a white solid. Yield 78% ¹H NMR (DMSO-d₆) δ 9.82 (1H, brs), 6.39 (2H, brs), 4.14 (2H, t, J= 6.6 Hz), 3.97 (2H, t, J= 6.9 Hz), 3.58 (3H, s), 2.45-2.20 (14H, m), 1.78-1.74 (2H, m), 1.66-1.62 (2H, m), 1.42-1.36 (2H, m), 0.92 (3H, t, J= 7.4 Hz).

### Example 14

### 2-Butoxy-7,8-dihydro-9-{2-[2-(4-methoxycarbonylpiperidin-1-yl)ethoxy]ethyl}-8-oxoadenine

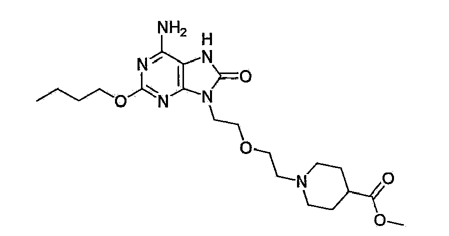

### Step (i)

### 2-Butoxy-9-[2-(2-chloroethoxy)ethyl]-8-methoxyadenine

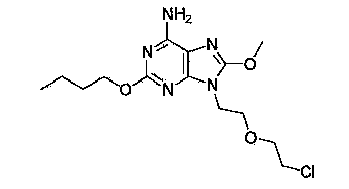

Using 2-butoxy-8-methoxyadenine 2.00g (8.43mmol), in the same manner as step (i) of example 1, there was obtained the object compound 1.18g as a white solid. Yield 41%
¹H NMR (CDCl₃) δ 5.45 (2H, brs), 4.34 (2H, t, J= 6.7 Hz), 4.23 (2H, t, J= 4.3 Hz), 4.19 (3H, s), 3.80 (2H, t, J= 4.3 Hz), 3.75 (2H, t, J= 4.8 Hz), 3.60 (2H, t, J= 4.8 Hz), 1.81-1.73 (2H, m), 1.52-1.46 (2H, m), 0.95 (3H, t, J= 7.4 Hz).

### Step (ii)

### 2-Butoxy-8-methoxy-9-{2-[2-(4-methoxycarbonylpiperidin-1-yl)ethoxy]ethyl}adenine

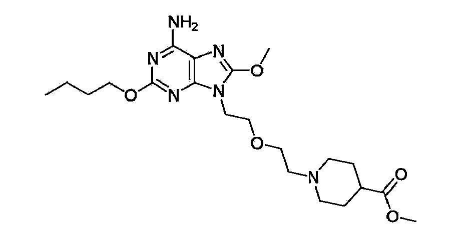

Using the compound 200mg (0.58mmol) obtained in step (i), in the same manner as step (ii) of example 1, there was obtained the object compound 82mg as a white solid. Yield 31%
¹H NMR (CDCl₃) δ 5.13 (2H, brs), 4.27 (2H, t, J= 6.6 Hz), 4.11 (2H, t, J= 5.8 Hz), 4.10 (3H, s), 3.76 (2H, t, J= 5.8 Hz), 3.69 (3H, s), 3.75 (2H, t, J= 5.7 Hz), 2.81-2.76 (2H, m), 2.46 (2H, t, J= 5.7 Hz), 2.29-2.20 (1H, m), 2.00-1.93 (2H, m), 1.81-1.70 (6H, m), 1.52-1.46 (2H, m), 0.96 (3H, t, J= 7.4 Hz).

### Step (iii)

### 2-Butoxy-7,8-dihydro-9-{2-[2-(4-methoxycarbonylpiperidin-1-yl)ethoxy]ethyl}-8-oxoadenine

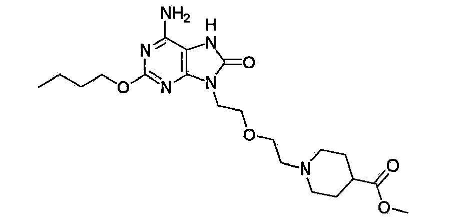

Using the compound 82mg (0.18mmol) obtained in step (ii), in the same manner as step (iii) of example 1, there was obtained the object compound 54mg as a white solid. Yield 68%
¹H NMR (DMSO-d₆) δ 9.84 (1H, brs), 6.41 (2H, brs), 4.14 (2H, t, J= 6.6 Hz), 3.82 (2H, t, J= 5.6 Hz), 3.65 (2H, t, J= 5.6 Hz), 3.59 (3H, s), 3.48 (2H, t, J= 5.7 Hz), 2.71-2.67 (2H, m), 2.36 (2H, t, J= 5.7 Hz), 2.28-2.18 (1H, m), 1.99-1.90 (2H, m), 1.70-1.62 (4H, m), 1.47-1.34 (4H, m), 0.92 (3H, t, J= 7.4 Hz).

### Example 15

### 2-Butoxy-7,8-dihydro-9-[6-(4-methoxycarbonylpiperidin-1-yl)hexyl]-8-oxoadenine

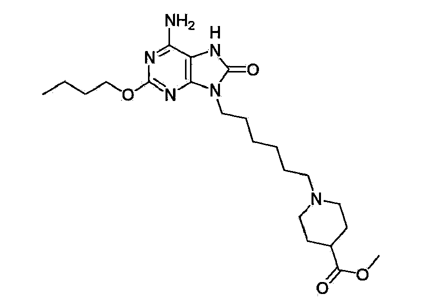

### Step (i)

### 2-Butoxy-9-(6-bromohexyl)-8-methoxyadenine

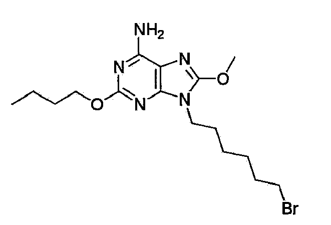

Using 2-butoxy-8-methoxyadenine 300mg (1.26mmol), in the same manner as step (i) of example 1, there was obtained the object compound 384mg as a white solid. Yield 76%
¹H NMR (CDCl₃) δ 5.14 (2H, brs), 4.27 (2H, t, J= 6.7 Hz), 4.11 (2H, s), 4.27 (2H, t, J= 7.2 Hz), 3.39 (2H, t, J= 6.8 Hz), 1.86-1.75 (6H, m), 1.52-1.46 (4H, m), 0.96 (3H, t, J= 7.4 Hz).

### Step (ii)

### 2-Butoxy-8-methoxy-9-[6-(4-methoxycarbonylpiperidin-1-yl)hexyl]adenine

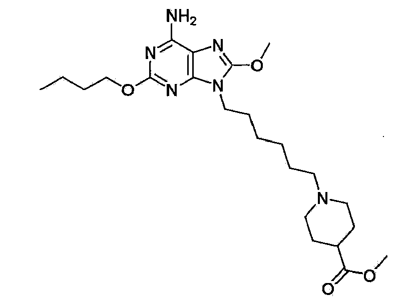

Using the compound 150mg (0.38mmol) obtained in step (i), in the same manner as step (ii) of example 1, there was obtained the object compound 144mg as a pale yellow oil. Yield 80%
¹H NMR (CDCl₃) δ 5.12 (2H, brs), 4.27 (2H, t, J= 6.7 Hz), 4.16 (3H, s), 3.91 (2H, t, J= 7.2 Hz), 3.67 (3H, s), 2.86 (2H, d, J= 11.3Hz), 2.30-2.24 (3H, m), 1.95-1.88 (4H, m), 1.78-1.73 (6H, m), 1.52-1.45 (4H, m), 1.32-1.30 (4H, m), 0.96 (3H, t, J= 7.4 Hz).

### Step (iii)

### 2-Butoxy-7,8-dihydro-9-[6-(4-methoxycarbonylpiperidin-1-yl)hexyl]-8-oxoadenine

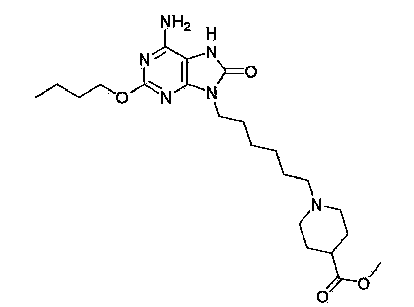

Using the compound 144mg (0.31mmol) obtained in step (ii), in the same manner as step (iii) of example 1, there was obtained the object compound 112mg as a white solid. Yield 80% ¹H NMR (DMSO-d₆) δ 9.84 (1H, brs), 6.41 (2H, brs), 4.14 (2H, t, J= 6.6 Hz), 3.65 (2H, t, J= 6.3 Hz), 3.60 (3H, s), 2.88-2.69 (2H, m), 2.36-2.15 (3H, m), 1.98-1.73 (4H, m), 1.68-1.56 (6H, m), 1.44-1.34 (6H, m), 1.27-1.23 (4H, m), 0.92 (3H, t, J= 7.4 Hz).

### Example 16

### 2-Butoxy-7,8-dihydro-9-[6-(4-methoxycarbonylpiperidin-1-yl)heptyl]-8-oxoadenine

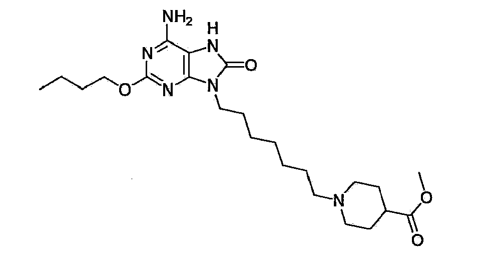

### Step (i)

### 2-Butoxy-9-(7-hydroxyheptyl)-8-methoxyadenine

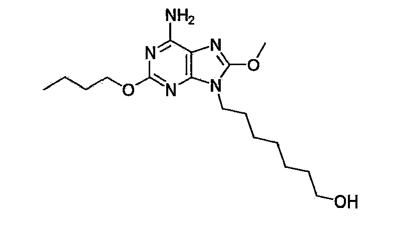

To 2-butoxy-8-methoxyadenine 300mg (1.26mmol) in DMF (10ml) were added potassium carbonate 350mg (2.53mmol) and 7-bromoheptanol 493mg (2.53mmol), and the mixture was stirred at room temperature for 5 hours. After removal of the solvent, to the residue was added water 30ml and the mixture was extracted with 5% methanol-chloroform (100 ml). The organic layer was washed with water and saturated brine, successively, dried over magnesium sulfate and concentrated in vacuo. The residue was purified by silica gel chromatography to give the object compound 198mg as a pale pink oil. Yield 45%
¹H NMR (CDCl₃) δ 5.14 (2H, brs), 4.28 (2H, t, J= 6.7 Hz), 4.11 (3H, s), 4.28 (2H, t, J= 6.7 Hz), 3.92 (2H, t, J= 7.2 Hz), 3.65-3.61 (2H, m), 3.38 (1H, brs), 1.78-1.67 (6H, m), 1.56-1.46 (4H, m), 1.33-1.25 (4H, m), 0.96 (3H, t, J= 7.4 Hz).

### Step (ii)

### 2-Butoxy-9-(7-methanesulfoxyheptyl)-8-methoxyadenine

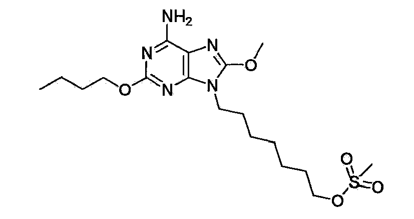

To 2-butoxy-9-(7-hydroxyheptyl)-8-methoxyadenine 198mg (0.56mmol) in THF (10ml) were added triethylamine 48µl (0.62mmol) and 4-dimethylaminopyridine 14mg (0.11mmol), and the mixture was stirred at room temperature for 10 minutes. After removal of the solvent, thereto was added water (20ml) and the mixture was extracted with 5% methanol-chloroform (100ml). The organic layer was washed with water and saturated brine, successively, dried over magnesium sulfate and concentrated in vacuo. The residue was purified by silica gel chromatography to give the object compound 168mg as a white solid.
Yield 70%
¹H NMR (CDCl₃) δ 5.11 (2H, brs), 4.27 (2H, t, J= 6.7 Hz), 4.21 (2H, t, J= 6.5 Hz), 4.11 (3H, s), 3.92 (2H, t, J= 7.1 Hz), 3.00 (3H, s), 1.79-1.71 (6H, m), 1.52-1.47 (2H, m), 1.38-1.30 (6H, m), 0.96 (3H, t, J= 7.4 Hz).

### Step (iii)

### 2-Butoxy-8-methoxy-9-[7-(4-methoxycarbonylpiperidin-1-yl)heptyl]adenine

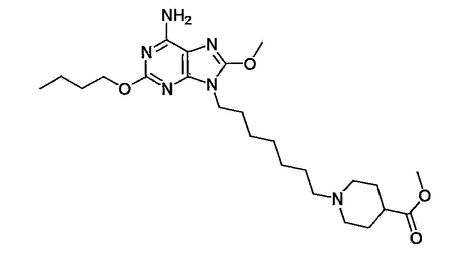

Using the compound 168mg (0.39mmol) obtained in step (ii), in the same manner as step (ii) of example 1, there was obtained the object compound 102mg as a pale yellow oil. Yield 55%
¹H NMR (CDCl₃) δ 5.13 (2H, brs), 4.27 (2H, t, J= 6.7 Hz), 4.11 (3H, s), 3.91 (2H, t, J= 7.2 Hz), 3.68 (3H, s), 2.86 (2H, d, J=11.1Hz), 2.31-2.25 (3H, m), 1.96-1.85 (4H, m), 1.78-1.73 (6H, m), 1.52-1.46 (4H, m), 1.31-1.27 (6H, m), 0.96 (3H, t, J= 7.4 Hz).

### Step (iv)

### 2-Butoxy-7,8-dihydro-9-[7-(4-methoxycarbonylpiperidin-1-yl)heptyl]-8-oxoadenine

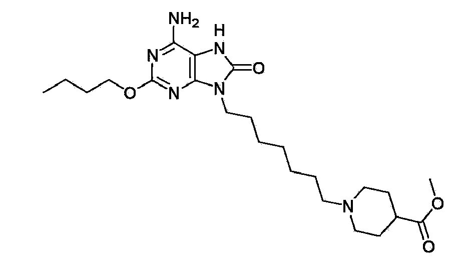

Using the compound 102mg (0.21mmol) obtained in step (iii), in the same manner as step (iii) of example 1, there was obtained the object compound 58mg as a white solid. Yield 59%
¹H NMR (DMSO-d₆) δ 9.84 (1H, brs), 6.40 (2H, brs), 4.14 (2H, t, J= 6.5 Hz), 3.65 (2H, t, J= 6.8 Hz), 3.60 (3H, s), 2.73 (2H, d, J= 10.9Hz), 2.20-2.11 (1H, m), 2.18 (2H, t, J= 7.3Hz), 1.87 (2H, t, J= 10.9Hz), 1.77 (2H, d, J= 12.1Hz), 1.66-1.62 (4H, m), 1.53-1.50 (2H, m), 1.42-1.36 (4H, m), 1.25-1.22 (6H, m), 0.92 (3H, t, J= 7.4 Hz).

### Example 17

### 2-Butoxy-7,8-dihydro-9-[8-(4-methoxycarbonylpiperidin-1-yl)octyl]-8-oxoadenine

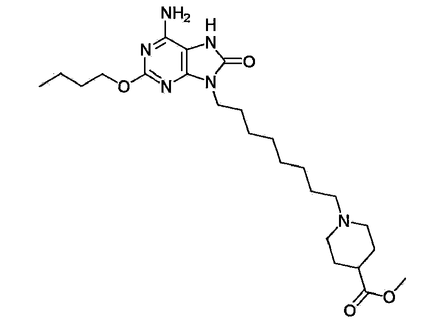

### Step (i)

### 9-(8-Bromooctyl)-2-butoxy-8-methoxyadenine

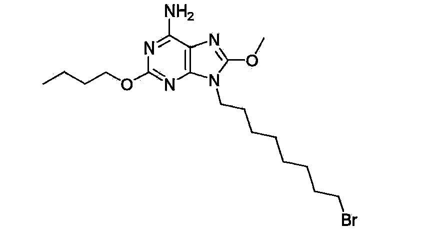

Using 2-butoxy-8-methoxyadenine300mg (1.26mmol), in the same manner as step (i) of example 1, there was obtained the object compound 361mg as a pale pink oil. Yield 70%
¹H NMR (CDCl₃) δ 5.12 (2H, brs), 4.27 (2H, t, J= 6.7 Hz), 4.11 (3H, s), 3.91 (2H, t, J= 7.2 Hz), 3.39 (2H, t, J= 6.8 Hz), 1.85-1.75 (6H, m), 1.52-1.29 (10H, m), 0.96 (3H, t, J= 7.4 Hz).

### Step (ii)

### 2-Butoxy-8-methoxy-9-[8-(4-methoxycarbonylpiperidin-1-yl)octyl]adenine

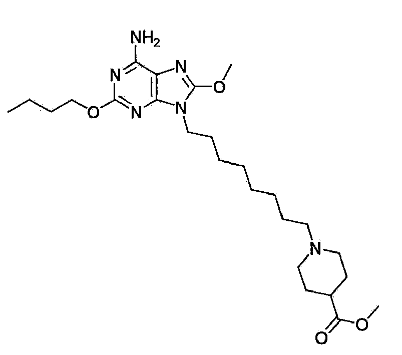

Using the compound 200 mg (0.46mmol) obtained in step (i), in the same manner as step (ii) of example 1, there was obtained the object compound 174mg as a pale pink oil. Yield 78%
¹H NMR (CDCl₃) δ 5.12 (2H, brs), 4.27 (2H, t, J= 6.7 Hz), 4.11 (3H, s), 3.91 (2H, t, J= 7.2 Hz), 3.68 (3H, s), 2.86 (2H, d, J=11.4Hz), 2.29-2.25 (3H, m), 1.96-1.88 (4H, m), 1.80-1.73 (6H, m), 1.52-1.45 (4H, m), 1.29-1.26 (8H, m), 0.96 (3H, t, J= 7.4 Hz).

### Step (iii)

### 2-Butoxy-7,8-dihydro-9-[8-(4-methoxycarbonylpiperidin-1-yl)octyl]-8-oxoadenine

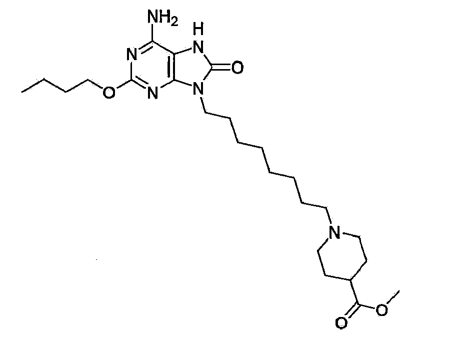

Using the compound 174mg (0.36mmol) obtained in step (ii), in the same manner as step (iii) of example 1, there was obtained the object compound 139mg as a white solid. Yield 85% ¹H NMR (DMSO-d₆) δ 9.82 (1H, brs), 6.45 (2H, brs), 4.14 (2H, t, J= 6.6 Hz), 3.65 (2H, t, J= 6.9 Hz), 3.60 (3H, s), 2.75 (2H, d, J= 10.9Hz), 2.31-2.17 (3H, m), 1.89-1.76 (4H, m), 1.66-1.59 (4H, m), 1.57-1.47 (2H, m), 1.42-1.34 (4H, m), 1.25-1.22 (8H, m), 0.92 (3H, t, J= 7.4 Hz).

### Example 18

### 2-Butoxy-7,8-dihydro-9-[4-(4-methoxycarbonylpiperidin-1-yl)butyl]-8-oxoadenine

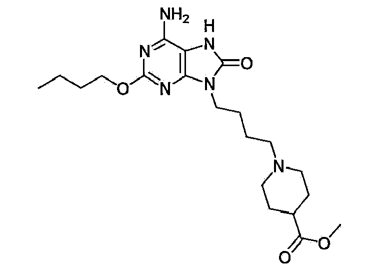

### Step (i)

### 9-(4-Bromobutyl)-2-butoxy-8-methoxyadenine

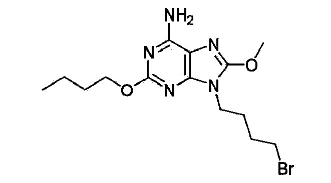

Using 2-butoxy-8-methoxyadenine 300mg (1.26mmol), in the same manner as step (i) of example 1, there was obtained the object compound 378mg as a white solid. Yield 81%
¹H NMR (CDCl₃) δ 5.18 (2H, brs), 4.27 (2H, t, J= 6.6 Hz), 4.12 (3H, s), 3.97 (2H, t, J= 6.7 Hz), 3.44 (2H, t, J= 6.5 Hz), 1.94-1.85 (4H, m), 1.78-1.75 (2H, m), 1.52-1.47 (2H, m), 0.97 (3H, t, J= 7.4 Hz).

### Step (ii)

### 2-Butoxy-8-methoxy-9-[4-(4-methoxycarbonylpiperidin-1-yl)butyl]adenine

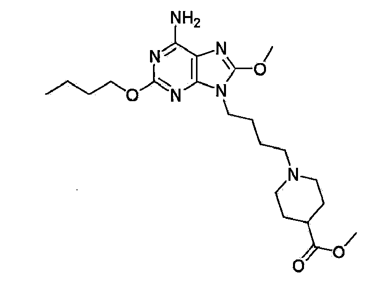

Using the compound 150mg (0.40mmol) obtained in step (i), in the same manner as step (ii) of example 1, there was obtained the object compound 141mg as a pale yellow oil. Yield 81%
¹H NMR (CDCl₃) δ 5.16 (2H, brs), 4.27 (2H, t, J= 6.7 Hz), 4.10 (3H, s), 3.94 (2H, t, J= 7.2 Hz), 3.67 (3H, s), 2.83 (2H, d, J= 11.3Hz), 2.34-2.26 (3H, m), 1.95-1.87 (4H, m), 1.78-1.71 (6H, m), 1.52-1.46 (4H, m), 0.96 (3H, t, J= 7.4 Hz).

### Step (iii)

### 2-Butoxy-7,8-dihydro-9-[4-(4-methoxycarbonylpiperidin-1-yl)butyl]-8-oxoadenine

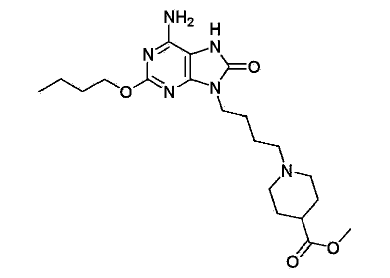

Using the compound 141mg (0.32mmol) obtained in step (ii), in the same manner as step (iii) of example 1, there was obtained the object compound 92mg as a white solid. Yield 68% ¹H NMR (DMSO-d₆) δ 9.84 (1H, brs), 6.41 (2H, brs), 4.14 (2H, t, J= 6.6 Hz), 3.66 (2H, t, J= 6.8 Hz), 3.59 (3H, s), 2.73 (2H, d, J= 11.3Hz), 2.26-2.22 (3H, m), 1.87-1.74 (4H, m), 1.66-1.62 (4H, m), 1.54-1.44 (2H, m), 1.41-1.36 (4H, m), 0.92 (3H, t, J= 7.4 Hz).

### Example 19

### 2-Butoxy-7,8-dihydro-9-[4-(3-methoxycarbonylpiperidin-1-yl)butyl]-8-oxoadenine

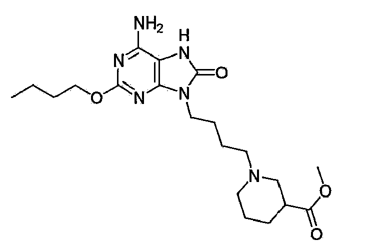

### Step (i)

### 2-Butoxy-8-methoxy-9-[4-(3-ethoxycarbonylpiperidin-1-yl)butyl]adenine

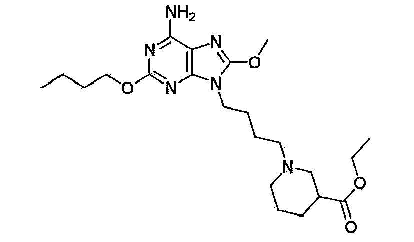

Using the compound 300mg (0.81mmol) obtained in step (i) of example 18 and 3-ethoxycarbonylpiperidin 633mg (4.0mmol), in the same manner as step (ii) of example 1, there was obtained the object compound 295mg as a pale yellow oil. Yield 82%
¹H NMR (CDCl₃) δ 5.14 (2H, brs), 4.27 (2H, t, J= 6.7 Hz), 4.15-4.10 (5H, m), 3.94 (2H, t, J= 7.2 Hz), 2.94-2.91 (1H, m), 2.73-2.70 (1H, m), 2.54-2.51 (1H, m), 2.35 (2H, t, J= 7.6Hz), 2.12-2.09 (1H, m), 1.94-1.91 (2H, m), 1.80-1.69 (5H, m), 1.52-1.46 (6H, m), 1.25 (3H, t, J= 7.1Hz), 0.96 (3H, t, J= 7.4 Hz).

### Step (ii)

### 2-Butoxy-7,8-dihydro-9-[4-(3-methoxycarbonylpiperidin-1-yl)butyl]-8-oxoadenine

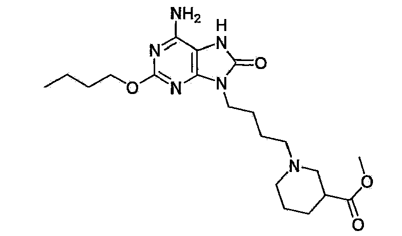

Using the compound 295mg (0.66mmol) obtained in step (ii), in the same manner as step (iii) of example 1, there was obtained the object compound 222mg as a white solid. Yield 80% ¹H NMR (DMSO-d₆) δ 9.84 (1H, brs), 6.41 (2H, brs), 4.14 (2H, t, J= 6.6 Hz), 3.66 (2H, t, J= 6.8 Hz), 3.58 (3H, s), 2.75-2.71 (1H, m), 2.56-2.47 (2H, m), 2.28-2.25 (2H, m), 2.11-2.07 (1H, m), 1.93-1.89 (1H, m), 1.76-1.72 (1H, m), 1.66-1.60 (5H, m), 1.41-1.34 (6H, m), 0.92 (3H, t, J= 7.4 Hz).

### Example 20

### 2-Butoxy-7,8-dihydro-9-[4-(2-methoxycarbonylpiperidin-1-yl)butyl]-8-oxoadenine

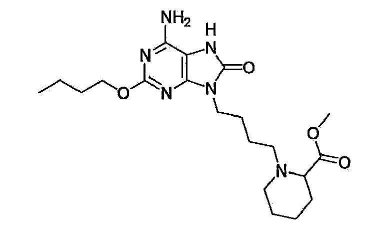

### Step (i)

### 2-Butoxy-8-methoxy-7,8-dihydro-9-[4-(2-ethoxycarbonylpiperidin-1-yl)butyl]adenine

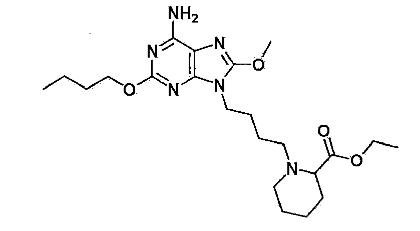

Using the compound 300mg (0.81mmol) obtained in step (i) of example 18 and 2-ethoxycarbonylpiperidin 633mg (4.0mmol), in the same manner as step (ii) of example 1, there was obtained the object compound 188mg as a pale yellow oil. Yield 52%
¹H NMR (CDCl₃) δ 5.12 (2H, brs), 4.27 (2H, t, J= 6.6 Hz), 4.21-4.16 (2H, m), 4.10 (3H, s), 3.93 (2H, t, J= 7.1 Hz), 3.35-3.23 (1H, m), 3.04-2.98 (1H, m), 2.57-2.48 (1H, m), 2.30-2.22 (1H, m), 2.12-2.06 (1H, m), 1.78-1.72 (4H, m), 1.57-1.47 (8H, m), 1.29-1.23 (5H, m), 0.96 (3H, t, J= 7.4 Hz).

### Step (ii)

### 2-Butoxy-7,8-dihydro-9-[4-(2-methoxycarbonylpiperidin-1-yl)butyl]-8-oxoadenine

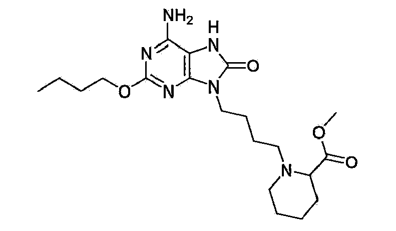

Using the compound 188mg (0.66mmol) obtained in step (ii), in the same manner as step (iii) of example 1, there was obtained the object compound 54mg as a white solid. Yield 31% ¹H NMR (DMSO-d₆) δ 9.87 (1H, brs), 6.41 (2H, brs), 4.14 (2H, t, J= 6.6 Hz), 3.65 (2H, t, J= 6.8 Hz), 3.59 (3H, s), 3.10-3.08 (1H, m), 2.88-2.82 (1H, m), 2.49-2.41 (1H, m), 2.27-2.19 (1H, m), 2.17-2.09 (1H, m), 1.68-1.61 (6H, m), 1.46-1.32 (8H, m), 0.92 (3H, t, J= 7.4 Hz).

### Example 21

### 2-Butoxy-7,8-dihydro-9-{4-[(4-methoxylcarbonylmethyl)piperazin-1-yl]butyl}-8-oxoadenine

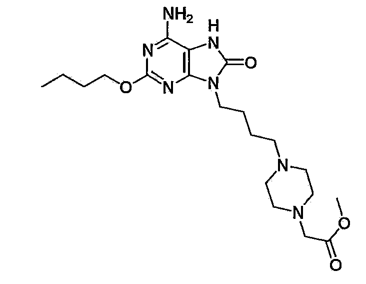

### Step (i)

### N-Methoxycarbonylmethylpiperazine hydrochloride

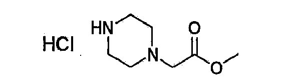

To t-butoxycarbonylpiperadine 3g (16mmol) in THF 50ml were added potassium carbonate 2.2g (16mmol) and methyl bromoacetate 4.9g (32mmol), and the mixture was stirred at room temperature for 15 hours. After concentration of the solvent, the residue was extracted with chloroform. The organic layer was dried over magnesium sulfate and then the solvent was concentrated to give a mixture of N-methoxycarbonylmethyl compound and methyl bromoacetate. To the mixture was added 4N-hydrochloric acid-dioxane 20ml and the mixture was stirred at room temperature for one hour. The resulting crystals were filtered with dioxane to give the object compound 2.4g as a white solid.
Yield 78%
¹H NMR (Methanol-d₄) δ 4.33 (2H, s), 3.88 (3H, s), 3.75-3.72 (4H, m), 3.66-3.63 (4H, m).

### Step (ii)

### 2-Butoxy-8-methoxy-9-{4-[(4-methoxycarbonylmethyl)piperazin-1-yl]butyl}adenine

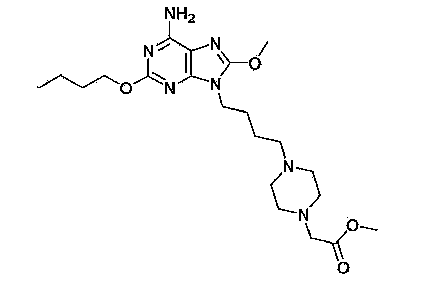

Using the compound 209mg (1.08 mmol) obtained in step (i) and 9-(4-bromobutyl)-2-butoxy-8-methoxyadenine 200mg (0.54mmol), in the same manner as step (ii) of example 1, there was obtained the object compound 164mg as a pale yellow oil. Yield 68%
¹H NMR (CDCl₃) δ 5.13 (2H, brs), 4.27 (2H, t, J= 6.7 Hz), 4.10 (3H, s), 3.94 (2H, t, J= 7.2 Hz), 3.72 (3H, s), 3.21 (2H, s), 2.58-2.49 (8H, m), 2.38-2.34 (2H, t, 7.6Hz), 1.81-1.71 (4H, m), 1.54-1.44 (4H, m), 0.96 (3H, t, J= 7.4 Hz).

### Step (iii)

### 2-Butoxy-7,8-dihydro-9-[4-(4-methoxycarbonylmethylpiperazin-1-yl)butyl]-8-oxoadenine

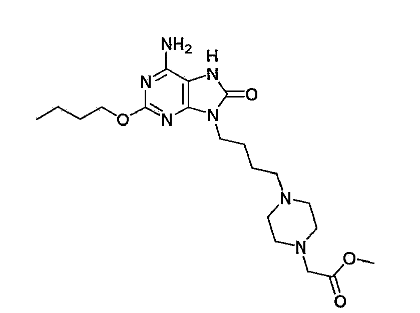

Using the compound 164mg (0.36mmol) obtained in step (ii), in the same manner as step (iii) of example 1, there was obtained the object compound 57mg as a white solid. Yield 36%
¹H NMR (DMSO-d₆) δ 9.84 (1H, brs), 6.41 (2H, brs), 4.15 (2H, t, J= 6.6 Hz), 3.67 (2H, t, J= 6.8 Hz), 3.60 (3H, s), 3.20 (2H, s), 2.47-2.31 (8H, m), 1.67-1.61 (4H, m), 1.54-1.34 (4H, m), 0.92 (3H, t, J= 7.4 Hz).

### Example 22

### 2-Butoxy-7,8-dihydro-9-{4-[4-(2-methoxycarbonylethyl)piperazin-1-yl]butyl}-8-oxoadenine

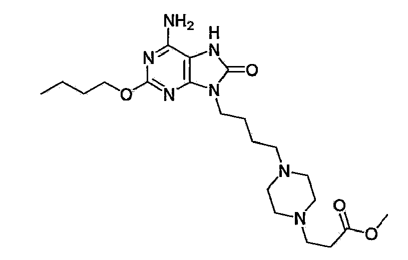

### Step (i)

### N-(2-Methoxycarbonylethyl)piperazine hydrochloride

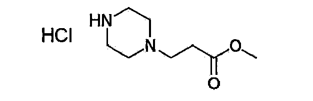

Using methyl 3-bromopropionate 3.2g (20mmol), in the same manner as step (i) of example 21, there was obtained the object compound 2.2g as a white solid. Yield 65%
¹H NMR (Methanol-d₄) δ 3.77 (3H, s), 3.73-3.62 (8H, m), 3.59 (2H, t, J= 7.0Hz), 2.99 (2H, t, J= 7.0Hz).

### Step (ii)

### 2-Butoxy-8-methoxy-9-{4-[4-(2-methoxycarbonylethyl)piperadine-1-yl]butyl}adenine

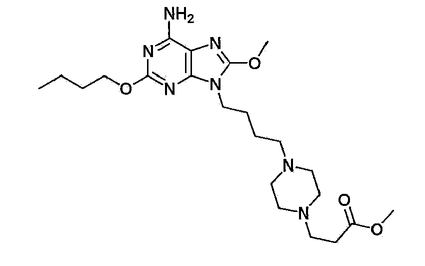

Using the compound 224mg (1.08 mmol) obtained in step (i) and 9-(4-bromobutyl)-2-butoxy-8-methoxyadenine 200mg (0.54mmol) in the same manner as step (ii) of example 1, there was obtained the object compound 161mg as a pale yellow oil. Yield 65%
¹H NMR (CDCl₃) δ 5.11 (2H, brs), 4.27 (2H, t, J= 6.6 Hz), 4.10 (3H, s), 3.93 (2H, t, J= 7.2 Hz), 3.67 (3H, s), 2.71-2.67 (2H, t, J= 7.4Hz), 2.52-2.45 (10H, m), 2.34 (2H, t, 7.6Hz), 1.79-1.74 (4H, m), 1.52-1.46 (4H, m), 0.96 (3H, t, J= 7.4 Hz).

### Step (iii)

### 2-Butoxy-7,8-dihydro-9-{4-[4-(2-methoxycarbonylethyl)piperazin-1-yl]butyl}-8-oxoadenine

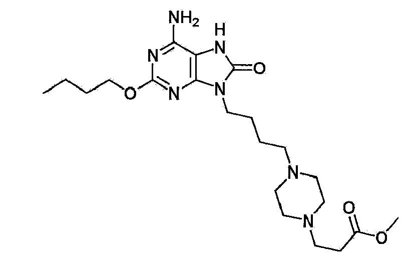

Using the compound 161mg (0.35mmol) obtained in step (ii), in the same manner as step (iii) of example 1, there was obtained the object compound 109mg as a white solid. Yield 70%
¹H NMR (DMSO-d₆) δ 9.84 (1H, brs), 6.40 (2H, brs), 4.14 (2H, t, J= 6.6 Hz), 3.66 (2H, t, J= 6.8 Hz), 3.58 (3H, s), 2.53-2.41 (4H, m), 2.30-2.21 (10H, m), 1.67-1.60 (4H, m), 1.41-1.34 (4H, m), 0.92 (3H, t, J= 7.4 Hz).

### Example 23

### 2-Butoxy-7,8-dihydro-9-[4-(4-methoxycarbonylmethylpiperidin-1-yl)butyl]-8-oxoadenine

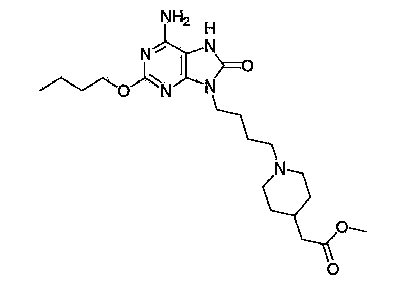

### Step (i)

### 2-Butoxy-8-methoxy-9-[4-(4-methoxycarbonylmethylpiperidin-1-yl)butyl]adenine

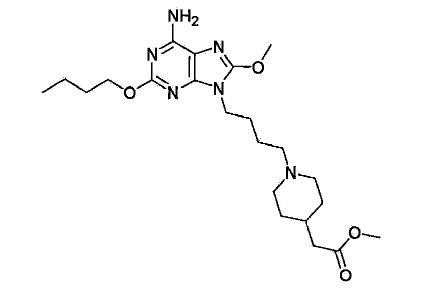

Using the compound 156mg (0.81mmol) obtained in step (i) of example 18 and 9-(4-bromobutyl)-2-butoxy-8-methoxyadenine 180mg (0.54mmol), in the same manner as step (ii) of example 1, there was obtained the object compound 171mg as a pale yellow oil. Yield 71%
¹H NMR (CDCl₃) δ 5.11 (2H, brs), 4.27 (2H, t, J= 6.7 Hz), 4.10 (3H, s), 3.93 (2H, t, J= 7.2 Hz), 3.66 (3H, s), 2.84 (2H, m), 2.31 (2H, m), 2.23 (2H, d, J= 7.0Hz), 1.89 (2H, m), 1.79-165 (4H, m), 1.52-1.46 (4H, m), 1.27 (2H, m), 0.96 (3H, t, J= 7.4 Hz).

### Step (ii)

### 2-Butoxy-7,8-dihydro-9-[4-(4-methoxycarbonylmethylpiperidin-1-yl)butyl]-8-oxoadenine

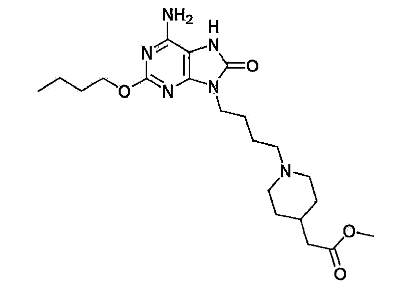

Using the compound 171mg (0.38mmol) obtained in step (i), in the same manner as step (iii) of example 1, there was obtained the object compound 88mg as a white solid. Yield 68%
¹H NMR (DMSO-d₆) δ 9.88 (1H, s), 6.45 (2H, brs), 4.16 (2H, t, J= 6.6 Hz), 3.70 (2H, t, J= 6.4 Hz), 3.60 (3H, s), 3.06 (2H, m), 2.89 (2H, m), 2.31 (2H, d, J= 6.3Hz), 1.91 (3H, m), 1.83-1.61 (6H, m), 1.42-1.35 (4H, m), 0.93 (3H, t, J= 7.4 Hz).

### Example 24

### 2-Butoxy-7,8-dihydro-9-[4-(3-methoxycarbonylethylpiperidin-1-yl)butyl]-8-oxoadenine

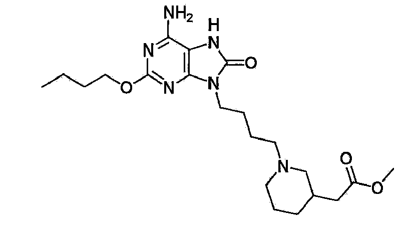

### Step (i)

### 2-Butoxy-8-methoxy-[4-(3-ethoxycarbonylmethylpiperidin-1-yl)butyl]adenine

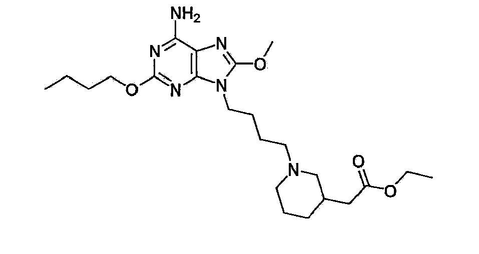

Using 3-ethoxycarbonylmethylpiperidin138mg (0.81 mmol) and 9-(4-bromobutyl)-2-butoxy-8-methoxyadenine 200mg (0.54mmol), in the same manner as step (ii) of example 1, there was obtained the object compound 201mg as a pale yellow oil. Yield 81%
¹H NMR (CDCl₃)δ 5.11 (2H, brs), 4.27 (2H, t, J= 6.7 Hz), 4.12 (5H, m), 3.93 (2H, t, J= 7.1 Hz), 2.76 (2H, m), 2.30 (2H, m), 2.19 (2H, m), 2.15 (1H, m), 1.82 (1H, m), 1.78-1.73 (6H, m), 1.69 (1H, m), 1.59 (1H, m), 1.52-1.48 (5H, m), 1.25 (3H, t, J= 7.2Hz), 0.96 (3H, t, J= 7.4 Hz).

### Step (ii)

### 2-Butoxy-7,8-dihydro-9-[4-(3-methoxycarbonylmethylpiperidin-1-yl)butyl]-8-oxoadenine

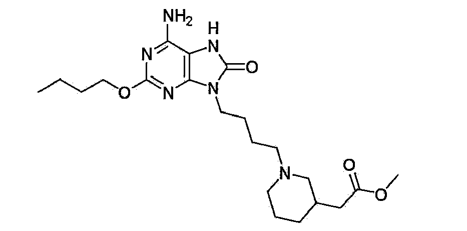

Using the compound 201mg (0.44mmol) obtained in step (i), in the same manner as step (iii) of example 1, there was obtained the object compound 173mg as a white solid. Yield 92%
¹H NMR (DMSO-d₆) δ 9.86 (1H, brs), 6.41 (2H, brs), 4.16 (2H, t, J= 6.6 Hz), 3.70 (2H, t, J= 6.9 Hz), 3.57 (3H, s), 3.06 (2H, m), 2.63 (2H, m), 2.24-2.18 (4H, m), 1.83 (2H, m), 1.68-1.62 (6H, m), 1.52 (2H, m), 1.42-1.34 (5H, m), 0.92 (3H, t, J= 7.4 Hz).

### Example 25

### 2-Butoxy-7,8-dihydro-9-(4-methoxycarbonylmethylmorpholin-2-ylmethyl)-8-oxoadenine

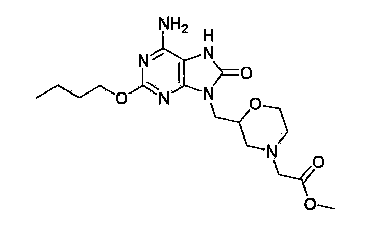

### Step (i)

### 4-tert-Butoxycarbonyl-2-methanesulfonyloxymethylmorpholine

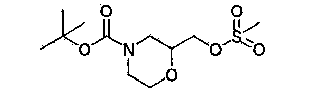

A solution of 4-tert-butoxy carbonyl-2-hydroxymethylmorpholine 1.0g (4.6 mmol) in THF 30ml was cooled to 0°C, and thereto were added methanesulfonyl chloride 0.71ml (9.2mmol), triethylamine 1.28ml (9.2mmol), and 4-dimethylaminopyridine 20mg, successively. The mixture was stirred for 30 minutes and then was warmed to room temperature, followed by stirring at room temperature for further 3 hours. After removal of the solvent, the residue was extracted with chloroform. The organic layer was washed with saturated sodium hydrogencarbonate solution, 5% aqueous citric acid, and saturated brine to give the object compound as a pale yellow liquid. Yield 1.32g (97%)
¹H NMR (CDCl₃) δ 4.24 (2H, d, J = 4.8 Hz), 3.92 (3H, m), 3.70 (1H, m), 3.55 (1H, m), 3.12 (3H, s), 2.95 (1H, m), 2.77 (1H, m), 1.45 (9H, s).

### Step (ii)

### 8-Bromo-2-butoxy-9-(4-tert-butoxycarbonylmorpholin-2-ylmethyl)adenine

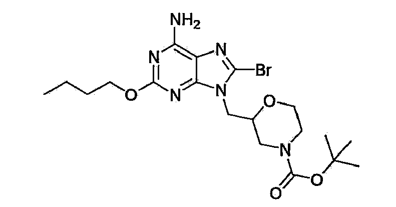

To a solution of 8-bromo-2-butoxyadenine 0.40g (1.40mmol) in DMF 30ml were added potassium carbonate 0.19g (1.40mmol) and 4-tert-butoxycarbonyl-2-methanesulfonyloxymethylmorpholine 1.31g (4.50mmol) obtained in step (i), and the mixture was stirred for 5 hours under heating at 120°C. After removal of the solvent, the residue was extracted with chloroform. The organic layer was concentrated and purified by silica gel chromatography to give the object compound 0.41g as a white solid. Yield 61%
¹H NMR (CDCl₃) δ 5.75 (2H, brs), 4.31 (2H, t, J = 6.6 Hz), 4.19 (2H, m), 3.87 (4H, m), 3.44 (1H, m), 2.95 (1H, m), 2.76 (1H, m), 1.77 (2H, m), 1.51 (2H, m), 1.45 (9H, s), 0.97 (3H, t, J = 7.3 Hz).

### Step (iii)

### 2-Butoxy-8-chloro-9-(4-methoxycarbonylmethylmorpholin-2-ylmethyl)adenine

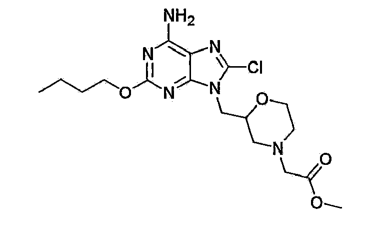

To 8-bromo-2-butoxy-9-(4-tert-butoxycarbonylmorpholin-2-ylmethyl)adenine 0.21g (0.43mmol) obtained in step (ii) was added 4 N-hydrochloric acid-dioxane 5ml and the mixture was stirred for 30 minutes. After removal of the solvent, to the residue were added DMF 15ml, methyl bromoacetate 0.051ml (0.52mmol) and potassium carbonate 0.14g (1.04mmol), successively and the mixture was stirred for 1.5 hours. After removal of the solvent, the residue was extracted with chloroform-ethanol (3:1). The organic layer was concentrated and purified by silica gel chromatography to give the object compound 0.17g as a white solid. Yield 99%
¹H NMR (CDCl₃) δ 5.42 (2H, brs), 4.30 (2H, t, J = 6.6 Hz), 4.22 (1H, m), 4.08 (2H, m), 3.86 (1H, m), 3.72 (3H, s), 3.65 (1H, m), 3.22 (2H, s), 2.81 (1H, d, J = 11.0 Hz), 2.70 (1H, m), 2.41 (1H, m), 2.26 (1H, dd, J = 11.0 Hz, 9.7 Hz), 1.77 (2H, m), 1.52 (2H, m), 0.97 (3H, t, J = 7.4 Hz).

### Step (iv)

### 2-Butoxy-7,8-dihydro-9-{[4-(2-methoxy-2-oxoethyl)morpholin-2-yl]methyl}-8-oxoadenine

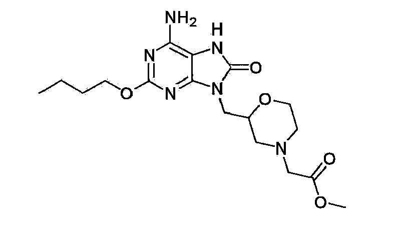

To a suspension of 2-butoxy-8-chloro-9-(4-methoxycarbonylmethylmorpholin-2-ylmethyl)adenine) 0.17g (0.42mmol) obtained in step (iii) in methanol 10ml was added 5M aqueous sodium hydroxide 10 ml, and the mixture was refluxed for 7 hours under stirring. After neutralization with concentrated hydrochloric acid, the solvent was removed to dryness. Thereto were added methanol 20ml and concentrated sulfuric acid 0.5ml and the mixture was refluxed for 4 hours under stirring. After being cooled to 0°C, the mixture was neutralized with saturated sodium hydrogencarbonate solution, and the resulting solid was filtered and washed with water to give the object compound 30mg as a white solid. Yield 18%
¹H NMR (DMSO-d₆) δ 10.03 (1H, brs), 6.47 (2H, brs), 4.14 (2H, t, J = 6.5 Hz), 3.80 (3H, m), 3.60 (3H, s), 3.58 (1H, m), 3.41 (1H, m), 3.26 (2H, s), 2.71 (1H, d, J = 11.0 Hz), 2.61 (1H, d, J = 11.6 Hz), 2.35 (1H, m), 2.14 (1H, dd, J = 10.8 Hz, 9.6 Hz), 1.64 (2H, tt, J = 7.5 Hz, 6.5 Hz), 1.38 (2H, tq, J = 7.5 Hz, 7.4 Hz), 0.92 (3H, t, J = 7.4 Hz).

### Example 26

### 2-Butaxy-7,8-dihydro-9-(1-methoxycarbonylmethylpiperidin-4-ylmethyl)-8-oxoadenine

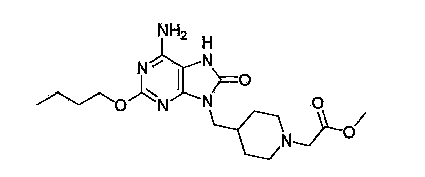

### Step (i)

### 1-tert-Butoxycarbonyl-4-methanesulfonyloxymethylpiperidine

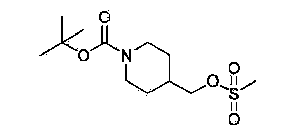

Using 1-tert-butoxycarbonyl-4-hydroxymethylpiperidine 2.00g (9.3mmol), in the same manner as step (i) of example 25, there was obtained the object compound 2.73g as a white solid. Yield 100%
¹H NMR (CDCl₃) δ 4.15 (2H, m), 4.07 (2H, d, J = 6.5 Hz), 3.02 (3H, s), 2.71 (2H, m), 1.92 (1H, m), 1.74 (2H, m), 1.46 (9H, s), 1.21 (2H, m).

### Step (ii)

### 8-Bromo-2-butoxy-9-(1-tert-butoxycarbonylpiperidin-4-ylmethyl)adenine

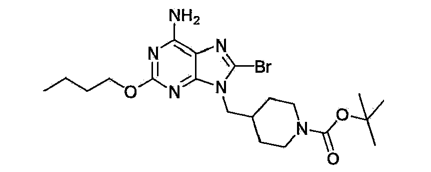

Using 8-bromo-2-butoxyadenine 0.30g (1.05mmol) and the compound 0.92g (3.15mmol) obtained in step (i), in the same manner as step (ii) of example 25, there was obtained the object compound 0.37g as a pale yellow solid. Yield 74%
¹H NMR (CDCl₃) δ 5.95 (2H, brs), 4.30 (2H, t, J = 6.6 Hz), 4.11 (2H, m), 4.00 (2H, d, J = 7.4 Hz), 2.66 (2H, m), 2.12 (1H, m), 1.78 (2H, m), 1.54 (4H, m), 1.46 (9H, s), 1.29 (2H, m), 0.97 (3H, t, J = 7.3 Hz).

### Step (iii)

### 2-Butoxy-7,8-dihydro-9-(1-methoxycarbonylmethylpiperidin-4-ylmethyl)-8-oxoadenine

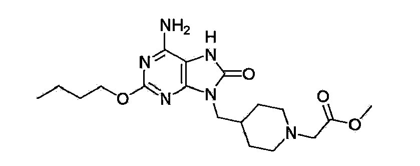

Using 8-bromo-2-butoxy-9-(1-tert-butoxycarbonylpiperidin-4-ylmethyl)adenine obtained in step (ii), in the same manner as step (iii) and then (iv) of example 25, there was obtained the object compound 91mg as a white solid. Yield 34% ¹H NMR (DMSO-d₆) δ 9.87 (1H, s), 6.41 (2H, brs), 4.14 (2H, t, J = 6.6 Hz), 3.59 (3H, s), 3.53 (2H, d, J = 7.1 Hz), 3.18 (2H, s), 2.77 (2H, m), 2.07 (2H, m), 1.75 (1H, m), 1.64 (2H, m), 1.48 (2H, m), 1.38 (2H, m), 1.21 (2H, m), 0.91 (3H, t, J = 7.3 Hz).

### Example 27

### 2-Butoxy-7,8-dihydro-9-[3-( 1-methoxycarbonylmethylpiperidin-4-yloxy)propyl]-8-oxoadenine

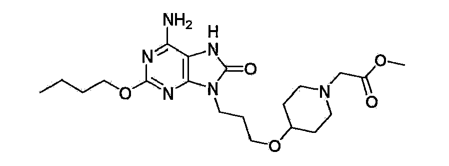

### Step (i)

### 1-tert-Butoxycarbonyl-4-(3-tert-butyldimethylsilyloxypropoxy)piperidine

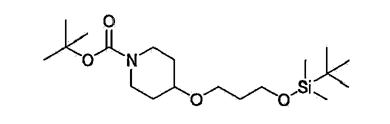

A solution of 1-tert-butoxycarbonyl-4-hydroxypiperidine 1.00g (5.0mmol) in DMF 10ml was cooled to 0°C and then thereto was added sodium hydride (55% dispersion in mineral oil) 0.52g (12.0mmol), followed by stirring at room temperature for 30 minutes. To the mixture was added 3-bromo-1-tert-butyldimethylsiloxypropane 3.5ml (15mmol). The mixture was stirred for 20 hours and then the reaction was quenched by adding water 1ml. After removal of the solvent, the residue was extracted with diethylether. The organic layer was concentrated and purified by silica gel chromatography to give the object compound 1.09g as a colorless transparent liquid. Yield 58%
¹H NMR (CDCl₃) δ 3.76 (2H, m), 3.70 (2H, t, J = 6.1 Hz), 3.53 (2H, t, J = 6.3 Hz), 3.42 (1H, m), 3.07 (2H, m), 1.79 (4H, m), 1.50 (2H, m), 1.45 (9H, s), 0.89 (9H, s), 0.05 (6H, s).

### Step (ii)

### 1-tert-Butoxycarbonyl-4-(3-hydroxypropoxy)piperidine

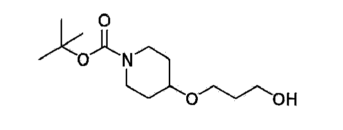

To a solution of 1-tert-butoxycarbonyl-4-(3-tert-butyldimethylsilyloxypropoxy)piperidine 1.09g (2.92mmol) obtained in step (i) in THF 15ml was added tetrabutylammonium fluoride in 1 M THF 5.8ml (5.8mmol), and the mixture was stirred for 2 hours. After removal of the solvent, the residue was extracted with chloroform. The organic layer was concentrated and purified by silica gel chromatography to give the object compound 0.74g as a colorless transparent liquid. Yield 98%
¹H NMR (CDCl₃) δ 3.76 (4H, m), 3.67 (2H, t, J = 5.6 Hz), 3.46 (1H, m), 3.11 (2H, m), 2.39 (1H, t, J = 5.4 Hz), 1.84 (4H, m), 1.53 (2H, m), 1.45 (9H, s).

### Step (iii)

### 1-tert-Butoxycarbonyl-4-(3-methanesulfonyloxypropoxy)piperidine

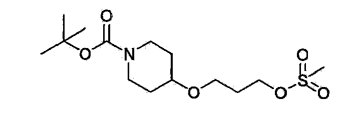

Using 1-tert-butoxycarbonyl-4-(3-hydroxypropoxy)piperidine 0.74g (2.85mmol) obtained in step (ii), in the same manner as step (i) of example 25, there was obtained the object compound 0.96g as a colorless transparent liquid. Yield 100%
¹H NMR (CDCl₃) δ 4.35 (2H, t, J = 6.2 Hz), 3.73 (2H, m), 3.57 (2H, t, J = 5.9 Hz), 3.44 (1H, m), 3.08 (2H, m), 3.01 (3H, s), 2.00 (2H, tt, J = 6.2 Hz, 5.9 Hz), 1.83 (2H, m), 1.50 (2H, m), 1.43 (9H, s).

### Step (iv)

### 8-Bromo-2-butoxy-9-[3-(1-tert-butoxycarbonylpiperidin-4-yloxy)propyl]adenine

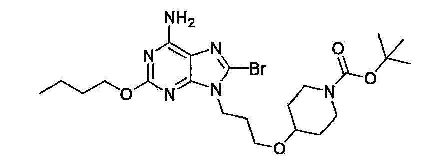

Using 8-bromo-2-butoxyadenine 0.40g (1.40mmol) and the compound 0.96g (2.85mmol) obtained in step (iii), in the same manner as step (ii) of example 25, there was obtained the object compound 0.51g as a pale yellow solid. Yield 69%
¹H NMR (CDCl₃) δ 5.65 (2H, brs), 4.30 (2H, t, J = 6.6 Hz), 4.25 (2H, t, J = 6.9 Hz), 3.73 (2H, m), 3.48 (2H, t, J = 5.8 Hz), 3.40 (1H, m), 3.08 (2H, m), 2.11 (2H, m), 1.79 (4H, m), 1.73 (4H, m), 1.45 (9H, s), 0.97 (3H, t, J = 7.3 Hz).

### Step (v)

### 2-Butoxy-8-chloro-9-[3-(1-methoxylcarbonylmethylpiperidin-4-yloxy) propyl] adenine

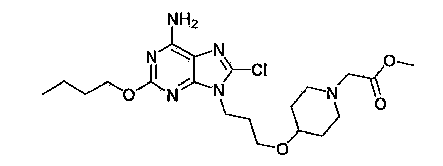

Using 8-bromo-2-butoxy-9-[3-(1-tert-butoxycarbonylpiperidin-4-yloxy)propyl]adenine 0.49g (0.94mmol) obtained in step (iv), in the same manner as step (iii) of example 25, there was obtained the object compound 0.37g as a white solid. Yield 95%
¹H NMR (CDCl₃) δ 5.52 (2H, brs), 4.30 (2H, t, J = 6.6 Hz), 4.24 (2H, t, J = 6.9 Hz), 3.72 (3H, s), 3.45 (2H, t, J = 5.9 Hz), 3.28 (1H, m), 3.21 (2H, s), 2.73 (2H, m), 2.33 (2H, m), 2.11 (2H, m), 1.86 (2H, m), 1.77 (2H, m), 1.65 (2H, m), 1.49 (2H, m), 0.97 (3H, t, J = 7.4 Hz).

### Step (vi)

### 2-Butoxy-7,8-dihydro-9-[3-(1-methoxycarbonylmethylpiperidin-4-yloxy)propyl]-8-oxoadenine

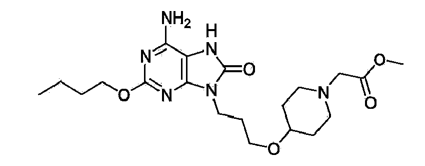

Using 2-butoxy-8-chloro-9-[3-(1-methoxycarbonylmethylpiperidin-4-yloxy)propyl]adenine 0.36g (0.80mmol) obtained in step (v), in the same manner as step (iv) of example 25, there was obtained the object compound 0.25g as a white solid. Yield 71% ¹H NMR (DMSO-d₆) δ 9.85 (1H, brs), 6.39 (2H, brs), 4.14 (2H, t, J = 6.6 Hz), 3.73 (2H, t, J = 6.8 Hz), 3.59 (3H, s), 3.39 (2H, t, J = 5.9 Hz), 3.20 (1H, m), 3.17 (2H, s), 2.65 (2H, m), 2.21 (2H, m), 1.85 (2H, m), 1.74 (2H, m), 1.65 (2H, m), 1.37 (4H, m), 0.91 (3H, t, J = 7.3 Hz).

### Example 28

### 2-Butoxy-7,8-dihydro-9-[3-(1-methoxycarbonylpiperidin-4-ylmethyloxy)propyl]-8-oxoadenine

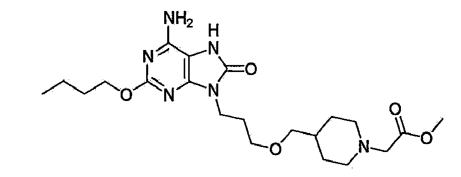

### Step (i)

### 1-tert-Butoxycarbonyl-4-(3-tert-butyldimethylsilyloxypropoxymethyl)piperidine

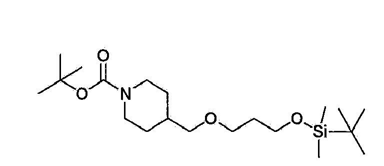

Using 1-tert-butoxycarbonyl-4-hydroxymethylpiperidine 1.08g (5.0mmol), in the same manner as step (i) of example 27, there was obtained the object compound 1.79g as a colorless transparent liquid.
Yield 82%
¹H NMR (CDCl₃) δ 4.10 (2H, m), 3.69 (2H, t, J = 6.2 Hz), 3.48 (2H, t, J = 6.3 Hz), 3.25 (2H, d, J = 6.1 Hz), 2.69 (2H, m), 1.75 (2H, m), 1.69 (3H, m), 1.46 (9H, s), 1.14 (2H, m), 0.90 (9H, s), 0.05 (6H, s).

### Step (ii)

### 1-tert-Butoxycarbonyl-4-(3-hydroxypropoxymethyl)piperidine

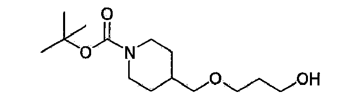

Using 1-tert-butoxycarbonyl-4-(3-tert-butyldimethylsilyloxypropoxymethyl)piperidine 1.79g (4.6mmol) obtained in step (i), in the same manner as step (ii) of example 27, there was obtained the object compound as a colorless transparent liquid. Yield 1.18g (94%) ¹H NMR (CDCl₃) δ 4.10 (2H, m), 3.77 (2H, dt, J = 5.5 Hz, 5.5 Hz), 3.62 (2H, t, J = 5.7 Hz), 3.29 (2H, d, J = 6.2 Hz), 2.69 (2H, m), 2.37 (1H, t, J = 5.5 Hz), 1.83 (2H, m), 1.73 (3H, m), 1.45 (9H, s), 1.15 (2H, m).

### Step (iii)

### 1-tert-Butoxycarbonyl-4-(3-methanesulfonyloxypropoxymethyl)piperidine

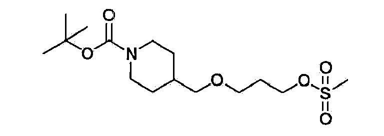

Using 1-tert-butoxycarbonyl-4-(3-hydroxypropoxymethyl)piperidine 1.18g (4.3mmol) obtained in step (ii), in the same manner as step (i) of example 25, there was obtained the object compound as a colorless transparent liquid. Yield 1.51g (100%)
¹H NMR (CDCl₃) δ 4.34 (2H, t, J = 6.3 Hz), 4.10 (2H, m), 3.51 (2H, t, J = 5.9 Hz), 3.26 (2H, d, J = 6.1 Hz), 2.96 (3H, s), 2.69 (2H, m), 2.00 (2H, m), 1.72 (3H, m), 1.46 (9H, s), 1.15 (2H, m).

### Step (iv)

### 8-Bromo-2-butoxy-9- [3-(1-tert-butoxycarbonylpiperidin-4-ylmethyloxy)propyl]adenine

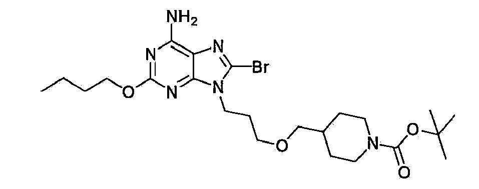

Using 8-bromo-2-butoxyadenine 0.50g (1.75mmol) and the compound 1.51 g (4.30mmol) obtained in step (iii), in the same manner as step (ii) of example 25, there was obtained the object compound 0.74g as a pale yellow solid. Yield 78%
¹H NMR (CDCl₃) δ 5.51 (2H, brs), 4.30 (2H, t, J = 6.6 Hz), 4.23 (2H, t, J = 6.9 Hz), 4.09 (2H, m), 3.44 (2H, t, J = 5.8 Hz), 3.21 (2H, d, J = 6.0 Hz), 2.68 (2H, m), 2.10 (2H, m), 1.77 (2H, m), 1.64 (3H, m), 1.51 (2H, m), 1.46 (9H, s), 1.12 (2H, m), 0.97 (3H, t, J = 7.4 Hz).

### Step (v)

### 2-Butoxy-7,8-dihydro-9-[3-(1-methoxycarbonylmethylpiperidin-4-ylmethyloxy)propyl]-8-oxoadenine

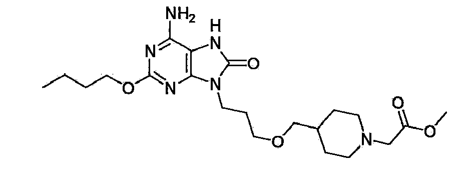

Using 8-bromo-2-butoxy-9-[3-(1-tert-butoxycarbonylpiperidin-4-ylmethyloxy)propyl]adenine obtained in step (iv), in the same manner as step (iii) and then step (iv) of example 25, there was obtained the object compound 0.32g as a white solid. Yield 53%
¹H NMR (DMSO-d₆) δ 9.90 (1H, brs), 6.40 (2H, brs), 4.14 (2H, t, J = 6.6 Hz), 3.72 (2H, t, J = 6.8 Hz), 3.59 (3H, s), 3.36 (2H, t, J = 5.9 Hz), 3.16 (2H, s), 3.13 (2H, d, J = 6.5 Hz), 2.77 (2H, m), 2.07 (2H, m), 1.86 (2H, m), 1.63 (2H, m), 1.55 (2H, m), 1.38 (3H, m), 1.10 (2H, m), 0.91 (3H, t, J = 7.3 Hz).

### Example 29

### 2-Butoxy-7,8-dihydro-9-(3-{1-[4-(dimethylamino)propoxycarbonylmethyl]piperidin-4-ylmethoxy}propyl)-8-oxoadenine

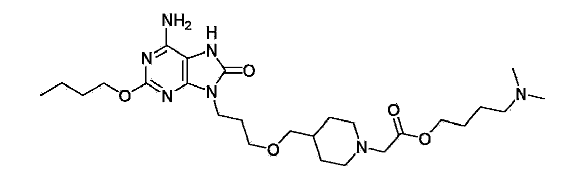

To a solution of the compound 70mg (0.16mmol) obtained by example 62 in DMF 5ml were added 1-ethyl-3-(dimethylaminopropyl)carbodimide·hydrochloride 153mg (0.80mmol), 1-hydroxybenztriazole 108mg (0.80mmol) and dimethylaminopropanol 0.16ml (0.80mmol), successively. The mixture was stirred at room temperature for 20 hours. After removal of the solvent, to the residue was added saturated sodium hydrogencarbonate solution. The resulting solid was filtered, washed with water and purified by HPLC to give the object compound 49.1mg (0.09mmol) as a yellow liquid-like substance. Yield 57%
¹H NMR (DMSO-d₆) δ 9.92 (1H, brs), 6.47 (2H, brs), 4.20-4.15 (2H, m), 4.14-4.09 (2H, m), 4.75-4.67 (2H, m), 4.58-4.53 (2H, m), 4.52-4.42 (2H, m), 4.39-4.32 (2H, m), 3.25-3.14 (2H, m), 3.09-3.01 (2H, m), 3.01-2.90 (2H, m), 2.84-2.67 (6H, m), 1.90-1.79 (4H, m), 1.78-1.69 (1H, m), 1.69-1.59 (6H, m), 1.55-1.42 (2H, m), 1.42-1.32 (2H, m), 0.89 (3H, t, J = 7.4 Hz).

### Example 30

### 2-Butoxy-7,8-dihydro-9-(1-methoxycarbonylmethylpiperidin-3-ylmethyl)-8-oxoadenine

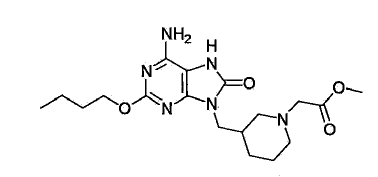

### Step (i)

### 2-Butoxy-9-(1-tert-butoxycarbonylpiperidin-3-ylinethyl)adenine

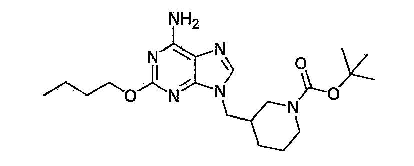

Using 1-tert-butoxycarbonyl-3-hydroxymethylpiperidine 0.88g (4.1mmol), in the same manner as step (i) of example 27, there was obtained 1-tert-butoxycarbonyl-3-methanesulfonyloxymethylpiperidine. Using 2-butoxyadenine 0.77g (3.72mmol) and 1-tert-butoxycarbonyl-3-methanesulfonyloxymethylpiperidin, in the same manner as step (ii) of example 27, there was obtained the object compound 1.19g as a pale yellow solid. Yield 79%
¹H NMR (DMSO-d₆) δ 7.91 (1H, s), 7.18 (2H, brs), 4.20 (2H, t, J = 6.6 Hz), 3.93 (2H, d, J = 7.3 Hz), 3.67 (2H, m), 2.82 (1H, m), 2.67 (1H, m), 2.00 (1H, m), 1.66 (4H, m), 1.40-1.20 (13H, m), 0.92 (3H, t, J = 7.3 Hz).

### Step (ii)

### 8-Bromo-2-butoxy-9-(1-tert-butoxycarbonylpiperidin-3-ylmethyl)adenine

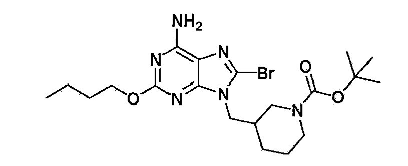

2-Butoxy-9-(1-tert-butoxycarbonylpiperidin-3-ylmethyl)adenine 0.28g (0.68mmol) obtained in step (i) and sodium acetate 0.10g (1.22mmol) were dissolved in chloroform 10 ml and thereto was dropped bromine 0.16g (1.03mmol), followed by stirring at room temperature for 5 hours. To the reaction mixture was added water and the mixture was extracted with chloroform. The organic layer was washed with saturated sodium hydrogen carbonate solution, saturated sodium hydrogensulfite solution and saturated brine, successively and dried over anhydrous magnesium sulfate and concentrated. The residue was purified by silica gel chromatography to give the object compound 0.30g as a pale orange liquid.
Yield 91%
¹H NMR (DMSO-d₆) δ 7.39 (2H, brs), 4.20 (2H, t, J = 6.6 Hz), 3.91 (2H, d, J = 7.4 Hz), 3.72 (1H, m), 3.60 (1H, m), 2.82 (1H, m), 2.67 (1H, m), 2.00 (1H, m), 1.66 (4H, m), 1.44-1.20 (13H, m), 0.92 (3H, t, J = 7.3 Hz).

### Step (iii)

### 2-Butoxy-7,8-dihydro-9-(1-methoxycarbonylmethylpiperidin-3-ylmethyl)-8-oxoadenine

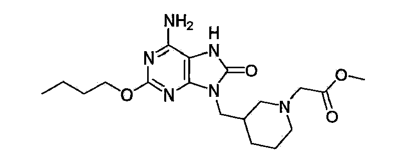

Using 8-bromo-2-butoxy-9-(1-tert-butoxycarbonylpiperidin-3-ylmethyl)adenine 0.30g obtained in step (ii), in the same manner as step (iii) and then step (iv) of example 27, there was obtained the object compound 69mg as a white solid. Yield 28%
¹H NMR (DMSO-d₆) δ 9.87 (1H, s), 6.42 (2H, brs), 4.14 (2H, t, J = 6.6 Hz), 3.57 (3H, s), 3.52 (2H, m), 3.18 (2H, s), 2.66 (2H, m), 2.17 (1H, m), 2.01 (2H, m), 1.64 (3H, m), 1.53 (1H, m), 1.38 (3H, m), 0.93 (1H, m), 0.92 (3H, t, J = 7.3 Hz).

### Example 31

### 2-Butoxy-7,8-dilxydro-9-[1-(3-methoxy-3-oxopropyl)piperidin-3-ylmethyl]-8-oxoadenine

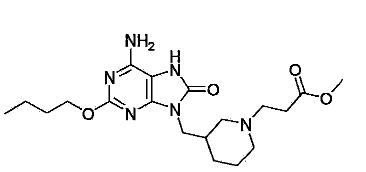

To 8-bromo-2-butoxy-9-(1-tert-butoxycarbonylpiperidin-3-ylmethyl)adenine 0.91mg (0.22mmol) obtained in step (i) of example 29 was added 4 N hydrochloric acid-dioxane 5ml, and the mixture was stirred for 30 minutes. After removal of the solvent, thereto were added DMF 5ml, THF 10ml, ethyl acrylate 0.036ml (0.34mmol) and triethylamine 0.034ml (0.25mmol), successively and the mixture was stirred for 8 hours. After removal of the solvent, the residue was treated with the same method as step (iv) of example 25 to give the object compound 10mg as a white solid.
Yield 11%
¹H NMR (DMSO-d₆) δ 9.89 (1H, s), 6.43 (2H, brs), 4.14 (2H, t, J = 6.6 Hz), 3.57 (3H, s), 3.57 (2H, m), 3.34 (2H, m), 2.56 (2H, m), 2.41 (2H, m), 1.96 (2H, m), 1.81 (2H, m), 1.62 (3H, m), 1.51 (1H, m), 1.37 (3H, m), 0.95 (1H, m), 0.91 (3H, t, J = 7.3 Hz).

### Example 32

### 2-Butoxy-7,8-dihydro-9-[2-(1-methoxycarbonylmethylpiperidin-4-yl)ethyl]-8-oxoadenine

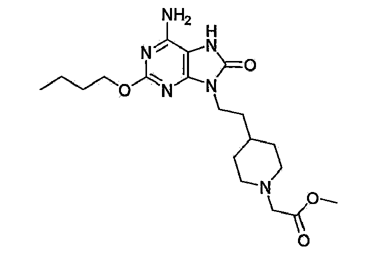

Using 1-tert-butoxycarbonyl-4-hydroxyethylpiperidine, in the same manner as step (i), step (ii), step (iii) and then step (iv) of example 25, there was obtained the object compound 56mg as a white solid.
¹H NMR (DMSO-d₆) δ 9.87 (1H, s), 6.41 (2H, brs), 4.13 (2H, t, J = 6.6 Hz), 3.69 (2H, t, J = 6.9 Hz), 3.59 (3H, s), 3.16 (2H, s), 2.76 (2H, m), 2.05 (2H, m), 1.69 (2H, m), 1.63 (2H, m), 1.55 (2H, m), 1.37 (2H, m), 1.13 (3H, m), 0.91 (3H, t, J = 7.4 Hz).

### Example 33

### 2-Butoxy-7,8-dihydro-9-{2-[1-(3-methoxy-3-oxopropyl)piperidin-4-yl]ethyl}-8-oxoadenine

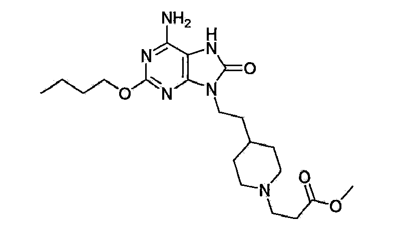

Using 1-tert-butoxycarbonyl-4-hydroxyethylpiperidine, in the same manner as step (i), step (ii), step (iii) and then step (iv) of example 25, there was obtained the object compound 100mg as a white solid.
¹H NMR (DMSO-d₆) δ 9.87 (1H, s), 6.41 (2H, brs), 4.13 (2H, t, J = 6.6 Hz), 3.68 (2H, t, J = 6.9 Hz), 3.57 (3H, s), 3.32 (2H, m), 2.76 (2H, m), 2.47 (2H, m), 1.81 (2H, m), 1.68 (2H, m), 1.62 (2H, m), 1.54 (2H, m), 1.40 (2H, m), 1.10 (3H, m), 0.91 (3H, t, J = 7.3 Hz).

### Example 34

### 2-Butoxy-7,8-dihydro-9-[2-(1-methoxycarbonylmethylpiperidin-2-yl)ethyl]-8-oxoadenine

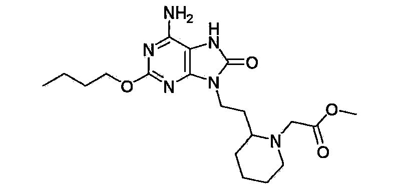

Using 1-tert-butoxycarbonyl-2-hydroxyethylpiperidine, in the same manner as step (i), step (ii), step (iii) and then step (iv) of example 25, there was obtained the object compound 5mg as a white solid.
¹H NMR (DMSO-d₆) δ 9.87 (1H, brs), 6.41 (2H, brs), 4.13 (2H, t, J = 6.6 Hz), 3.70 (2H, m), 3.53 (3H, s), 3.45 (2H, m), 2.73 (2H, m), 1.83 (2H, m), 1.75-1.59 (5H, m), 1.55-1.20 (6H, m), 0.91 (3H, t, J = 7.4 Hz).

### Example 35

### 2-Butoxy-7,8-dihydro-9-{[1-(3-methoxy-3-oxopropyl)piperidin-4-yl]methyl}-8-oxoadenine

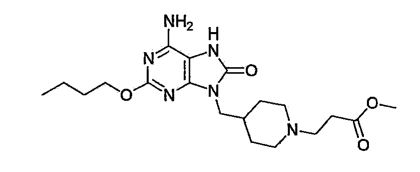

Using 8-bromo-2-butoxy-9-(1-tert-butoxycarbonylpiperidin-4-ylinethyl)adenine 0.18g (0.38mmol) obtained in step (ii) of example 26, in the same manner as step (iii) and then step (iv) of example 25, there was obtained the object compound 0.11g as a white solid. Yield 71%
¹H NMR (DMSO-d₆) δ 9.97 (1H, brs), 6.44 (2H, brs), 4.13 (2H, t, J = 6.6 Hz), 3.57 (3H, s), 3.52 (2H, d, J = 7.2 Hz), 2.77 (2H, m), 2.45 (2H, m), 1.83 (2H, m), 1.75 (1H, m), 1.65 (2H, m), 1.48 (2H, m), 1.41 (2H, m), 1.19 (2H, m), 0.91 (3H, t, J = 7.3 Hz).

### Example 36

### 2-Butoxy-7,8-dihydro-9-{2-[4-(2-methoxy-2-oxoethyl)morpholin-2-yl]ethyl}-8-oxoadenine

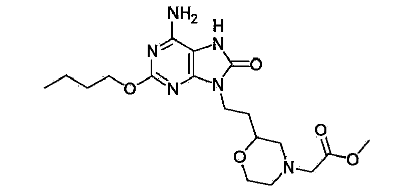

### Step (i)

### 8-Bromo-2-butoxy-9-(2-morpholin-2-ylethyl)adenine

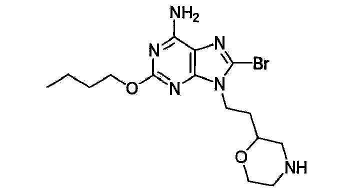

Using 4-tert-butoxycarbonyl-2-hydroxyethylmorpholine, after treating it in the same method as step (i) and then step (ii) of example 25, thereto were added methanol 10ml and concentrated sulfuric acid 0.1ml and the mixture was stirred at room temperature for 5 hours. After neutralization with saturated sodium hydrogencarbonate solution, the mixture was extracted with chloroform. The organic layer was dried over magnesium sulfate and the solvent was removed in vacuo. The residue was purified by silica gel chromatography to give the object compound 0.11g as a colorless transparent liquid. Yield 45%
¹H NMR (DMSO-d₆) δ 7.34 (2H, brs), 4.20 (2H, t, J = 6.6 Hz), 4.11 (2H, t, J= 7.1 Hz), 3.68 (2H, m), 3.36 (2H, m), 2.70 (1H, m), 2.65 (2H, m), 2.33 (1H, m), 1.78 (2H, m), 1.67 (2H, m), 1.43 (2H, m), 0.92 (3H, t, J = 7.3 Hz).

### Step (ii)

### 8-Bromo-2-butoxy-9-{2-[4-(2-methoxy-2-oxoethyl)morpholin-2-yl]ethyl}oxoadenine

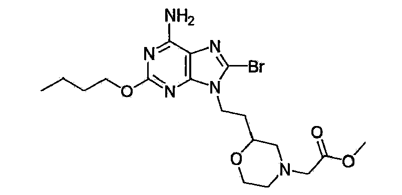

To 8-bromo-2-butoxy-9-(2-morpholin-2-ylethyl)adenine 62mg (0.16mmol) obtained in step (i) were added DMF 5ml, methyl bromoacetate 0.024ml (0.25mmol), and potassium carbonate 18mg (0.13mmol), successively and the mixture was stirred for 2 hours. After removal of the solvent, the residue was extracted with chloroform. The organic layer was concentrated and purified by silica gel chromatography to give the object compound 62mg as a colorless transparent liquid. Yield 82%
¹H NMR (DMSO-d₆) δ 7.37 (2H, brs), 4.20 (2H, t, J = 6.6 Hz), 4.10 (2H, t, J= 7.1 Hz), 3.74 (2H, m), 3.59 (3H, s), 3.41 (1H, m), 3.21 (2H, s), 2.67 (2H, m), 2.31 (1H, m), 2.04 (1H, m), 1.82 (2H, m), 1.67 (2H, m), 1.41 (2H, m), 0.92 (3H, t, J = 7.4 Hz).

### Step (iii)

### 2-Butoxy-7,8-dihydro-9-{2-[4-(2-methoxy-2-oxoethyl)morpholin-2-yl]ethyl}-8-oxoadenine

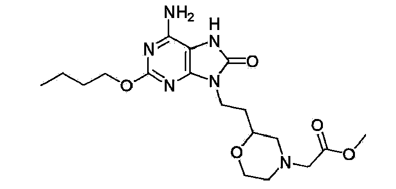

Using 8-Bromo-2-butoxy-9-{2-[4-(2-methoxy-2-oxoethyl)morpholin-2-yl]ethyl}oxoadenine 62mg (0.16mmol) obtained in step (ii), in the same manner as step (iv) of example 25, there was obtained the object compound 0,51mg as a white solid. Yield 96%
¹H NMR (DMSO-d₆) δ 9.85 (1H, brs), 6.40 (2H, brs), 4.14 (2H, t, J = 6.6 Hz), 3.74 (3H, m), 3.60 (3H, s), 3.41 (2H, m), 3.21 (2H, s), 2.62 (2H, m), 2.27 (1H, m), 2.02 (1H, m), 1.71 (2H, m), 1.64 (2H, m), 1.38 (2H, m), 0.92 (3H, t, J = 7.4 Hz).

### Example 37

### 2-Butoxy-7,8-dihydro-9-[5-(4-hydroxylcarbonylpiperidin-1-yl)pentyl]-8-oxoadenine

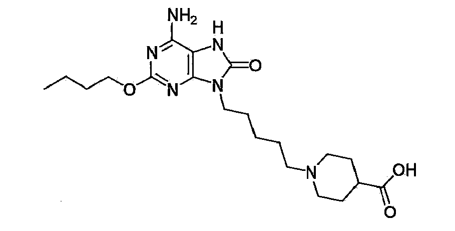

To the compound 55mg (0.13mmol) obtained by example 1 was added 1N aqueous sodium hydroxide (4ml) and the mixture was refluxed for 1.5 hours. After neutralization with concentrated hydrochloric acid, the solvent was removed. To the residue was added water, and the resulting solid was filtered to give the object compound 26mg as a white solid. Yield 49% ¹H NMR (DMSO-d₆) δ 10.04 (1H, bs), 6.51 (1H, bs), 4.14 (2H, t, J= 6.6 Hz), 3.66 (2H, t, J= 6.8 Hz), 3.10-2.96 (2H, m), 2.68-2.54 (2H, m), 2.51-2.48 (2H, m), 2.40-2.28 (2H, m), 1.91-1.81 (2H, m), 1.70-1.59 (6H, m), 1.59-1.50 (2H, m), 1.55-1.35 (2H, m), 1.27-1.18 (2H, m), 0.91 (3H, t, J= 7.4 Hz).

### Example 38

### 2-Butoxy-7,8-dihydro-9-[5-(4-hydroxylcarbonylmethylpiperidin-1-yl)pentyl]-8-oxoadenine

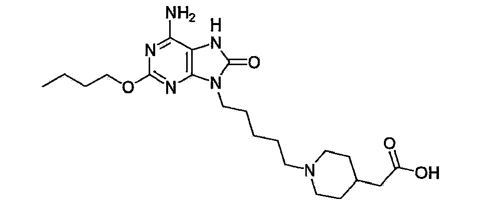

Using the compound 100mg (0.22mmol) obtained by example 2, in the same manner as in example 37, there was obtained the object compound 21mg as a pale pink oil. Yield 22% ¹H NMR (DMSO-d₆) δ 12.10 (1H, bs), 10.23 (1H, bs), 6.54 (2H, bs), 4.13 (2H, t, J= 6.6 Hz), 3.64 (2H, t, J= 6.9 Hz), 2.78-2.72 (2H, m), 2.16 (2H, t, J= 7.3 Hz), 2.05 (2H, d, J= 6.4 Hz), 1.81-1.73 (2H, m), 1.68-1.54 (7H, m), 1.45-1.33 (4H, m), 1.25-1.13 (4H, m), 0.91 (3H, t, J= 7.4 Hz).

### Example 39

### 2-Butoxy-7,8-dihydro-9-[5-(3-hydroxylcarbonylmethylpiperidin-1-yl)pentyl]-8-oxoadenine

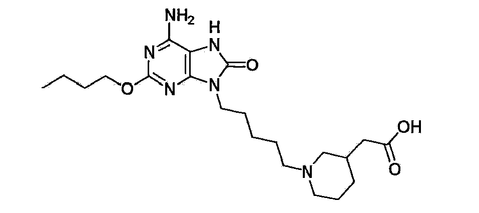

Using the compound 50mg (0.12mmol) obtained by example 3, in the same manner as in example 37, there was obtained the object compound 26mg as a pale pink oil. Yield 54% ¹H NMR (DMSO-d₆) δ 10.08 (1H, bs), 6.52 (2H, bs), 4.13 (2H, t, J= 6.6 Hz), 3.65 (2H, t, J= 6.8 Hz), 3.00-2.90 (2H, m), 2.23-2.07 (4H, m), 2.07-1.91 (2H, m), 1.72-1.59 (7H, m), 1.59-1.45 (3H, m), 1.45-1.33 (2H, m), 1.28-1.18 (2H, m), 1.05-0.94 (1H, m), 0.91 (3H, t, J= 7.4 Hz).

### Example 40

### 2-Butoxy-7,8-dihydro-9-[2-(4-hydroxylcarbonylpiperidin-1-yl)ethyl]-8-oxoadenine

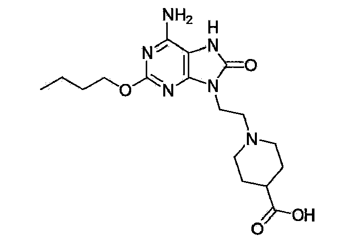

To the compound 22mg (0.056mmol) obtained by example 4 in methanol (10ml) was added aqueous lithium hydroxide 50mg (10ml), and the mixture was stirred at room temperature for 12 hours. After neutralization with 1N hydrochloric acid, the solvent was removed. To the residue was added a small amount of water and the resulting solid was filtered to give the object compound 18mg as a white solid. Yield 85% ¹H NMR (DMSO-d₆) δ 6.81 (2H, brs), 4.13 (2H, t, J= 6.6 Hz), 3.75 (2H, t, J= 6.6 Hz), 2.86-2.82 (2H, m), 2.54 (2H, t, J= 6.6 Hz), 1.97-1.91 (3H, m), 1.70-1.61 (4H, m), 1.44-1.35 (4H, m), 0.91 (3H, t, J= 7.4 Hz).

### Example 41

### 2-Butoxy-7,8-dihydro-9-[2-(3-hydroxycarbonylpiperidin-1-yl)ethyl]-8-oxoadenine

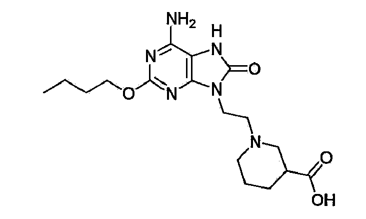

Using the compound 78mg (0.20mmol) obtained by example 5 and reacting it in the same manner as in example 37 and then purifying by RPHPLC, there was obtained the object compound 32mg as a white solid.
Yield 33%
¹H NMR (DMSO-d₆) δ 9.30(1H, brs), 6.60 (2H, brs), 4.15 (2H, t, J= 6.7 Hz), 4.09 (2H, m), 3.84 (1H, m), 3.70 (1H, m), 3.45 (2H, m), 3.01 (1H, m), 2.91 (1H, m), 2.67 (1H, m), 2.10-1.36 (8H, m), 0.92 (3H, t, J= 7.4 Hz).

### Example 42

### 2-Butoxy-7,8-dihydro-9-{2-(2-hydroxylcarbonylpiperidin-1-yl)ethyl}-8-oxoadenine

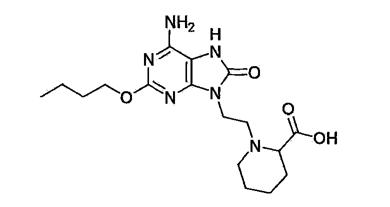

Using the compound 30mg (0.08mmol) obtained by example 6, in the same manner as in example 37, there was obtained the object compound 10mg as a white solid. Yield 33% ¹H NMR (DMSO-d₆) δ 14.03 (1H, bs), 10.16 (1H, bs), 6.60 (1H, bs), 4.15 (2H, t, J= 7.4 Hz), 4.12-4.03 (3H, m), 3.81-3.70 (1H, m), 3.68-3.60 (1H, m), 3.49-3.36 (1H, m), 3.18-3.05 (1H, m), 2.18-2.05 (1H, m), 1.88-1.45 (7H, m), 1.44-1.34 (2H, m), 0.91 (3H, t, J= 7.4 Hz).

### Example 43

### 2-Butoxy-7,8-dihydro-9-[2-{4-(2-carboxyethyl)piperazin-1-yl}ethyl]-8-oxoadenine

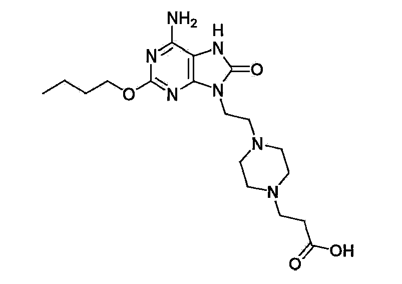

Using the compound 30mg (0.01mmol) obtained by example 7, in the same manner as in example 37, there was obtained the object compound 29mg as a white solid. Yield 99%
¹H NMR (DMSO-d₆) δ 10.21 (1H, s), 6.59 (1H, s), 4.13 (1H, t, J= 6.6 Hz), 3.78 (1H, t, J= 6.4 Hz), 2.75-2.61 (6H, m), 2.61-2.51 (2H, m), 2.51-2.49 (6H, m), 1.67-1.59 (2H, m), 1.43-1.33 (2H, m), 0.91 (3H, t, J= 7.4 Hz).

### Example 44

### 2-Butoxy-7,8-dihydro-9-[3-(4-hydroxycarbonylpiperidin-1-yl)propyl]-8-oxoadenine

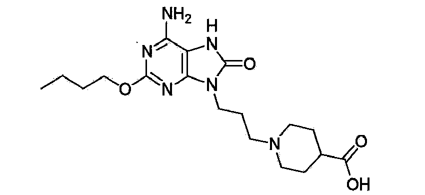

Using the compound 60mg (0.15mmol) obtained by example 8, in the same manner as example 37, there was obtained the object compound 40mg as a white solid. Yield 69%
¹H NMR (DMSO-d₆) δ 6.75 (2H, brs), 4.14 (2H, t, J= 6.6 Hz), 3.67 (2H, t, J= 6.9 Hz), 2.76-2.71 (2H, m), 2.24 (2H, t, J= 7.0 Hz), 1.98-1.34 (13H, m), 0.91 (3H, t, J= 7.4 Hz).

### Example 45

### 2-Butoxy-7,8-dihydro-9-[3-(3-hydroxycarbonylpiperidin-1-yl)propyl]-8-oxoadenine

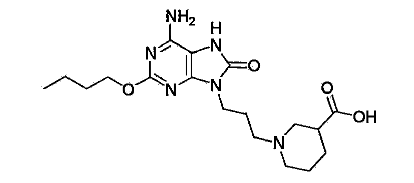

Using the compound 60mg (0.15mmol) obtained by example 9, in the same manner as example 37, there was obtained the object compound 30mg as a white solid. Yield 52%
¹H NMR (DMSO-d₆) δ 6.87 (2H, brs), 4.13 (2H, t, J= 6.6 Hz), 3.68 (2H, t, J= 6.9 Hz), 2.89 (1H, m), 2.66 (1H, m), 2.24 (2H, t, J= 7.0 Hz), 2.07 (1H, m), 1.81-1.14 (12H, m), 0.91 (3H, t, J= 7.4 Hz).

### Example 46

### 2-Butoxy-7,8-dihydro-9-[3-(2-hydroxycarbonylpiperidin-1-yl)propyl]-8-oxoadenine

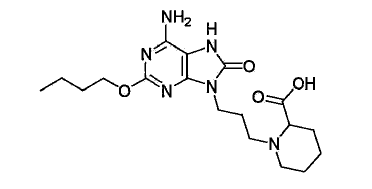

### Step (i)

### 2-Butoxy-9-[3-(2-ethoxylcarbonylpiperidin-1-yl)propyl]-8-methoxyadenine

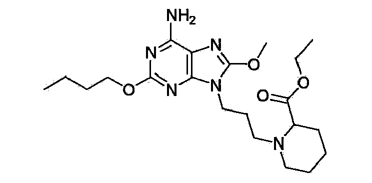

Using the compound 150mg (0.42mmol) obtained in step (i) of example 4, in the same manner as step (ii) of example 1, there was obtained the object compound 60mg as a white solid. Yield 33% ¹H NMR (CDCl₃) δ 5.15 (2H, brs), 4.26 (2H, t, J= 6.7 Hz), 4.19-4.10 (2H, m), 4.10 (3H, s), 4.09-3.91 (2H, m), 3.14-2.95 (2H, m), 2.58 (1H, m), 2.35 (1H, m), 2.18 (1H, m), 1.94-1.45 (12H, m), 1.22 (3H, t, J= 7.1 Hz), 0.96 (3H, t, J= 7.4 Hz).

### Step (ii)

### 2-Butoxy-7,8-dihydro-9-[3-(2-hydroxycarbonylpiperidin-1-yl)propyl]-8-oxoadenine

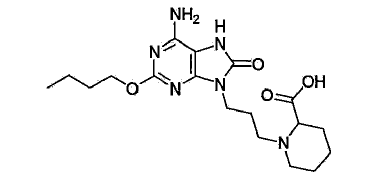

Using the compound 60 mg (0.14mmol) obtained in step (i), in the same manner as step (i) of example 1 and then example 37, there was obtained the object compound 42mg as a white solid. Yield 78%
¹H NMR (DMSO-d₆) δ 10.47 (1H, brs), 6.70 (2H, brs), 4.14 (2H, t, J= 6.6 Hz), 3.66 (2H, m), 3.10 (2H, m), 2.90 (1H, m), 2.64 (1H, m), 2.48 (1H, m), 1.98-1.34 (12H, m), 0.92 (3H, t, J= 7.4 Hz).

### Example 47

### 2-Butoxy-7,8-dihydro-9-{3-[4-(2-hydroxy-2-oxoethyl)piperidin-1-yl]propyl}-8-oxoadenine

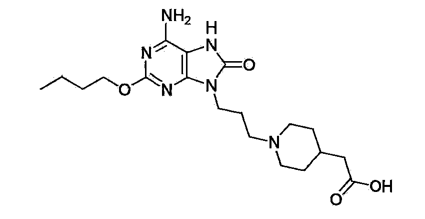

Using the compound 35mg (0.083mmol) obtained in example 11, in the same manner as example 37, there was obtained the object compound 23mg as a white solid. Yield 68%
¹H NMR (DMSO-d₆) δ 10.41 (1H, brs), 6.56 (2H, brs), 4.14 (2H, t, J= 6.6 Hz), 3.68 (2H, t, J= 6.9 Hz), 2.75 (2H, m), 2.26 (2H, t, J= 6.8 Hz), 2.02 (2H, d, J= 6.3 Hz), 1.78-1.58 (9H, m), 1.42-1.35 (2H, m), 1.15-0.97 (2H, m), 0.91 (3H, t, J= 7.4 Hz).

### Example 48

### 2-Butoxy-7,8-dihydro-9-{3-[3-(2-hydroxy-2-oxoethyl)piperidin-1-yl]propyl}-8-oxoadenine

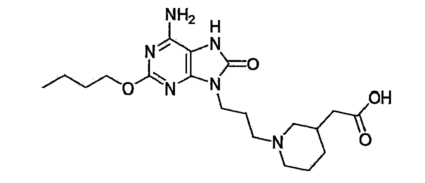

Using the compound 50mg (0.12mmol) obtained in example 12, in the same manner as example 37, there was obtained the object compound 31mg as a white solid. Yield 64%
¹H NMR (DMSO-d₆) δ 6.60 (2H, brs), 4.14 (2H, t, J= 6.6 Hz), 3.68-3.64 (2H, m), 2.69 (2H, m), 2.21 (2H, m), 2.08-2.03 (2H, m), 1.82-1.35 (13H, m), 0.91 (3H, t, J= 7.4 Hz).

### Example 49

### 2-Butoxy-7,8-dihydro-9-{3-[4-(3-hydroxy-3-oxopropyl)pyperazin-1-yl]propyl}-8-oxoadenine

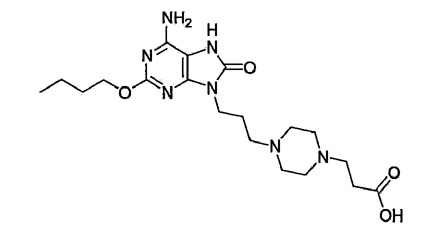

Using the compound 40mg (0.092mmol) obtained in example 13, in the same manner as example 37, there was obtained the object compound 33mg as a white solid. Yield 85% ¹H NMR (DMSO-d₆) δ 10.17 (1H, brs), 6.57 (2H, brs), 4.14 (2H, t, J= 6.6 Hz), 3.69 (2H, t, J= 6.9 Hz), 2.55-2.23 (14H; m), 1.80-1.75 (2H, m), 1.68-1.60 (2H, m), 1.42-1.36 (2H, m), 0.92 (3H, t, J= 7.4 Hz).

### Example 50

### 2-Butoxy-7,8-dihydro-9-{2-[2-(4-hydroxylcarbonylpiperidin-1-yl)ethoxy]ethyl}-8-oxoadenine

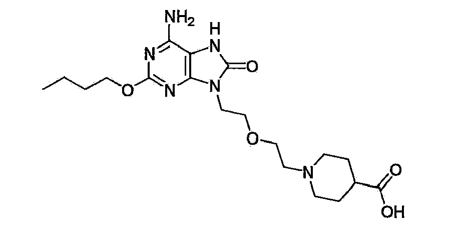

Using the compound 30mg (0.069mmol) obtained in example 14, reacting it in the same manner as example 37 and purifying by RPHPLC, there was obtained the object compound 23mg as a white solid. Yield 80% ¹H NMR (DMSO-d₆) δ 12.56 (1H, brs), 9.07 (1H, brs), 6.50 (2H, brs), 4.14 (2H, t, J= 6.6 Hz), 3.90 (2H, t, J= 5.4 Hz), 3.77 (2H, t, J= 5.4 Hz), 3.48-1.63 (15H, m), 1.41-1.37 (2H, m), 0.92 (3H, t, J= 7.4 Hz).

### Example 51

### 2-Butoxy-7,8-dihydro-9-[6-(4-hydroxycarbonylpiperidin-1-yl)hexyl]-8-oxoadenine

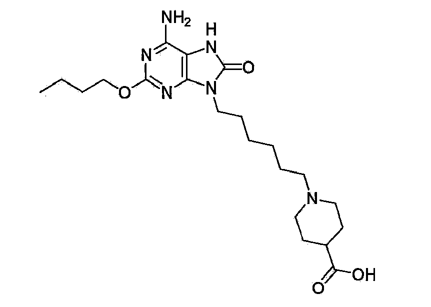

Using the compound 50mg (0.11mmol) obtained in example 15, in the same manner as example 37, there was obtained the object compound 33mg as a white solid. Yield 68%
¹H NMR (DMSO-d₆) δ 10.86 (1H, brs), 6.70 (2H, brs), 4.14 (2H, t, J= 6.4 Hz), 3.64 (2H, t, J= 6.6Hz), 2.72 (2H, d, J= 10.5 Hz), 2.16 (2H, t, J= 7.0Hz), 2.02-1.91 (1H, m), 1.81 (2H, t, J= 10.5Hz), 1.72-1.62 (6H, m), 1.49-1.25 (10H, m), 0.92 (3H, t, J= 7.3 Hz)

### Example 52

### 2-Butoxy-7,8-dihydro-9-[7-(4-hydroxycarbonylpiperidin-1-yl)heptyl]-8-oxoadenine

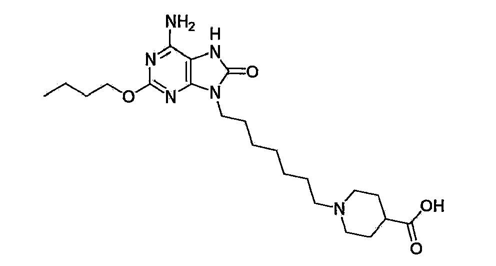

Using the compound 38mg (0.082mmol) obtained in example 16, in the same manner as example 37, there was obtained the object compound 21mg as a white solid. Yield 57%
¹H NMR (DMSO-d₆) δ 11.24 (1H, brs), 6.83 (2H, brs), 4.13 (2H, t, J= 6.6 Hz), 3.65 (2H, t, J= 6.6Hz), 2.69 (2H, d, J= 10.5 Hz), 2.15 (2H, t, J= 7.2Hz), 1.85-1.79 (3H, m), 1.68-1.61 (6H, m), 1.49-1.21 (12H, m), 0.92 (3H, t, J= 7.4 Hz).

### Example 53

### 2-Butoxy-7,8-dihydro-9-[8-(4-hydroxycarbonylpiperidin-1-yl)octyl]-8-oxoadenine

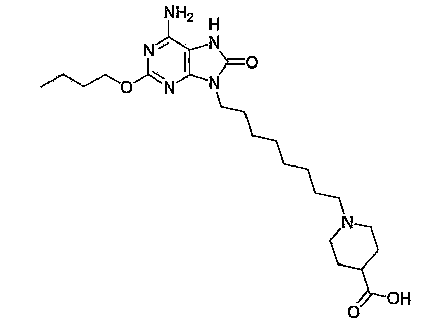

Using the compound 50mg (0.11mmol) obtained in example 17, in the same manner as example 37, there was obtained the object compound 31mg as a white solid. Yield 63%
¹H NMR (DMSO-d₆) δ 11.52 (1H, brs), 6.81 (2H, brs), 4.13 (2H, t, J= 6.6 Hz), 3.66 (2H, t, J= 6.4 Hz), 2.69 (2H, d, J= 11.0 Hz), 2.15 (2H, t, J= 7.0 Hz), 1.86-1.74 (3H, m), 1.67-1.62 (6H, m), 1.50-1.21 (14H, m), 0.92 (3H, t, J= 7.4 Hz).

### Example 54

### 2-Butoxy-7,8-dihydro-9-[4-(4-hydroxycarbonylpiperidin-1-yl)butyl]-8-oxoadenine

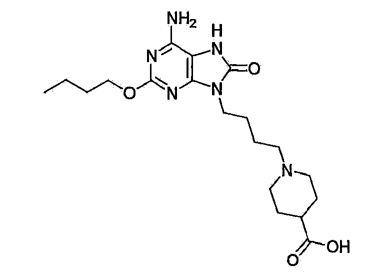

Using the compound 40mg (0.11mmol) obtained in example 18, in the same manner as example 37, there was obtained the object compound 24mg as a white solid. Yield 61%
¹H NMR (DMSO-d₆) δ 10.79 (1H, brs), 6.73 (2H, brs), 4.14 (2H, t, J= 6.6 Hz), 3.65 (2H, t, J= 6.8Hz), 2.70 (2H, d, J= 10.5 Hz), 2.22 (2H, t, J= 7.2Hz), 1.96-1.89 (3H, m), 1.69-1.62 (6H, m), 1.42-1.36 (6H, m), 0.92 (3H, t, J= 7.4 Hz).

### Example 55

### 2-Butoxy-7,8-dihydro-9-[4-(3-hydroxycarbonylpiperidin-1-yl)butyl]-8-oxoadenine

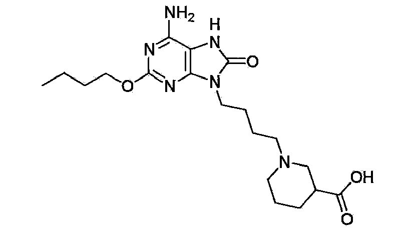

Using the compound 50mg (0.12mmol) obtained in example 24, in the same manner as example 37, there was obtained the object compound 33mg as a white solid. Yield 68% ¹H NMR (DMSO-d₆) δ 10.34 (1H, brs), 6.60 (2H, brs), 4.14 (2H, t, J= 6.6 Hz), 3.66 (2H, t, J= 6.8Hz), 2.82-2.79 (1H, m), 2.61-2.58 (1H, m), 2.32-2.29 (3H, m), 2.07-2.04 (1H, m), 1.93-1.89 (1H, m), 1.75-1.72 (1H, m), 1.67-1.58 (5H, m), 1.43-1.34 (6H, m), 0.91 (3H, t, J= 7.4 Hz).

### Example 56

### 2-Butoxy-7,8-dihydro-9-[4-(2-hydroxycarbonylpiperidin-1-yl)butyl]-8-oxoadenine

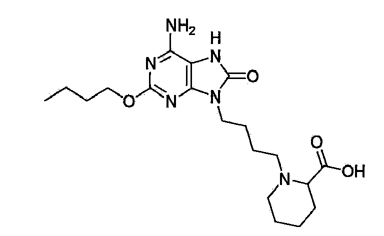

Using the compound 34mg (0.081mmol) obtained in example 20, in the same manner as example 37, there was obtained the object compound 25mg as a white solid. Yield 75%
¹H NMR (DMSO-d₆) δ 10.16 (1H, brs), 6.52 (2H, brs), 4.14 (2H, t, J= 6.6 Hz), 3.67 (2H, t, J= 6.8Hz), 3.21-3.18 (1H, m), 3.12-3.09 (1H, m), 3.01-2.94 (1H, m), 2.76-2.69 (1H, m), 2.59-2.52 (1H, m), 1.91-1.85 (1H, m), 1.67-1.54 (10H, m), 1.42-1.35 (3H, m), 0.91 (3H, t, J= 7.4 Hz).

### Example 57

### 2-Butoxy-7,8-dihydro-9-[4-(4-hydroxycarbonylmethylpiperazin-1-yl)butyl]-8-oxoadenine

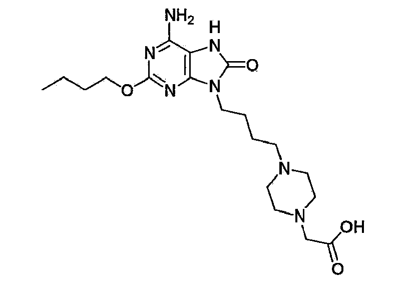

Using the compound 101mg (0.23mmol) obtained in example 21, in the same manner as example 37, there was obtained the object compound 34mg as a white solid. Yield 34%
¹H NMR (DMSO-d₆) δ 10.07 (1H, brs), 6.59 (2H, brs), 4.14 (2H, t, J= 6.6 Hz), 3.66 (2H, t, J= 6.8Hz), 3.13 (2H, s), 2.65-2.39 (8H, m), 2.28 (2H, t, J= 7.2Hz), 1.67-1.61 (4H, m), 1.42-1.36 (4H, m), 0.92 (3H, t, J= 7.4 Hz).

### Example 58

### 2-Butoxy-7,8-dihydro-9-{4-[4-(2-hydroxycarbonylethyl)piperazin-1-yl]butyl}-8-oxoadenine

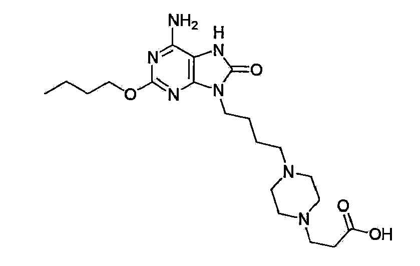

Using the compound 50mg (0.11mmol) obtained in example 22, in the same manner as example 37, there was obtained the object compound 24mg as a white solid. Yield 50%
¹H NMR (DMSO-d₆) δ 10.97 (1H, brs), 6.89 (2H, brs), 4.14 (2H, t, J= 6.6 Hz), 3.65 (2H, t, J= 6.7Hz), 2.47-2.13 (12H, m), 1.65-1.60 (4H, m), 1.41-1.34 (4H, m), 0.92 (3H, t, J= 7.3 Hz).

### Example 59

### 2-Buthoxy-7,8-dihydro-9-[4-(4-hydroxycarbonylmethylpiperidin-1-yl)butyl]-8-oxoadenine

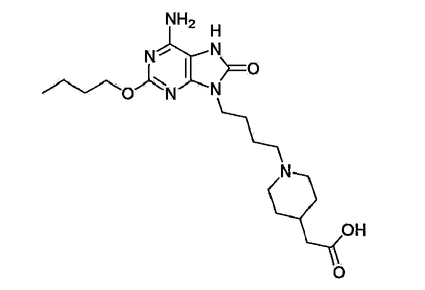

Using the compound 50mg (0.12mmol) obtained in example 23, in the same manner as example 37, there was obtained the object compound 38mg as a white solid. Yield 79%
¹H NMR (DMSO-d₆) δ 11.39 (1H, brs), 6.85 (2H, brs), 4.14 (2H, t, J= 6.6 Hz), 3.65 (2H, t, J= 6.7Hz), 2.72 (2H, m), 2.21 (2H, m), 1.87 (2H, d, J= 6.4Hz), 1.75 (2H, m), 1.65-1.57 (7H, m), 1.40-1.36 (4H, m), 1.05 (2H, m), 0.92 (3H, t, J= 7.4 Hz).

### Example 60

### 2-Butoxy-7,8-dihydro-9-[4-(3-hydroxycarbonylmethylpiperidin-1-yl)butyl]-8-oxoadenine

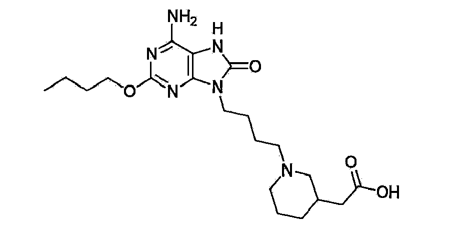

Using the compound 50mg (0.12mmol) obtained in example 24, in the same manner as example 37, there was obtained the object compound 44mg as a p white solid. Yield 91%
¹H NMR (DMSO-d₆) δ 10.91 (1H, brs), 6.73 (2H, s), 4.14 (2H, t, J= 6.6 Hz), 3.65 (2H, t, J= 6.7Hz), 2.70 (2H, m), 2.21 (2H, m), 1.95 (2H, m), 1.81 (2H, m), 1.67-1.62 (5H, m), 1.53 (2H, m), 1.41-1.34 (5H, m), 0.92 (3H, t, J= 7.4 Hz).

### Example 61

### 2-Butoxy-7,8-dihydro-9-[3-(1-hydroxycarbonylmethylpiperidin-4-yloxy)propyl]-8-oxoadenine

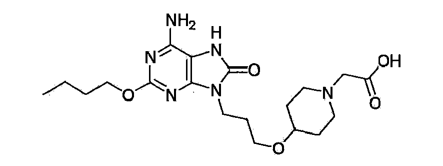

Using the compound 0.17g (0.39mmol) obtained in example 27, in the same manner as example 37, there was obtained the object compound 0.14g as a white solid. Yield 86%
¹H NMR (DMSO-d₆) δ 10.74 (1H, brs), 6.68 (2H, brs), 4.13 (2H, t, J = 6.6 Hz), 3.73 (2H, t, J = 6.6 Hz), 3.37 (3H, m), 2.99 (2H, s), 2.87 (2H, m), 2.45 (2H, m), 1.87 (2H, m), 1.79 (2H, m), 1.65 (2H, m), 1.51 (2H, m), 1.39 (2H, m), 0.91 (3H, t, J = 7.4 Hz).

### Example 62

### 2-Butoxy-7,8-dihydro-9-{3-[1-(2-hydroxy-2-oxoethyl)piperidin-4-ylmethyloxy]propyl}-8-oxoadenine

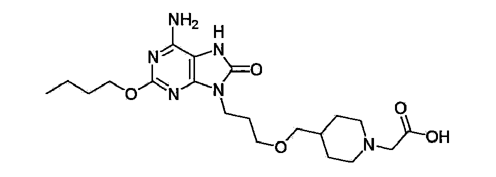

Using the compound 0.21g (0.46mmol) obtained in example 28, in the same manner as example 37, there was obtained the object compound 0.15g as a white solid. Yield 76%
¹H NMR (DMSO-d₆) δ 10.27 (1H, brs), 6.56 (2H, brs), 4.14 (2H, t, J = 6.6 Hz), 3.73 (2H, t, J = 6.6 Hz), 3.37 (2H, t, J = 5.8 Hz), 3.27 (2H, s), 3.25 (2H, m), 3.16 (2H, d, J = 6.2 Hz), 2.64 (2H, m), 1.87 (2H, m), 1.63 (5H, m), 1.37 (4H, m), 0.91 (3H, t, J = 7.3 Hz).

### Example 63

### 2-Butoxy-7,8-dihydro-9-(1-hydroxycarbonylmethylpiperidin-3-ylmethyl)-8-oxoadenine

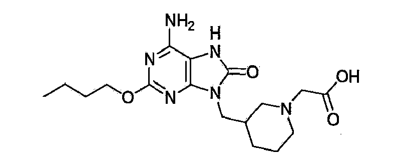

Using the compound 30mg (0.076mmol) obtained in example 30, in the same manner as example 37, there was obtained the object compound 25mg as a white solid. Yield 87%
¹H NMR (DMSO-d₆) δ 10.05 (1H, s), 6.51 (2H, brs), 4.14 (2H, t, J = 6.6 Hz), 3.58 (2H, m), 3.23 (2H, s), 3.01 (2H, m), 2.48 (1H, m), 2.33 (1H, m), 2.21 (1H, m), 1.60 (5H, m), 1.38 (2H, m), 1.03 (1H, m), 0.92 (3H, t, J = 7.3 Hz).

### Example 64

### 2-Butoxy-7,8-dihydro-9-{[1-(3-hydroxy-3-oxopropyl)piperidin-4-yl]methyl}-8-oxoadenine

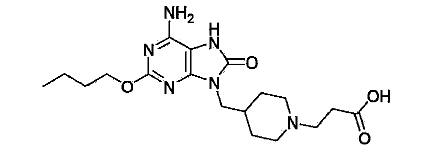

Using 2-butoxy-9-{[1-(3-methoxy-3-oxopropyl)piperidin-4-yl]methyl}-8-oxoadenine 0.37mg (0.09mmol) obtained in example 35, in the same manner as example 37, there was obtained the object compound 11mg as a white solid. Yield 31%
¹H NMR (DMSO-d₆) δ 10.98 (1H, brs), 6.70 (2H, brs), 4.13 (2H, t, J = 6.6 Hz), 3.52 (2H, d, J = 7.1 Hz), 2.81 (2H, m), 2.16 (2H, t, J = 7.4 Hz), 1.86 (2H, m), 1.76 (1H, m), 1.65 (2H, m), 1.48 (2H, m), 1.37 (2H, m), 1.17 (2H, m), 0.91 (3H, t, J = 7.3 Hz).

The following compounds were synthesized in accordance with the methods described in the present specification.

### Example 65

### 2-Butoxy-7,8-dihydro-9-{2-[(R)-2-methoxycarbonylpyrrolidin-1-yl]ethyl}-8-oxoadenine

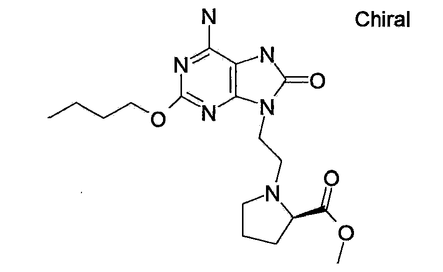

1H NMR δ(CD3OD) 4.30 (2H, t), 4.08 - 3.98 (1H, m), 3.94 - 3.84 (1H, m), 3.59 (3H, s), 3.39 - 3.19 (3H, m), 2.81 - 2.71 (1H, m), 2.59 - 2.49 (1H, m), 2.14 - 1.98 (1H, m), 1.93 - 1.70 (5H, m), 1.57 - 1.43 (2H, m), 1.00 (3H, t)

### Example 66

### 2-Butoxy-7,8-dihydro-9-{2-[(S)-2-methoxypyrrolidin-1-yl]ethyl}-8-oxoadenine

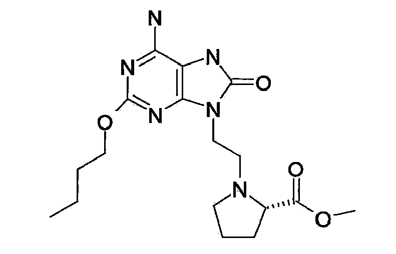

1H NMR δ(DMSO) 9.81 (1H, s), 6.36 (2H, s), 4.15 (2H, t), 3.85 - 3.65 (2H, m), 3.50 (3H, s), 3.30 (1H, t), 3.15 - 3.00 (2H, m), 2.64 (1H, m), 2.52 - 2.43 (1H, m), 2.01 - 1.89 (1H, m), 1.79 - 1.60 (5H, m), 1.39 (2H, sextet), 0.92 (3H, t)

### Example 67

### 2-Butoxy-7,8-dihydro-9-{3-[(S)-2-t-butoxycarbonylpyrrolidin-1-yl]propyl}-8-oxoadenine

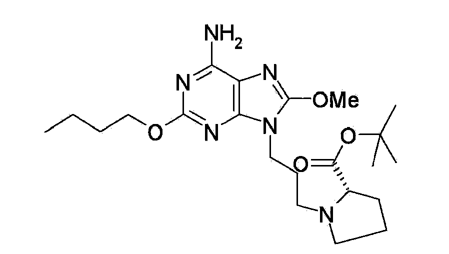

¹H NMR δ_{(CD3OD)} 4.27 (2H, t), 4.13 (3H, s), 4.09 - 3.91 (2H, m), 3.16 - 2.94 (2H, m), 2.76 - 2.63 (1H, m), 2.50 - 2.29 (2H, m), 2.11 - 2.01 (1H, m), 2.01 - 1.91 (2H, m), 1.86 - 1.68 (5H, m), 1.54 - 1.45 (2H, m), 1.44 (9H, s), 0.98 (3H, t)

### Example 68

### 2-Butoxy-7,8-dihydro-9-{3-[(S)-2-methoxycarbonylpyrrohdin-1-yl]propyl}-8-oxoadenine

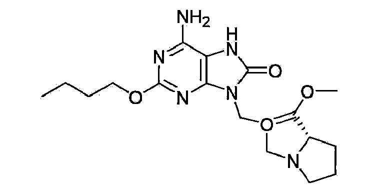

¹H NMR δ_{(CD3OD)} 4.28 (2H, t), 3.94 - 3.77 (2H, m), 3.66 (3H, s), 3.23 - 3.17 (1H, m), 3.14 - 3.07 (1H, m), 2.83 - 2.74 (1H, m), 2.51 - 2.33 (2H, m), 2.16 - 2.04 (1H, m), 2.00 - 1.78 (5H, m), 1.74 (2H, quintet), 1.48 (2H, sextet), 0.98 (3H, t)

### Example 69

### 2-Butoxy-7,8-dihydro-9-{3-[(S)-2-carboxypyrrolidin-1-yl]propyl}-8-oxoadenine

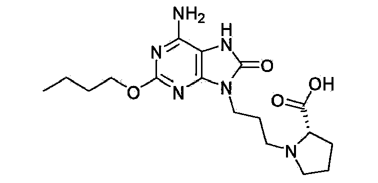

1H NMR δ (DMSO + DC1) 4.45 (2H, t), 4.36 (1H, t), 3.91 - 3.77 (2H, m), 3.74 - 3.60 (1H, m), 3.44 - 3.30 (1H, m), 3.26 - 3.08 (2H, m), 2.50 - 2.36 (1H, m), 2.22 - 1.81 (5H, m), 1.73 (2H, quintet), 1.43 (2H, sextet), 0.94 (3H, t)

### Example 70

### 2-Butoxy-7,8-dihydro-9-{4-[(S)-2-methoxycarbonylpyrrolidin-1-yl]butyl}-8-oxoadenine

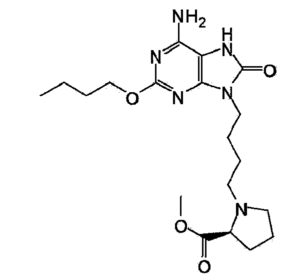

¹H NMR δ_{(CDCL3)} 5.11 (2H, s), 4.27 (2H, t), 4.10 (3H, s), 3.93 (2H, t), 3.69 (3H, s), 3.17 - 3.08 (2H, m), 2.73 - 2.64 (1H, m), 2.43 - 2.34 (1H, m), 2.28 (1H, q), 2.13 - 2.04 (1H, m), 1.95 - 1.86 (2H, m), 1.83 - 1.71 (5H, m), 1.54 - 1.45 (4H, m), 0.96 (3H, t)

### Example 71

### 2-Butoxy-7,8-dihydro-9-{4-[(S)-2-methoxycarbonylpyrrolidin-1-yl]butyl}-8-oxoadenine fumalate

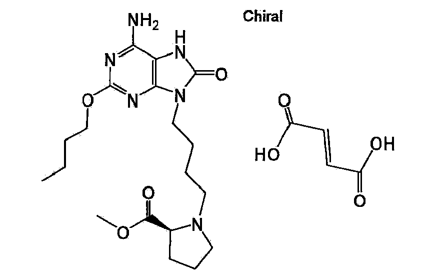

¹H NMR δ_{(DMSO)} 9.87 (1H, s), 6.62 (2H, s), 6.40 (2H, s), 4.14 (2H, t), 3.66 (2H, t), 3.59 (3H, s), 3.19 - 3.12 (1H, m), 2.99 - 2.92 (1H, m), 2.69 - 2.58 (1H, m), 2.42 - 2.25 (2H, m), 2.05 - 1.95 (1H, m), 1.82 - 1.57 (7H, m), 1.38 (4H, septet), 0.92 (3H, t)

### Example 72

### 2-Butoxy-7,8-dihydro-9-[2-(4-methoxycarbonylmethylpiperadin-1-yl)ethyl]-8-oxoadenine

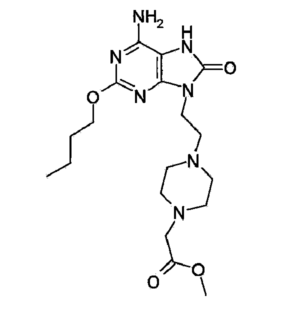

¹H NMR δ_{(DMSO)} 9.83 (1H, s), 6.38 (2H, s), 4.14 (2H, t), 3.77 (2H, t), 3.59 (3H, s), 3.42 - 3.22 (4H, m), 2.57 (2H, t), 2.48 - 2.37 (6H, m), 1.63 (2H, quintet), 1.38 (2H, sextet), 0.91 (3H, t)

### Example 73

### Human TLR7 reporter assay

HEK293 cells in which human TLR7 or rat TLR7 plasmid and reporter plasmid (NF-kB-SEAP) are stably introduced are dispersed in DMEM broth (10% FBS, 1% NEAA, 10ug/mL blastocidin S HCl, 100 ug/mL Zeocin), and were seeded to 96 well plate per 90µl/well (hTLR7/seap-293:20000cells/well, rTLR7/seap-293:25000cells/well).

Test compound (DMSO stock solution (2µl) was diluted with the broth (200µl) by 100times) was added to the seeded cells to a 96well plate (10µl/well) (final concentration; 1nM - 10µM, common ratio 3). After stirring by tapping side of the plate, the cells were cultured in a CO₂ incubator for 20 hours. A substrate (50µl/well) for reporter assay (substrate for SEAP, pNPP) was added to cells stimulated by test sample. Ten minutes after adding the substrate, the reaction quenching solution (4N NaOH) was added by 50µl/well to cease enzymatic reaction. Sealing a top seal A on the plate, the absorbance was measured by a micro plate reader (405nm).

Human TLR7 binding activity (EC₅₀) of each compound is shown in Table 1.

**Table 1**

| Compound | EC₅₀ (nM) |
|---|---|
| Example 1 | 144.6 |
| Example 3 | 570.7 |
| Example 5 | 807.4 |
| Example 7 | 4792.0 |
| Example 8 | 1225.0 |
| Example 9 | 989.1 |
| Example 11 | 3241.9 |
| Example 12 | 1606.0 |
| Example 15 | 95.2 |
| Example 16 | 144.6 |
| Example 17 | 147.8 |
| Example 19 | 630.8 |

## Claims

1. An adenine compound represented by the following formula (1): 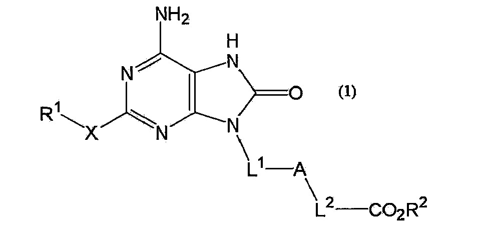 [wherein R¹ is optionally substituted alkyl group, optionally substituted alkenyl group, optionally substituted alkynyl group, optionally substituted cycloalkyl group, optionally substituted aryl group or optionally substituted heteroaryl group;
R2 is hydrogen atom, optionally substituted alkyl group, optionally substituted alkenyl group, optionally substituted alkynyl group or optionally substituted cycloalkyl group;
X is oxygen atom, sulfur atom, NR⁴ (wherein R⁴ is hydrogen atom or C ₁₋₆ alkyl group), SO, SO₂ or a single bond;
A is an optionally substituted and saturated or unsaturated 4 to 8 membered heterocyclic group containing 1 to 2 hetero atoms selected from 1 to 2 nitrogen atoms, 0 to 1 oxygen atom and 0 to 1 sulfur atom; and
L¹ and L² are independently, a straight or branched chain alkylene or a single bond and one to three methylene groups in said alkylene may be substituted by oxygen atom, sulfur atom, NR⁵ (wherein R⁵ is hydrogen atom or alkyl group.), SO, SO₂ or carbonyl group.]
or its pharmaceutically acceptable salt.

2. The adenine compound or its pharmaceutically acceptable salt according to claim 1, wherein substituted alkyl group, substituted alkenyl group, substituted alkynyl group and substituted cycloalkyl group in R² are substituted by one or more substituents independently selected from the group consisting of halogen atom, hydroxy group, carboxy group, mercapt group, C₁₋₆ alkoxy group, C₁₋₆ haloalkoxy group, C₂₋₆ alkoxycarbonyl group, C₁₋₆ alkylsulfonyl group, C₁₋₆ alkylsulfinyl group, C₂₋₆ alkylcarbonyloxy group, C₁₋₆ alkylthio group, optionally substituted amino group, optionally substituted carbamoyl group, optionally substituted sulfamoyl group and 3 to 8 membered cycloalkyl group (said cycloalkyl group may be substituted by halogen atom, hydroxy group, carboxy group, C₁₋₄ alkyl group or C₁₋₄ alkoxy group.),
substituents of the above substituted amino group, substituted carbamoyl group and substituted sulfamoyl group are one or two substituents independently selected from the group (a'), or a substituent selected from the group (b'):
(a') C₁₋₆ alkyl group, C₂₋₆ alkenyl group, C₂₋₆ alkynyl group, C₂₋₆ alkylcarbonyl group, C₂₋₆ alkoxycarbonyl group, C₁₋₆ alkylsulfonyl group, C₁₋₆ alkylsulfinyl group, 3 to 8 membered cycloalkyl group, 3 to 8 membered cycloalkylcarbonyl group, 3 to 8 membered cycloalkoxycarbonyl group, 3 to 8 membered cycloalkylsulfonyl group and 3 to 8 membered cycloalkylsulfinyl group (wherein the group in this group may be substituted by halogen atom, hydroxy group, C₁₋₆ alkoxy group, carboxy group or C₂₋₅ alkoxycarbonyl group.);
(b') 4 to 7 membered saturated heterocyclic group having one to two hetero atoms selected from 1 to 2 nitrogen atoms, 0 to 1 oxygen atom and 0 to 1 sulfur atom which is formed by combining two substituents with the nitrogen atom (said saturated heterocyclic group may be substituted on its carbon atom or nitrogen atom, if chemically stable, by halogen atom, hydroxy group, carboxy group, C₁₋₆ alkyl group, C₁₋₆ alkoxy group, C₂₋₆ alkoxycarbonyl group or C₂₋₆ alkylcarbonyl group.),
A may be substituted by one or more substituesnts independently select from the group consisting of halogen atom, hydroxy group, carboxy group, C₁₋₆ alkyl group, C₁₋₆ alkoxy group, C ₂₋₆ alkoxycarbonyl group, C₂₋₆ alkylcarbonyl group, C₁₋₆ alkylsulfonyl group and C₁₋₆ alkylsulfinyl group,
substituted alkyl group, substituted alkenyl group and substituted alkynyl group in R¹ are substituted by one or more substituents independently selected from the group consisting of (a) to (c) below:
(a) halogen atom, hydroxy group, carboxy group, C₁₋₆ haloalkoxy group, and mercapt group;
(b) C₁₋₆ alkoxy group, C₁₋₆ alkylthio group, C₂₋₆ alkylcarbonyl group, C₂₋₆ alkylcarbonyloxy group, C₁₋₆ alkylsulfonyl group, C₁₋₆ alkylsulfinyl group, and C₂₋₆ alkoxycarbonyl group (wherein the group of this group may be substituted by one or more substituents independently selected from the group consisting of halogen atom, hydroxy group, carboxy group, C₁₋₆ alkoxy group, C₂₋₆ alkoxycarbonyl group, amino group optionally substituted by the same or different and one or two C₁₋₆ alkyl groups, carbamoyl group optionally substituted by the same or different and one or two C₁₋₆ alkyl groups, sulfamoyl group optionally substituted by the same or different and one or two C₁₋₆ alkyl groups and C₁₋₆ alkylsulfonyl group.);
(c) optionally substituted amino group, optionally substituted carbamoyl group and optionally substituted sulfamoyl group (wherein the group of this group may be substituted by one or more substituents selected from groups (j), (k) and (l) below), optionally substituted 3 to 8 membered cycloalkyl group and optionally substituted 4 to 8 membered saturated heterocyclic group (wherein the group of this group may be substituted by one or more substituents selected from groups (d), (e) and (f) below), and optionally substituted 6 to 10 membered aryl group, optionally substituted 5 to 10 membered heteroaryl group, optionally substituted 6 to 10 membered aryloxy group and optionally substituted 5 to 10 membered heteroaryloxy group (wherein the group of this group may be substituted by one or more substituents selected from groups (g), (h) and (i) below);
cycloalkyl group in R¹ may be substituted by one or more substituents independently selected from the group consisting of (d) to (f) below:
(d) halogen atom, hydroxy group, carboxy group, mercapt group, C₁₋₆ haloalkyl group and C₁₋₆ haloalkoxy group;
(e) C₁₋₆ alkyl group, C₂₋₆ alkenyl group, C₂₋₆ alkynyl group, C₁₋₆ alkoxy group, C₂₋₆ alkylcarbonyl group, C₂₋₆ alkylcarbonyloxy group, C₂₋₆ alkoxycarbonyl group, C₁₋₆ alkylthio group, C₁₋₆ alkylsulfonyl group, and C₁₋₆ alkylsulfinyl group (wherein the group of this group may be substituted by one or more substituents independently selected from the group consisting of halogen atom, hydroxy group, carboxy group, C₁₋₆ alkoxy group, C₂₋₆ alkoxycarbonyl group, amino group optionally substituted by the same or different and one or two C₁₋₆ alkyl groups, carbamoyl group optionally substituted by the same or different and one or two C₁₋₆ alkyl groups, sulfamoyl group optionally substituted by the same or different and one ore two C₁₋₆ alkyl groups and C₁₋₆ alkylsulfonyl group.);
(f) optionally substituted amino group, optionally substituted carbamoyl group and optionally substituted sulfamoyl group (the group of this group may be substituted by one or two substituents selected from the group consisting of (j), (k) and (l) below), and optionally substituted 6 to 10 membered aryl group and optionally substituted 5 to 10 membered heteroaryl group (the group of this group may be substituted by one or more substituents selected from the group consisting of (g), (h) and (i) below);
Substituted aryl group and substituted heteroaryl group in R¹ are substituted by one or more substituents independently selected from the group consisting of (g) to (i):
(g) halogen atom, hydroxy group, mercapt group, cyano group, nitro group, C₁₋₆ haloalkyl group, and C₁₋₆ haloalkoxy group;
(h) C₁₋₆ alkyl group, C₂₋₆ alkenyl group, C₂₋₆ alkynyl group, C₁₋₆ alkoxy group, C₂₋₆ alkylcarbonyl group, C₂₋₆ alkoxycarbonyl group, C₂₋₆ alkylcarbonyloxy group, C₁₋₆ alkylthio group, C₁₋₆ alkylsulfonyl group, C₁₋₆ alkylsulfinyl group, 3 to 8 membered cycloalkyl group and 4 to 8 membered saturated heterocyclic group (wherein the group of this group may be substituted by one or more substituents independently selected from a group consisting of halogen atom, hydroxy group, carboxy group, C₁₋₆ alkyl group, C₁₋₆ alkoxy group, C₂₋₆ alkoxycarbonyl group, amino group optionally substituted by the same or different and one or two C₁₋₆ alkyl groups, carbamoyl group optionally substituted by the same or different, and one or two C₁₋₆ alkyl groups, sulfamoyl group optionally substituted by the same or different and one or two C₁₋₆ alkyl groups and C₁₋₆ alkylsulfonyl group.);
(i) optionally substituted amino group, optionally substituted carbamoyl group, and optionally substituted sulfamoyl group (the group of this group may be substituted by one or two substituents selected from the group consisting of (j), (k) and (l) below);
substituted amino group, substituted carbamoyl group and substituted sulfamoyl group in the above (a) to (i) are substituted by one or two substituents independently selected from the group consisting of (j) to (l) below:
(j) C₁₋₆ alkyl group, C₂₋₆ alkenyl group, C₂₋₆ alkynyl group, C₂₋₆ alkylcarbonyl group, C₂₋₆ alkoxycarbonyl group, C₁₋₆ alkylsulfonyl group, C₁₋₆ alkylsulfinyl group, 3 to 8 membered cycloalkyl group, 3 to 8 membered cycloalkylcarbonyl group, 3 to 8 membered cycloalkoxycarbonyl group, 3 to 8 membered cycloalkylsulfonyl group, and 3 to 8 membered cycloalkylsulfinyl group (wherein the group of this group may be substituted by one or more substituents independently selected from the group consisting of halogen atom, hydroxy group, carboxy group, C₁₋₆ alkyl group, C₁₋₆ alkoxy group, C₂₋₆ alkoxycarbonyl group, amino group optionally substituted by the same or different and one or two C₁₋₆ alkyl groups, carbamoyl group optionally substituted by the same or different and one or two C₁₋₆ alkyl groups, sulfamoyl group optionally substituted by the same or different and one or two C₁₋₆ alkyl groups and C₁₋₆ alkylsulfonyl group.);
(k) 6 to 10 membered aryl group, 6 to 10 membered arylcarbonyl group, 6 to 10 membered aryloxycarbonyl group, a 6 to 10 membered arylsulfonyl group, 6 to 10 membered arylsulfinyl group, 5 to 10 membered heteroaryl group, 5 to 10 membered heteroarylcarbonyl group, 5 to 10 membered heteroaryloxycarbonyl group, 5 to 10 membered heteroarylsulfonyl group, and 5 to 10 membered heteroarylsulfinyl group (wherein the group of this group may be substituted by halogen atom, hydroxy group, C₁₋₆ alkyl group, C₁₋₆ alkoxy group, amino group optionally substituted by the same or different and one or two C₁₋₆ alkyl groups, carbamoyl group optionally substituted by the same or different and one or two C₁₋₆ alkyl groups, sulfamoyl group optionally substituted by the same or different and one or two C₁₋₆ alkyl groups, or C₁₋₆ alkylsulfonyl group.);
(1) 4 to 7 membered saturated heterocyclic group containing 1 or 2 hetero atoms selected from 1 to 2 nitrogen atoms, 0 to 1 oxygen atom, and 0 to 1 sulfur atom which is formed by combining two substituents with the nitrogen atm (said saturated heterocyclic group may be substituted on appropriate carbon atom or nitrogen atom, if chemically stable, by halogen atom, hydroxy group, carboxy group, C₁₋₆ alkyl group, C₁₋₆ alkoxy group, C₂₋₆ alkoxycarbonyl group, C ₂₋₆ alkylcarbonyl group, amino group which may be substituted by the same or different and one or two C₁₋₆ alkyl groups, carbamoyl group which may be substituted by the same or
different and one or two C₁₋₆ alkyl groups, sulfamoyl group which may be substituted by the same or different and one or two C₁₋₆ alkyl groups, or C₁₋₆ alkylsulfonyl group.).

3. The adenine compound or its pharmaceutically acceptable salt according to claim 1 or 2, wherein in the formula (1), A is pyrrolidine, piperidine, piperazine, morpholine, thiomorpholine, thiomorpholine-1-oxide or thiomorpholine-1,1-dioxide.

4. The adenine compound or its pharmaceutically acceptable salt according to any one of claims 1 to 3, wherein the formula (1), R² is C₁₋₄ alkyl group.

5. The adenine compound or its pharmaceutically acceptable salt according to claim 4, wherein in the formula (1), R² is methyl group.

6. The adenine compound or its pharmaceutically acceptable salt according to any one of claims 1 to 3, wherein in the formula (1), R² is C₂₋₆ alkyl group substituted by optionally substituted amino group.

7. The adenine compound or its pharmaceutically acceptable salt according to any one of claims 1 to 6, wherein in the formula (1), L¹ is a following formula:
(CH₂)ₙ-(Y¹)ₘ-(CH₂)₁ₐ
[wherein Y¹ is oxygen atom or NR⁵' (wherein R⁵' is hydrogen atom or C₁₋₆ alkyl group.), n and la are independently an integer of 0 to 5, and m is 0 or 1.],
and L² is a single bond or straight chained C₁₋₄ alkylene.

8. The adenine compound or its pharmaceutically acceptable salt according to claim 1 wherein the compound is selected from the group consisting of the following compounds:
2-Butoxy-7,8-dihydro-9-[5-(4-methoxycarbonylpiperidin-1-yl)pentyl]-8-oxoadenine;
2-Butoxy-7,8-dihydro-9-[5-(4-methoxycarbonylmethylpiperidin-1-yl)pentyl]-8-oxoadenine;
2-Butoxy-7,8-dihydro-9-[5-(3-methoxycarbonylmethylpiperidin-1-yl)pentyl]-8-oxoadenine;
2-Butoxy-7,8-dihydro-9-[2-(4-methoxycarbonylpiperidin-1-yl)ethyl]-8-oxoadenine;
2-Butoxy-7,8-dihydro-9-[2-(3-methoxycarbonylpiperidin-1-yl)ethyl]-8-oxoadenine;
2-Butoxy-7,8-dihydro-9-{2-(2-methoxycarbonylpiperidin-1-yl)ethyl}-8-oxoadenine;
2-Butoxy-7,8-dihydro-9-[2-{4-(2-methoxycarbonylethyl)piperidin-1-yl}ethyl]-8-oxoadenine;
2-Butoxy-7,8-dihydro-9-[3-(4-methoxycarbonylpiperidin-1-yl)propyl]-8-oxoadenine;
2-Butoxy-7,8-dihydro-9-[3-(3-methoxycarbonylpiperidin-1-yl)propyl]-8-oxoadenine;
2-Butoxy-7,8-dihydro-9-[3-(2-methoxycarbonylpiperidin-1-yl)propyl]-8-oxoadenine;
2-Butoxy-7,8-dihydro-9-{3-[4-(2-methoxy-2-oxoethyl)piperidin-1-yl]propyl}-8-oxoadenine;
2-Butoxy-7,8-dihydro-9-{3-[3-(2-methoxy-2-oxoethyl)piperidin-1-yl]propyl}-8-oxoadenine;
2-Butoxy-7,8-dihydro-9-{3-[4-(3-methoxy-3-oxopropyl)piperazin-1-yl]propyl}-8-oxoadenine;
2-Butoxy-7,8-dihydro-9-{2-[2-(4-methoxycarbonylpiperidin-1-yl)ethoxy]ethyl}-8-oxoadenine;
2-Butoxy-7,8-dihydro-9-[6-(4-methoxycarbonylpiperidin-1-yl)hexyl]-8-oxoadenine;
2-Butoxy-7,8-dihydro-9-[7-(4-methoxycarbonylpiperidin-1-yl)heptyl]-8-oxoadenine;
2-Butoxy-7,8-dihydro-9-[8-(4-methoxycarbonylpiperidin-1-yl)octyl]-8-oxoadenine;
2-Butoxy-7,8-dihydro-9-[4-(4-methoxycarbonylpiperidin-1-yl)butyl]-8-oxoadenine;
2-Butoxy-7,8-dihydro-9-[4-(3-methoxycarbonylpiperidin-1-yl)butyl]-8-oxoadenine;
2-Butoxy-7,8-dihydro-9-[4-(2-methoxycarbonylpiperidin-1-yl)butyl]-8-oxoadenine;
2-Butoxy-7,8-dihydro-9-{4-[(4-methoxycarbonylinethyl)piperazin-1-yl]butyl}-8-oxoadenine;
2-Butoxy-7,8-dihydro-9-{4-[4-(2-methoxycarbonylethyl)piperazin-1-yl]butyl}-8-oxoadenine;
2-Butoxy-7,8-dihydro-9-[4-(4-methoxycarbonylmethylpiperidin-1-yl)butyl]-8-oxoadenine;
2-Butoxy-7,8-dihydro-9-[4-(3-methoxycarbonylmethylpiperidin-1-yl)butyl]-8-oxoadenine;
2-Butoxy-7,8-dihydro-9-(4-methoxycarbonylmethylmorpholin-2-ylmethyl)-8-oxoadenine;
2-Butoxy-7,8-dihydro-9-(1-methoxycarbonylmethylpiperidin-4-ylmethyl)-8-oxoadenine;
2-Butoxy-7,8-dihydro-9-[3-(1-methoxycarbonylmethylpiperidin-4-yloxy)propyl]-8-oxoadenine;
2-Butoxy-7,8-dihydro-9-{3-[1-(2-methoxy-2-oxoethyl)piperidin-4-ylmethyloxy]propyl}-8-oxoadenine;
2-Butoxy-7,8-dihydro-9-(3-{1-[4-(dimethylamino)propoxycarbonylmethyl]piperidin-4-ylmethoxy}propyl)-8-oxoadenine;
2-Butoxy-7,8-dihydro-9-(1-methoxycarbonylmethylpiperidin-3-ylmethyl)-8-oxoadenine;
2-Butoxy-7,8-dihydro-9-[1-(3-methoxy-3-oxopropyl)piperidin-3-ylmethyl]-8-oxoadenine;
2-Butoxy-7,8-dihydro-9-[2-(1-methoxycarbonylmethylpiperidin-4-yl)ethyl]-8-oxoadenine;
2-Butoxy-7,8-dihydro-9-{2-[1-(3-methoxy-3-oxopropyl)piperidin-4-yl]ethyl}-8-oxoadenine;
2-Butoxy-7,8-dihydro-9-[2-(1-methoxycarbonylmethylpiperidin-2-yl)ethyl]-8-oxoadenine;
2-Butoxy-7,8-dihydro-9-{[1-(3-methoxy-3-oxopropyl)piperidin-4-yl]methyl}-8-oxoadenine;
2-Butoxy-7,8-dihydro-9-{2-[4-(2-methoxy-2-oxoethyl)morpholin-2-yl]ethyl}-8-oxoadenine;
2-Butoxy-7,8-dihydro-9-{5-(4-hydroxycarbonylpiperidin-1-yl)pentyl}-8-oxoadenine;
2-Butoxy-7,8-dihydro-9-[5-(4-hydroxycarbonylmethylpiperidin-1-yl)pentyl]-8-oxoadenine;
2-Butoxy-7,8-dihydro-9-[5-(3-hydroxycarbonylmethylpiperidin-1-yl)pentyl]-8-oxoadenine;
2-Butoxy-7,8-dihydro-9-{2-(4-hydroxycarbonylpiperidin-1-yl)ethyl}-8-oxoadenine;
2-Butoxy-7,8-dihydro-9-[2-(3-hydroxycarbonylpiperidin-1-yl)ethyl]-8-oxoadenine;
2-Butoxy-7,8-dihydro-9-{2-(2-hydroxycarbonylpiperidin-1-yl)ethyl}-8-oxoadenine;
2-Butoxy-7,8-dihydro-9-[2-{4-(2-carboxyethyl)piperidin-1-yl}ethyl]-8-oxoadenine;
2-Butoxy-7,8-dihydro-9-[3-(4-hydroxycarbonylpiperidin-1-yl)propyl]-8-oxoadenine;
2-Butoxy-7,8-dihydro-9-[3-(3-hydroxycarbonylpiperidin-1-yl)propyl]-8-oxoadenine;
2-Butoxy-7,8-dihydro-9-[3-(2-hydroxycarbonylpiperidin-1-yl)propyl]-8-oxoadenine;
2-Butoxy-7,8-dihydro-9-{3-[4-(2-hydroxy-2-oxoethyl)piperidin-1-yl]propyl}-8-oxoadenine;
2-Butoxy-7,8-dihydro-9-{3-[3-(2-hydroxy-2-oxoethyl)piperidin-1-yl]propyl}-8-oxoadenine;
2-Butoxy-7,8-dihydro-9-{3-[4-(3-hydroxy-3-oxopropyl)pyperazin-1-yl]propyl}-8-oxoadenine;
2-Butoxy-7,8-dihydro-9-{2-[2-(4-hydroxycarbonylpiperidin-1-yl) ethoxy]ethyl}-8-oxoadenine;
2-Butoxy-7,8-dihydro-9-{6-(4-hydroxycarbonylpiperidin-1-yl)hexyl}-8-oxoadenine;
2-Butoxy-7,8-dihydro-9-{7-(4-hydroxycarbonylpiperidin-1-yl)heptyl}-8-oxoadenine;
2-Butoxy-7,8-dihydro-9-{8-(4-hydroxycarbonylpiperidin-1-yl)octyl}-8-oxoadenine;
2-Butoxy-7,8-dihydro-9-{4-(4-hydroxycarbonylpiperidin-1-yl)butyl}-8-oxoadenine;
2-Butoxy-7,8-dihydro-9-{4-(3-hydroxycarbonylpiperidin-1-yl)butyl}-8-oxoadenine;
2-Butoxy-7,8-dihydro-9-{4-(2-hydroxycarbonylpiperidin-1-yl)butyl}-8-oxoadenine;
2-Butoxy-7,8-dihydro-9-{4-[(4-hydroxycarbonylmethyl)piperazin-1-yl]butyl}-8-oxoadenine;
2-Butoxy-7,8-dihydro-9-{4-[4-(2-hydroxycarbonylethyl)piperazin-1-yl]butyl}-8-oxoadenine;
2-Butoxy-7,8-dihydro-9-[4-(4-hydroxycarbonylmethylpiperidin-1-yl)butyl]-8-oxoadenine;
2-Butoxy-7,8-dihydro-9-[4-(3-hydroxycarbonylmethylpiperidin-1-yl)butyl]-8-oxoadenine;
2-Butoxy-7,8-dihydro-9-[3-(1-hydroxycarbonylmethylpiperidin-4-yloxy)propyl]-8-oxoadenine;
2-Butoxy-7,8-dihydro-9-{3-[1-(2-hydroxy-2-oxoethyl)piperidin-4-ylmethyloxy]propyl}-8-oxoadenine;
2-Butoxy-7,8-dihydro-9-(1-hydroxycarbonylmethylpiperidin-3-ylmethyl)-8-oxoadenine;
2-Butoxy-7,8-dihydro-9-{[1-(3-hydroxy-3-oxopropyl)piperidin-4-yl]methyl}-8-oxoadenine;
2-Butoxy-7,8-dihydro-9-{2-[(R)-2-methoxycarbonylpyrrolidin-1-yl]ethyl}-8-oxoadenine;
2-Butoxy-7,8-dihydro-9-{2-[(S)-2-methoxycarbonylpyrrolidin-1-yl]ethyl}-8-oxoadenine;
2-Butoxy-7,8-dihydro-9-{3-[(S)-2-t-butoxycarbonylpyrrolidin-1-yl]propyl}-8-oxoadenine;
2-Butoxy-7,8-dihydro-9-{3-[(S)-2-methoxycarbonylpyrrolidin-1-yl]propyl}-8-oxoadenine;
2-Butoxy-7,8-dihydro-9-{3-[(S)-2-carboxypyrrolidin-1-yl]propyl}-8-oxoadenine;
2-Butoxy-7,8-dihydro-9-{4- [(S)-2-methoxycarbonylpyrrolidin-1-yl]butyl}-8-oxoadenine;
2-Butoxy-7,8-dihydro-9-{4-[(S)-2-methoxycarbonylpyrrolidin-1-yl]butyl}-8-oxoadenine fumarate and
2-Butoxy-7,8-dihydro-9-[2-(4-methoxycarbonylmethylpiperazin-1-yl)ethyl]-8-oxoadenine.

9. A pharmaceutical composition containing the adenine compound or a pharmaceutically acceptable salt thereof as described in any one of claims 1 to 8 as an active ingredient.

10. A TLR7 activator containing the adenine compound or a pharmaceutically acceptable salt thereof as described in any one of claims 1 to 8 as an active ingredient.

11. An immuno-modifier containing the adenine compound or a pharmaceutically acceptable salt thereof as described in any one of claim 1 to 8 as an active ingredient.

12. A therapeutic or prophylactic agent for allergic diseases, viral diseases or cancers containing the adenine compound or a pharmaceutically acceptable salt thereof as described in any one of claims 1 to 8 as an active ingredient.

13. A therapeutic or prophylactic agent for asthma, COPD, allergic rhinitis, allergic conjunctivitis, atopic dermatosis, cancer, hepatitis B, hepatitis C, HIV, HPV, a bacterial infectious disease, or dermatosis containing the adenine compound or a pharmaceutically acceptable salt thereof as described in any one of claims 1 to 8 as an active ingredient.

14. A medicament for topical administration containing the adenine compound or a pharmaceutically acceptable salt thereof as described in any one of claims 1 to 8 as an active ingredient.
